(19) Europäisches Patentamt
European Patent Office
Office européen des brevets

(11) Publication number: 0 129 478 B1

(12) **EUROPEAN PATENT SPECIFICATION**

(45) Date of publication of patent specification: 17.01.90

(51) Int. Cl.[5]: C 07 D 333/62, C 07 D 409/12, C 07 D 413/12, C 07 D 409/04, A 61 K 31/38

(21) Application number: 84401227.8

(22) Date of filing: 14.06.84

# (54) 2-Sulfamoylbenzo (b) thiophene derivatives, their preparation and pharmaceutical composition for the treatment of elevated intraocular pressure.

(30) Priority: 20.06.83 US 506092
31.10.83 US 547191

(43) Date of publication of application:
27.12.84 Bulletin 84/52

(45) Publication of the grant of the patent:
17.01.90 Bulletin 90/03

(84) Designated Contracting States:
AT BE CH DE FR GB IT LI LU NL SE

(56) References cited:
EP-A-0 070 239
EP-A-0 079 269
EP-A-0 091 367
DE-C-1 059 921

CHEMICAL ABSTRACTS, vol. 81, no. 17, 28th October 1974, page 496, left-hand column, abstract no. 105154d, Columbus, Ohio, US; I.M. NASYROV et al.: "Structure of sulfonation products of 3,5-dimethyl-1-thiaindene and its sulfone studies by NMR"; & DOKL. AKAD. NAUK TADSH. SSR, 1974, 17 (3), 36-38

(73) Proprietor: MERCK & CO. INC.
126, East Lincoln Avenue P.O. Box 2000
Rahway New Jersey 07065-0900 (US)

(72) Inventor: Shepard, Kenneth L.
136 Cardinal Way
North Wales Pennsylvania 19454 (US)
Inventor: Graham, Samuel L.
143 Ardwick Terrace
Lansdale Pennsylvania 19446 (US)
Inventor: Czaja, Robert F.
1282 Christine Circle
Scotch Plains New Jersey 07076 (US)
Inventor: Melillo, David G.
2637 Crest Lane
Scotch Plains New Jersey 07076 (US)
Inventor: Hoffman, Jacob M., Jr.
Pennbrooke Gardens, Apt. 17A Church Road
North Wales Pennsylvania 19454 (US)

(74) Representative: Ahner, Francis et al
CABINET REGIMBEAU 26, avenue Kléber
F-75116 Paris (FR)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 129 478 B1

Courier Press, Leamington Spa, England.

**Description**

Summary of the Invention

This invention relates to novel 2-sulfamoylbenzo[b]thiophenes which are useful in the reduction of elevated intraocular pressure. More particularly this invention relates to compounds having the structural formula:

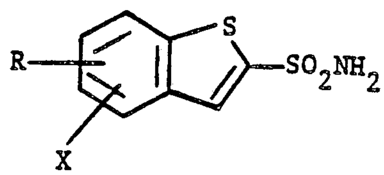

wherein R and X are as hereinafter defined, as well as the pharmaceutically and opthalmologically acceptable salts thereof. This invention also relates to pharmaceutical compositions for systemic and ophthalmic use employing a novel compound of this invention as active ingredient for the treatment of elevated intraocular pressure, especially when accompanied by pathological damage such as in the disease known as glaucoma.

Background of the Invention

Glaucoma is an ocular disorder associated with elevated intraocular pressures which are too high for normal function and may result in irreversible loss of visual function. If untreated, glaucoma may eventually lead to blindness. Ocular hypertension, i.e., the condition of elevated intraocular pressure without optic nerve head damage or characteristic glaucomatous visual field defects, is now believed by many ophthalmologists to represent the earliest phase of glaucoma.

Many of the drugs formerly used to treat glaucoma proved not entirely satisfactory. Indeed, few advances were made in the treatment of glaucoma since pilocarpine and physostigmine were introduced. Only recently have clinicians noted that many β-adrenergic blocking agents are effective in reducing intraocular pressure. While many of these agents are effective in reducing intraocular pressure, they also have other characteristics, e.g. membrane stabilizing activity, that are not acceptable for chronic ocular use. (S)-1-*tert*-Butylamino-3-[(4-morpholino-1,2,5-thiadiazol-3-yl)oxy]-2-propanol, a β-adrenergic blocking agent, was found to reduce intraocular pressure and to be devoid of many unwanted side effects associated with pilocarpine and, in addition, to possess advantages over many other β-adrenergic blocking agents, e.g. to be devoid of local anesthetic properties, to have a long duration of activity, and to display minimal tolerance.

Although pilocarpine, physostigmine and the β-blocking agents mentioned above reduce intraocular pressure, none of these drugs manifests its action by inhibiting the enzyme carbonic anhydrase and, thereby, impeding the contribution to aqueous humor formation made by the carbonic anhydrase pathway.

Agents referred to as carbonic anhydrase inhibitors, block or impede this inflow pathway by inhibiting the enzyme, carbonic anhydrase. While such carbonic anhydrase inhibitors are now used to treat intraocular pressure by oral, intravenous or other systemic routes, they thereby have the distinct disadvantage of inhibiting carbonic anhydrase throughout the entire body. Such a gross disruption of a basic enzyme system is justified only during an acute attack of alarmingly elevated intraocular pressure, or when no other agent is effective. Despite the desirability of directing the carbonic anhydrase inhibitor only to the desired ophthalmic target tissue, no topically effective carbonic anhydrase inhibitors are available for clinical use.

However, topically effective carbonic anhydrase inhibitors are reported in European Patent applications 0,070,239, 0,079,269 and 0 091 364. The compounds reported therein are 5 (and 6)-hydroxy-2-benzothiazolesulfonamides and acyl esters thereof. The state of the prior art can be further illustrated by the documents C. A. *81*, 105154d (1974) and DE—C—1 059 921 which respectively describe the synthesis of 2-sulfamoyl-3,5-dimethylbenzothiophene and of 3,5-sulfamoylthiophene derivatives, these last ones being known for their diuretic activity.

To be an effective and acceptable topical agent, an ophthalmic carbonic anhydrase inhibitor must not only penetrate the ophthalmic tissues to reach the active sites within the eye, but it must also be devoid of those side effects including irritation, allergic reaction and the like which would militate against long term administration.

Detailed Description of the Invention

The novel compounds of this invention have structural formula:

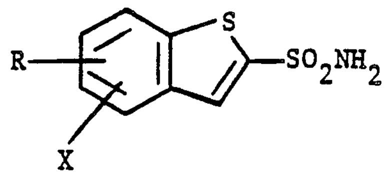

or an ophthalmologically or pharmaceutically acceptable salt thereof, wherein:

X is hydrogen, halo, such as chloro, bromo or fluoro, $C_{1-3}$alkyl, hydroxy or $C_{1-3}$alkoxy; and

R is:

1) hydroxy,

2) $R^1_\alpha$ wherein $R^1_\alpha$ is

a) $C_{1-18}$ alkyl either straight or branched chain substituted with one or more of

i) $C_{3-6}$ cycloalkyl,

ii) halo, such as chloro, bromo or fluoro,

iii) aryl, wherein the aryl group is carbocyclic such as phenyl or naphthyl, or heterocyclic such as pyridinyl, furanyl, pyrazinyl, morpholinyl, oxazolinyl, dioxolinonyl, imidazolyl, thienyl or the like and wherein the aryl group can be substituted with one or more of $C_{1-10}$ alkyl, halo, $C_{1-4}$ alkoxy, $C_{2-5}$ alkanoyl, or trifluoromethyl,

iv) hydroxy,

v) $C_{1-3}$ alkoxy,

vi) aryl-$C_{1-3}$alkoxy,

vii) $C_{1-4}$ alkoxy-$C_{1-3}$alkoxy,

viii) amino,

ix) ($C_{1-3}$ alkyl)amino,

x) di($C_{1-3}$ alkyl)amino,

xi) 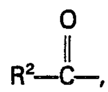

wherein $R^2$ is

1) HO—,

2) $M^+O^-$, wherein $M^+$ is a pharmaceutically acceptable cation such as that from an alkali metal, or an ammonium,

3) $C_{1-10}$ alkoxy,

4) $R^3R^4N$— wherein $R^3$ and $R^4$ are independently hydrogen, hydroxy, $C_{1-15}$ alkyl, or taken together form a 3—7 membered heterocycle with the nitrogen to which they are attached such as piperidino or pyrrolidino, or

xii) $C_{2-5}$ alkanoyl;

b) $C_{3-6}$ cycloalkyl,

c) $C_{1-18}$ alkyl-$C_{3-6}$ cycloalkyl,

d) aryl as previously defined,

e) $R^3R^4N$—

f) $C_{2-6}$ alkenyl,

g) aryl-$C_{2-6}$ alkenyl,

h) $C_{2-6}$ alkynyl, or

i) heterocyclyl of 5 or 6 members with one or two heteroatoms selected from O, N and S, such as tetrahydropyrrolyl, tetrahydrofuranyl, or imidazolidinyl,

3) $R^1_\alpha$—O—

4) 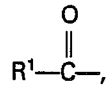

wherein $R^1$ is $R^1_\alpha$ or $C_{1-18}$alkyl,

5) 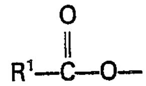

6) 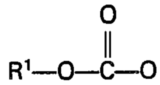

7) 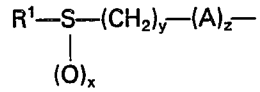

wherein x is 0—2; y is 0—3; z is 0 or 1; and A is a heteroatom such as S, O or N,

8) 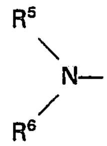

where $R^5$ and $R^6$ are independently:

a) hydrogen,

b) $C_{1-18}$ alkyl, either straight or branched chain,

c) $C_{3-6}$ cycloalkyl,

d) $C_{3-6}$ cycloalkyl-$C_{1-3}$ alkyl,

e) aryl-$C_{1-3}$ alkyl wherein the aryl group is either unsubstituted or substituted with one or more of chloro, bromo, fluoro, $C_{1-3}$ alkyl or $C_{1-3}$ alkoxy,

f) 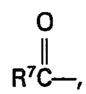 or 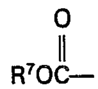

wherein $R^7$ is

i) $C_{1-18}$ alkyl, either straight or branched chain,

ii) aryl, either unsubstituted or substituted with one or more of chloro, bromo, fluoro, $C_{1-3}$ alkyl, or $C_{1-3}$ alkoxy,

iii) aryl-$C_{1-3}$ alkyl wherein the aryl group is either unsubstituted or substituted with one or more of chloro, bromo, fluoro, $C_{1-3}$ alkyl or $C_{1-3}$ alkoxy,

iv) amino-$C_{1-18}$ alkyl either straight or branched chain; or

(g) $R^5$ and $R^6$ if lower alkyl, are joined together directly or through a heteroatom selected from O or N to form a 5 or 6 membered heterocycle with the nitrogen to which they are attached such as pyrrolidine, piperidine, morpholine, or piperazine.

9) 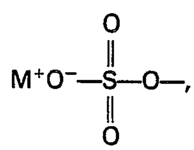

wherein $M^+$ is an ophthalmologically acceptable cation selected from sodium, potassium ammonium, tetra($C_{1-4}$alkyl)ammonium, pyridinium, imidazolium, pralidoxime, and thiamine

10) 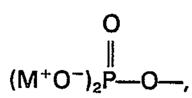

wherein $M^+$ is as previously defined;

11) 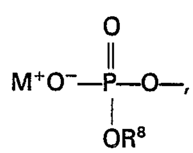

wherein $R^8$ is $C_{1-3}$ alkyl or phenyl-$C_{1-3}$ alkyl; or

12) 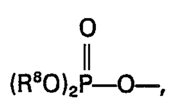

wherein $R^8$ is as previously defined, and the two may be the same or different; and R and X, joined together, represent methylenedioxy.

In the preferred embodiments of this invention, X is hydrogen and R is HO—,

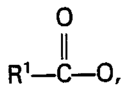, 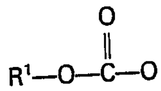 or $R^5R^6N$—,

especially wherein $R^1$ is $C_{1-18}$alkyl, and more especially $C_{1-4}$alkyl. It is also preferred that the substituent R be in the 5- or 6-position of the benzo[b]thiophene, especially the 6-position.

Preferred species of this invention are:

5(or 6)-hydroxy-2-sulfamoylbenzo[b]thiophene;
5(or 6)-(2-sulfamoylbenzo[b]thienyl) acetate;
5(or 6)-(2-sulfamoylbenzo[b]thienyl) 2,2-dimethylpropionate;
5(or 6)-(2-sulfamoylbenzo[b]thienyl) 2-methylpropionate.
5(or 6)-(2-sulfamoylbenzo[b]thienyl) 3-methoxycarbonylpropionate;

Representative carbonic anhydrase inhibitors of this invention include:

5(or 6)-hydroxy-2-sulfamoylbenzo[b]thiophene;
5(or 6)-(2-sulfamoylbenzo[b]thienyl) benzoate;
5(or 6)-(2-sulfamoylbenzo[b]thienyl) propionate;
5(or 6)-(2-sulfamoylbenzo[b]thienyl) butyrate;
5(or 6)-(2-sulfamoylbenzo[b]thienyl) 2,2-dimethylpropionate;
5(or 6)-(2-sulfamoylbenzo[b]thienyl) octanoate
5(or 6)-(2-sulfamoylbenzo[b]thienyl) dodecanoate;
5(or 6)-(2-sulfamoylbenzo[b]thienyl) 4,4-dimethylcyclohexane carboxylate;
5(or 6)-(2-sulfamoylbenzo[b]thienyl) 3-chloro-2,2-dimethylpropionate;
5(or 6)-(2-sulfamoylbenzo[b]thienyl) 4-methylbenzoate;
5(or 6)-(2-sulfamoylbenzo[b]thienyl) 4-chlorobenzoate;
5(or 6)-(2-sulfamoylbenzo[b]thienyl) 4-methoxybenzoate;
5(or 6)-(2-sulfamoylbenzo[b]thienyl) 2-(4-chlorophenyl)acetate;
5(or 6)-(2-sulfamoylbenzo[b]thienyl) 3-(4-ethylphenyl)propionate;
5(or 6)-(2-sulfamoylbenzo[b]thienyl) 3-hydroxy-2,2-dimethylpropionate;
5(or 6)-(2-sulfamoylbenzo[b]thienyl) 4-aminobutyrate HCl;
5(or 6)-(2-sulfamoylbenzo[b]thienyl) acrylate;
5(or 6)-(2-sulfamoylbenzo[b]thienyl) crotonate;
5(or 6)-(2-sulfamoylbenzo[b]thienyl) propiolate;
5(or 6)-(2-sulfamoylbenzo[b]thienyl) 3-phenyl-2-propenoate;
5(or 6)-(2-sulfamoylbenzo[b]thienyl) cyclopentaneacetate;
5(or 6)-(2-sulfamoylbenzo[b]thienyl) phenylacetate;
5(or 6)-(2-sulfamoylbenzo[b]thienyl) cyclohexanecarboxylate;
5(or 6)-(2-sulfamoylbenzo[b]thienyl) acetate;
5(or 6)-(2-sulfamoylbenzo[b]thienyl) 3-carboxypropionate;
5(or 6)-(2-sulfamoylbenzo[b]thienyl) 3-carboxypropionate, sodium salt;
5(or 6)-(2-sulfamoylbenzo[b]thienyl) 2-ethoxycarbonylacetate;
5(or 6)-(2-sulfamoylbenzo[b]thienyl) acetoacetate;
5(or 6)-(2-sulfamoylbenzo[b]thienyl) 3-aminocarbonylpropionate;
5(or 6)-(2-sulfamoylbenzo[b]thienyl) N-acetylpiperidine-4-carboxylate;
5(or 6)-(2-sulfamoylbenzo[b]thienyl) nicotinoate;
5(or 6)-(2-sulfamoylbenzo[b]thienyl) 1-methyl-4-imidazolylacetate;
5(or 6)-(2-sulfamoylbenzo[b]thienyl) 2-methoxybutyrate;
5(or 6)-(2-sulfamoylbenzo[b]thienyl) 2-methoxysuccinate;
5(or 6)-(2-sulfamoyl-benzo[b]thienyl) phenyl carbonate;
5(or 6)-(2-sulfamoyl-benzo[b]thienyl) ethyl carbonate;
5(or 6)-(2-sulfamoyl-benzo[b]thienyl) propyl carbonate;
5(or 6)-(2-sulfamoyl-benzo[b]thienyl) 1,1-dimethylethyl carbonate;
5(or 6)-(2-sulfamoyl-benzo[b]thienyl) *n*-heptyl carbonate;
5(or 6)-(2-sulfamoyl-benzo[b]thienyl) undecanyl carbonate;
5(or 6)-(2-sulfamoyl-benzo[b]thienyl) 4,4-dimethylcyclohexyl carbonate;
5(or 6)-(2-sulfamoyl-benzo[b]thienyl) 2-chloro-1,1-dimethylethyl carbonate;
5(or 6)-(2-sulfamoyl-benzo[b]thienyl) 4-methylphenyl carbonate;
5(or 6)-(2-sulfamoyl-benzo[b]thienyl) 4-chlorophenyl carbonate;
5(or 6)-(2-sulfamoyl-benzo[b]thienyl) 4-methoxyphenyl carbonate;
5(or 6)-(2-sulfamoyl-benzo[b]thienyl)-4-chlorobenzyl carbonate;
5(or 6)-(2-sulfamoyl-benzo[b]thienyl) 2-(4-ethylphenyl)ethyl carbonate;
5(or 6)-(2-sulfamoyl-benzo[b]thienyl) 2-methylpropyl carbonate;
5(or 6)-(2-sulfamoyl-benzo[b]thienyl) allyl carbonate;
5(or 6)-(2-sulfamoyl-benzo[b]thienyl) 2-propynyl carbonate;
5(or 6)-(2-sulfamoyl-benzo[b]thienyl) 3-phenyl-2-propenyl carbonate;
5(or 6)-(2-sulfamoyl-benzo[b]thienyl) cyclopentylmethyl carbonate;

5(or 6)-(2-sulfamoyl-benzo[b]thienyl) benzyl carbonate;
5(or 6)-(2-sulfamoyl-benzo[b]thienyl) cyclohexyl carbonate;
5(or 6)-(2-sulfamoyl-benzo[b]thienyl) methyl carbonate;
5(or 6)-amino-2-sulfamoylbenzo[b]thiophene;
5(or 6)-ethylamino-2-sulfamoylbenzo[b]thiophene;
5(or 6)-diethylamino-2-sulfamoylbenzo[b]thiophene;
5(or 6)-[(1-methylethyl)amino]-2-sulfamoylbenzo[b]thiophene;
5(or 6)-[N-ethyl-N-(2-propyl)amino]-2-sulfamoylbenzo[b]thiophene;
5(or 6)-[(N-benzyl-N-ethyl)amino]-2-sulfamoylbenzo[b]thiophene;
5(or 6)-cyclohexylamino-2-sulfamoylbenzo[b]thiophene;
5(or 6)-cyclopentylmethylamino-2-sulfamoylbenzo[b]thiophene;
5(or 6)-pivaloylamino-2-sulfamoylbenzo[b]thiophene;
5(or 6)-[(N-methyl-N-pivaloyl)amino]-2-sulfamoylbenzo[b]thiophene;
5(or 6)-pivaloyloxycarbonylamino-2-sulfamoylbenzo[b]thiophene;
5(or 6)-acetylamino-2-sulfamoylbenzo[b]thiophene;
5(or 6)-butyrylamino-2-sulfamoylbenzo[b]thiophene;
5(or 6)-benzoylamino-2-sulfamoylbenzo[b]thiophene;
5(or 6)-[(4-methylbenzoyl)amino]-2-sulfamoylbenzo[b]thiophene;
5(or 6)-[(4-fluorobenzoyl)amino]-2-sulfamoylbenzo[b]thiophene;
5(or 6)-(4-methoxybenzoyl)amino-2-sulfamoylbenzo[b]thiophene;
5(or 6)-nicotinoylamino-2-sulfamoylbenzo[b]thiophene;
5(or 6)-thienylcarbonylamino-2-sulfamoylbenzo[b]thiophene;
5(or 6)-alanylamino)-2-sulfamoylbenzo[b]thiophene;
5(or 6)-(N-ethyl-N-hydroxy)amino-2-sulfamoylbenzo[b]thiophene;
5(or 6)-(N-ethyl-N-methoxy)amino-2-sulfamoylbenzo[b]thiophene;
5(or 6)-(1-morpholino)-2-sulfamoylbenzo[b]thiophene;
2-sulfamoylbenzo[b]thiophene-6(or 5)-acetic acid;
2-sulfamoylbenzo[b]thiophene-6(or 5)-propionic acid;
5(or 6)-(2-hydroxyethyl)-2-sulfamoylbenzo[b]thiophene;
5(or 6)-(2,3-dihydroxypropoxy)-2-sulfamoylbenzo[b]thiophene;
5(or 6)-(dioxolin-2-one-4-ylmethoxy)-2-sulfamoylbenzo[b]thiophene;
5(or 6)-(5-oxazolinylmethoxy)-2-sulfamoylbenzo[b]thiophene;
5(or 6)-(1-methylimidazol-4-yloxy)-2-sulfamoylbenzo[b]thiophene;
5(or 6)-furfuryl-2-sulfamoylbenzo[b]thiophene;
5(or 6)-(2-morpholinylethyl)-2-sulfamoylbenzo[b]thiophene;
5(or 6)-morpholinylmethyl-2-sulfamoylbenzo[b]thiophene;
5(or 6)-hydroxymethyl-2-sulfamoylbenzo[b]thiophene;
5(or 6)-(acetyloxymethyl-2-sulfamoylbenzo[b]thiophene;
5(or 6)-(2-acetyloxyethyl)-2-sulfamoylbenzo[b]thiophene;
5(or 6)-benzoyl-2-sulfamoylbenzo[b]thiophene;
5(or 6)-propionyl-2-sulfamoylbenzo[b]thiophene;
5(or 6)-butyryl-2-sulfamoylbenzo[b]thiophene;
5(or 6)-(2,2-dimethylpropionyl)-2-sulfamoylbenzo[b]thiophene;
5(or 6)-octanoyl-2-sulfamoylbenzo[b]thiophene;
5(or 6)-dodecanoyl-2-sulfamoylbenzo[b]thiophene;
5(or 6)-(4,4-dimethylcyclohexanecarbonyl)-2-sulfamoylbenzo[b]thiophene;
5(or 6)-(3-chloro-2,2-dimethylpropionyl)-2-sulfamoylbenzo[b]thiophene;
5(or 6)-(2-methylbenzoyl)-2-sulfamoylbenzo[b]thiophene;
5(or 6)-(4-chlorobenzoyl)-2-sulfamoylbenzo[b]thiophene;
5(or 6)-(4-methoxybenzoyl)-2-sulfamoylbenzo[b]thiophene;
5(or 6)-(4-chlorophenylacetyl)-2-sulfamoylbenzo[b]thiophene;
5(or 6)-[3-(4-ethylphenyl)propionyl)]-2-sulfamoylbenzo[b]thiophene;
5(or 6)-(3-hydroxy-2,2-dimethylpropionyl)-2-sulfamoylbenzo[b]thiophene;
5(or 6)-(4-aminobutyryl)-2-sulfamoylbenzo[b]thiophene;
5(or 6)-(acryloyl)-2-sulfamoylbenzo[b]thiophene;
5(or 6)-(crotonyl)-2-sulfamoylbenzo[b]thiophene;
5(or 6)-propiolyl-2-sulfamoylbenzo[b]thiophene;
5(or 6)-(3-phenyl-2-propenoyl)-2-sulfamoylbenzo[b]thiophene;
5(or 6)-cyclopentaneacetyl-2-sulfamoylbenzo[b]thiophene;
5(or 6)-phenylacetyl-2-sulfamoylbenzo[b]thiophene;
5(or 6)-cyclohexanecarbonyl-2-sulfamoylbenzo[b]thiophene;
5(or 6)-acetyl-2-sulfamoylbenzo[b]thiophene;
5(or 6)-(3-carboxypropionyl)-2-sulfamoylbenzo[b]thiophene;
5(or 6)-ethoxycarbonylacetyl-2-sulfamoylbenzo[b]thiophene;
5(or 6)-acetoacetyl-2-sulfamoylbenzo[b]thiophene;

5(or 6)-(3-aminocarbonylpropionyl)-2-sulfamoylbenzo[b]thiophene;
5(or 6)-(N-acetylpiperidine-4-carbonyl)-2-sulfamoylbenzo[b]thiophene;
5(or 6)-(4-imidazolyl)-2-sulfamoylbenzo[b]thiophene;
5(or 6)-pyrazinyl-2-sulfamoylbenzo[b]thiophene;
5(or 6)-(4-imidazolylcarbonyl)-2-sulfamoylbenzo[b]thiophene;
5(or 6)-(4-imidazolylsulfonyl)-2-sulfamoylbenzo[b]thiophene;
5(or 6)-(trifluoromethylsulfonyl)-2-sulfamoylbenzo[b]thiophene;
5-[N-(tertbutoxycarbonyl)glycyloxy]-2-sulfamoylbenzo[b]thiophene, m.p. 168—170°C (dec);
5-(2-hydroxyethoxy)-2-sulfamoylbenzo[b]thiophene, m.p. 127—128°C;
5-(glycyloxy)-2-sulfamoylbenzo[b]thiophene hydrochloride, m.p. 223—233°C;
5-(N,N-Dimethylcarbamoylmethoxy)-2-sulfamoylbenzo[b]thiophene, m.p. 259—261°C;
5-(L-prolyloxy)-2-sulfamoylbenzo[b]thiophene hydrochloride, m.p. 229—231°C;
5-(2-dimethylaminoethoxy)-2-sulfamoylbenzo[b]thiophene, m.p. 204.5—205.5°C;
5-(2-methoxyethoxy)-2-sulfamoylbenzo[b]thiophene, m.p. 153—154°C;
5-[2-(isopropoxy)ethoxymethyl]-2-sulfamoylbenzo[b]thiophene, m.p. 65—69°C;
5-(2-dimethylaminoethyl)-2-sulfamoylbenzo[b]thiophene, m.p. 208—209°C;
6-(1-triphenylmethylimidazol-4-yl)methoxy-2-sulfamoylbenzo[b]thiophene, m.p. 238—239°C;
6-(1-imidazol-4-yl)-2-sulfamoylbenzo[b]thiophene hydrochloride, m.p. 221—223°C.

6-Methoxy-2-sulfamoylbenzo[b]thiophene, a novel compound and a key intermediate for many of the other novel compounds of this invention, is obtained by reacting bromoacetaldehyde diethyl acetal in the presence of a base with at least a molar equivalent of *m*-methoxybenzenethiol in a suitable inert solvent, preferably acetone, at a temperature extending to reflux. After reaction is essentially complete the 1-[(2,2-diethoxy)ethylthio]-3-methoxybenzene obtained is collected and suitably extracted, washed and dried. The dried product is then slowly added to at least a molar equivalent of the catalyst boron trifluoride.etherate in an inert solvent such as chloroform at a temperature extending to reflux until reaction is essentially completed. Preferably the reaction is conducted under an inert atmosphere such as nitrogen. A mixture of 6-methoxybenzo[b]thiophene and 4-methoxybenzo[b]thiophene in an approximate ratio of 10:1 is obtained upon evaporation of the solvent. The mixture is treated with at least a molar equivalent of n-butyl lithium in an inert solvent such as tetrahydrofuran at a reduced temperature, preferably below −10°C, most suitably at −70°C. Sulfur dioxide gas is then passed over the solution at a rate such that the reaction mixture does not exceed a temperature of −10°C. After the reaction is complete, the solvents are removed and the product reacted with at least a molar equivalent of N-chlorosuccinimide at reduced temperature, generally 0°C or less. The mixture of 4- and 6-methoxybenzo[b]thiophene-2-sulfonyl chlorides that is produced is treated with concentrated aqueous ammonia. The resulting mixture of 4- and 6-methoxy-2-sulfamoylbenzo[b]thiophenes is then collected and the isomers are separated by fractional crystallization. Each is further purified by reprecipitation from aqueous base by treatment with acid and/or recrystallization from an appropriate solvent.

The novel process for preparing the compounds wherein R is hydroxy comprises treatment of a methoxy-2-sulfamoylbenzo[b]thiophene with at least an equimolar amount of pyridine hydrochloride at a temperature from about the fusion point to about 200°C, and preferably from about 180°—190°C for from about 1 to 4 hours, preferably about 2 hours, until the reaction is substantially complete.

The novel process to prepare those compounds wherein R is

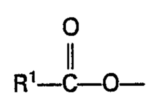

is represented by the following reaction scheme:

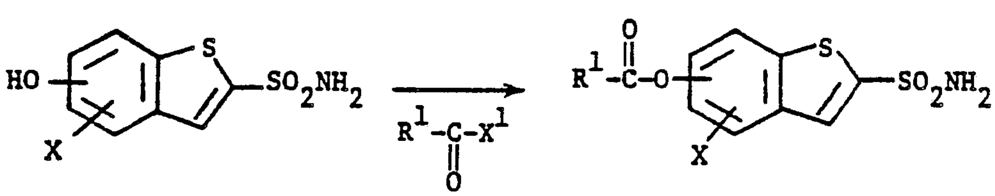

where $R^1$ has the meanings hereinbefore designated, and $X^1$ is chloro, bromo, iodo,

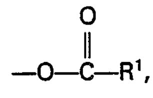, 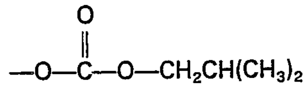 or 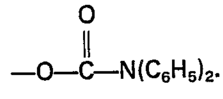.

Generally equimolar amounts of the hydroxy-2-sulfamoylbenzo[b]thiophene and

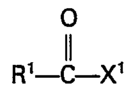

are employed, although use of an excess of the more readily available reactant is satisfactory.

The reaction is conducted in a suitable, inert solvent such as acetone, dimethylformamide, pyridine, ethyl acetate, tetrahydrofuran or benzene and the like with at least an equimolar amount of a hydrohalide acceptor when the acylating agent is an acyl halide or with a carboxylic acid acceptor when the acylating agent is an acid anhydride. Bases such as triethylamine, pyridine and the like may be employed for this purpose.

The reaction may be conducted with or without a catalyst at temperatures of from 0°C to the boiling point of the solvent used but preferably from about 15°C to 50°C.

When a catalyst is employed, a 4,4-dialkylaminopyridine such as 4-dimethylaminopyridine or 4-pyrrolidinopyridine is preferred.

The compounds wherein R is

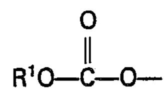

of this invention are most suitably prepared by reacting a compound

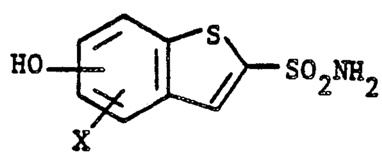

with an appropriate haloformate, particularly a chloroformate of the formula:

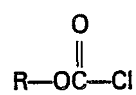

or a *bis* carbonate of the formula:

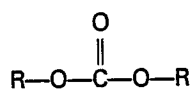

The reaction is conducted in a suitable solvent such as dimethylformamide, pyridine, acetone, ethyl acetate, tetrahydrofuran or benzene and the like with at least an equimolar amount of a hydrohalide acceptor. Bases such as triethylamine, pyridine and the like may be employed for this purpose.

The reaction may be conducted with or without a catalyst at temperatures of from 0°C to the boiling point of the solvent used but preferably from 15°C to 50°C.

When a catalyst is employed, triethylamine or a 4,4-dialkylaminopyridine such as 4-dimethylaminopyridine or 4-pyrrolidinopyridine is preferred.

In the novel process of this invention for preparing the ethers of hydroxy-2-sulfamoyl-benzo[b]thiophenes, the hydroxy compound is treated with an "alkylating" agent of formula $R^1—X^2$ wherein $X^2$ is a halide such as chloride, bromide or iodide, or other good leaving group such as mesylate, tosylate or benzenesulfonate in a suitable solvent such as dimethyl formamide, hexamethyl phosphoramide, or the like in the presence of a base such as an alkali metal alkoxide, preferably sodium methoxide, at about 0°C to 35°C, usually about room temperature for about 10 to 30 hours.

An alternate synthesis of ethers comprises protecting the sulfonamide group as an N,N-disubstituted formamidine which is removed after formation of the desired ether. The formamidine derivative is prepared by adding, for example, N,N-dimethylformamide dimethylacetal to a suspension of the hydroxy-2-sulfamoylbenzo[b]thiophene in an inert organic solvent such as acetonitrile at about −10 to +35°C, preferably room temperature for about 0.5 to about 3 hours.

The ethers are then readily prepared by treating the hydroxy compound with the "alkylating" agent, $R^1—X^2$, in a solvent such as dimethyl sulfoxide, preferably in the presence of an acid acceptor such as potassium carbonate or the like, pyridine or the like or basic ion exchange resin. The reaction is conducted at about 25 to 100°C, preferably about 60°C, for about 10 to 36 hours, preferably about 18 hours.

The protecting group is then removed from the sulfonamide by treating the compound with dilute alkali such as 2*N* sodium hydroxide at about 20 to 60°C, preferably about 40°C for about 0.5 to 3 hours, preferably about 1 hour. Also, 6*N* HCl at about 100°C for 2—5 hours can be used to effect the desired deprotection.

The novel compounds of this invention with no substituent, i.e. R=H and those carrying fairly stable

substituents such as wherein R is $R^1$ and $R^1$ is alkyl, cycloalkyl, cycloalkyl-alkyl, alkylcycloalkyl, alkoxyalkyl, alkenyl; R is $R^1$—O— wherein $R^1$ is as defined above; R is $R^5R^6$—N— wherein $R^5$ and $R^6$ are not hydrogen are conveniently prepared by formation of the sulfonamide group on the intact benzo[b]thiophene moiety. This is accomplished by the procedure described earlier for preparation of 6-methoxy-2-sulfamoylbenzo[b]thiophene.

The *O*-sulfates of this invention are prepared by reacting an hydroxy-2-sulfamoylbenzo[b]thiophene with sulfamic acid in pyridine at elevated temperatures (about 50° to the boiling point) for about 18 to 48 hours to provide the ammonium salt followed, if desired, by titration with hydroxides of the formula MOH to provide the other salts.

Similarly the *O*-phosphates of this invention are prepared by treatment of a hydroxy-2-sulfamoylbenzo[b]thiophene with phosphorus oxychloride, an alkyl dichlorophosphate or a dialkyl chlorophosphate in pyridine or similar basic solvent at about −5 to +5°C for about 0.25 to 1.0 hour.

Example 1

6-Hydroxy-2-sulfamoylbenzo[b]thiophene

*Step A:* Preparation of 1-[(2,2-diethoxy)ethylthio]-3-methoxybenzene

Bromoacetaldehyde diethyl acetal (16.5 ml, 0.11 mol) was added dropwise to a mixture of m-methoxybenzenethiol (15.0 ml, 0.12 mol) and $K_2CO_3$ (16.6 g, 0.12 mol) in acetone (150 ml) at room temperature. The reaction mixture was stirred for 16 hours and then filtered. The solid was washed with acetone, and the combined filtrate and washes were concentrated *in vacuo*. The residue was diluted with $H_2O$ and extracted with $Et_2O$. The $Et_2O$ extracts were washed with 0.5 *M* KOH, $H_2O$, and brine, dried over $Na_2SO_4$, filtered, and concentrated *in vacuo* to give 27.4 g of 1-[(2,2-diethoxy)ethylthio]-3-methoxybenzene as a dark yellow oil. 'H NMR, δ: ($CDCl_3$): 1.18 (6H, t, —$OCH_2CH_3$); 3.13 (2H, d, —SC*H*—); 3.43—3.73 (4H, m, —$OCH_2CH_3$); 3.77 (3H, s, —$OCH_3$); 4.67 (1H, t, —$SCH_2CH$—); 6.60—7.27 (4H, m, aromatics).

*Step B:* Preparation of 6-methoxybenzo[b]thiophene

A solution of 1-[(2,2-diethoxy)ethylthio]-3-methoxybenzene (13.0 g, 0.051 mmol) in $CH_2Cl_2$ (100 ml) was added dropwise to a solution of $BF_3.Et_2O$ (6.7 ml, 0.054 mol) in $CH_2Cl_2$ (1000 ml) at room temperature under a nitrogen atmosphere. The reaction mixture was stirred for 0.5 hours, treated with aqueous $NaHCO_3$ solution, and stirred until both phases were clear. The $CH_2Cl_2$ layer was separated, and the aqueous layer was extracted with $CH_2Cl_2$. The combined organic extracts were dried over $Na_2SO_4$, filtered, and concentrated *in vacuo* to give 8.68 g of an approximately 10:1 mixture of 6- and 4-methoxybenzo[b]thiophene as a dark brown oil. Purification by vacuum distillation (bp = 65—7°C at 0.3 mmHg) gave 5.58 g of pale yellow oil. Major isomer: 'H NMR, δ: ($CDCl_3$): 3.85 (3H, s, —$OCH_3$); 6.98 (1H, dd, J=4.5, $H_5$); 7.23 (2H, s, $H_2$, $H_3$); 7.35 (1H, d, J=1.5, $H_7$); 7.68 (1H, d, J=4.5, $H_4$).

*Step C:* Preparation of 6-methoxy-2-sulfamoylbenzo[b]thiophene

A solution of the mixture of 6- and 4-methoxybenzo[b]thiophene (6.04 g, 37 mmol) in 40 ml of dry THF was treated with n-butyl lithium (27 ml of a 1.64 M solution in hexane, 44 mmol) at −20°C. After stirring for 1 hour at −20°C, $SO_2$ gas was passed over the surface of the solution at such a rate to maintain the internal temperature of the reaction at −10°C. The reaction is judged complete when the temperature falls and the reaction mixture has changed from red to a yellow suspension. The solvents were evaporated *in vacuo* and the residue dissolved in 75 ml saturated $NaHCO_3$ solution. After cooling to 0°C, N-chlorosuccinimide (6.5 g, 48 mmol) was added in four portions over 5 minutes. A solid precipitate was collected by filtration. The aqueous filtrate was extracted with ethyl acetate and the organic phase washed with brine and dried. The solvent was evaporated to yield an oil. The oil and the initial solid precipitate were combined in a small volume of acetone and added to a solution of 25 ml of $NH_4OH$ in 100 ml of acetone. After 30 minutes, the solvents were evaporated and the residue was partitioned between 100 ml 1N KOH and ether (100 ml). The ether layer was extracted again with 1N KOH (2 × 50 ml). The combined basic extract was neutralized with concentrated HCl. The precipitate was extracted into ethyl acetate. The organic extract was washed with brine and dried. Evaporation of the solvent gave 8.11 g of a tan solid m.p. 155—160°C. Recrystallization from dichloroethane affords the pure 6-methoxy isomer m.p. 166—167°C. 'H—NMR δ (DMSO-$d_6$): 7.85 (1H, dd, 5=9), 7.77 (1H, s), 7.72 (2H, br s), 7.60 (1H, dd, J=2), 7.05 (1H, dd, J=9 and 2), 4.81 (3H, s).

| | | | |
|---|---|---|---|
| Analysis Calculated for $C_9H_9NO_3S_2$: | C, 44.43; | H, 3.73; | N, 5.76 |
| Found: | C, 44.61; | H, 3.75; | N, 5.82. |

Concentration of the mother liquors from the recrystallization afforded a second crop of crystals which was largely 4-methoxy-2-sulfamoylbenzo[b]thiophene. A second recrystallization of this material gave the pure compound, m.p. 180—182°C. 'H—NMR δ: (DMSO-$d_6$): 7.83 (1H, brs), 7.80 (1H, s), 7.60 (1H, d), 7.44 (1H, t), 6.95 (1H, d), 3.92 (3H, s).

| | | | |
|---|---|---|---|
| Analysis calculated for $C_9H_9NO_3S_2$: | C, 44.43; | H, 3.73; | N, 5.76 |
| Found: | C, 44.31; | H, 3.62; | N, 5.73. |

Employing the procedures substantially as described in Example 1, Steps A, B and C, except for the separation of isomers in Step C, and starting with 3,4-methylenedioxybenzenethiol, there is produced in sequence:

1-[(2,2-diethoxy)ethylthio]-3,4-methylenedioxybenzene;

5,6-methylenedioxybenzo[b]thiophene; and
5,6-methylenedioxy-2-sulfamoylbenzo[b]thiophene.

*Step D:* Preparation of 6-Hydroxy-2-sulfamoylbenzo[b]thiophene

A mixture of 3.6 g of 6-methoxy-2-sulfamoylbenzo[b]thiophene and 37 g of pyridine hydrochloride was heated to 180—190°C for 2 hours. After cooling, the solid was dissolved in 200 ml water. The product was isolated by extraction with ethyl acetate (3 × 75 ml). The organic extract was washed with dilute HCl and brine, and dried over $Na_2SO_4$. The solvent was evaporated yielding 3.2 g of a brown solid. The solid was dissolved in 200 ml acetone and treated with 1.3 g of decolorizing carbon. The solution was filtered and the solvent evaporated to yield 2.8 g of a yellow solid. The solid was dissolved in a minimal volume of ethyl acetate and triturated with hexane. A white solid m.p. 211—214°C, weighing 1.70 g was obtained. 'H—NMR δ (DMSO-$d_6$): 10.02 (1H, br s) 7.81 (1H, d, J=9), 7.75 (1H, s), 7.71 (2H, s), 7.33 (1H, d, J=2); 6.96 (1H, dd, J=9 and 2). Further recrystallization from water or nitromethane provides material of greater than 99.9% purity, m.p. 215—216°C.

Analysis: Calculated for $C_8H_7NO_3S_2$: C, 41.91; H, 3.08; N, 6.11.
Found: C, 41.87; H, 2.91; N, 6.17.

Demethylation of the 4-methoxy isomer occurs under the same conditions. A single recrystallization from water gave tan needles m.p. 212—213. 'H—NMR, δ (acetone $d_6$): 9.3 (1H, br s), 7.96 (1H, s), 7.4 (2H, m), 6.9 (2H, br s and 1H, dd).

Analysis calculated for $C_8H_7NO_3S_2$: C, 41.91; H, 3.08; N, 6.11.
Found: C, 41.84; H, 2.94; N, 6.21.

Employing the procedures substantially as described in Example 1, Steps A, B, C and D, but substituting for the m-methoxybenzenethiol used in Step A thereof, an approximately equimolar amount of:

a) 2,4-dimethoxybenzenethiol;
b) 2,5-dimethoxybenzenethiol;
c) 3,4-dimethoxybenzenethiol;
d) 3-methyl-4-methoxybenzenethiol; and
e) 3-methoxy-4-methylbenzenethiol; there are produced

*in Step B:*

a) 5,7-dimethoxybenzo[b]thiophene;
b) 4,7-dimethoxybenzo[b]thiophene;
c) 5,6-dimethoxybenzo[b]thiophene and 4,5-dimethoxybenzo[b]thiophene;
d) 5-methoxy-6-methylbenzo[b]thiophene; and
e) 5-methyl-6-methoxybenzo[b]thiophene;

*in Step C:*

a) 5,7-dimethoxy-2-sulfamoylbenzo[b]thiophene;
b) 4,7-dimethoxy-2-sulfamoylbenzo[b]thiophene;
c) 5,6-dimethoxy-2-sulfamoylbenzo[b]thiophene and 4,5-dimethoxy-2-sulfamoylbenzo[b]thiophene;
d) 5-methoxy-6-methyl-2-sulfamoylbenzo[b]thiophene; and
e) 5-methyl-6-methoxy-2-sulfamoylbenzo[b]thiophene; and,

*in Step D:*

a) 5,7-dihydroxy-2-sulfamoylbenzo[b]thiophene;
b) 4,7-dihydroxy-2-sulfamoylbenzo[b]thiophene;
c) 5,6-dihydroxy-2-sulfamoylbenzo[b]thiophene and 4,5-dihydroxy-2-sulfamoylbenzo[b]thiophene;
d) 5-hydroxy-6-methyl-2-sulfamoylbenzo[b]thiophene; and
e) 6-hydroxy-5-methyl-2-sulfamoylbenzo[b]thiophene.

Example 2

6-(2-Sulfamoylbenzo[b]thienyl) 2,2-Dimethylpropionate

To a solution of 6-hydroxy-2-sulfamoyl benzo[b]thiophene (125 mg, 0.54 mmol) in 4 ml of THF was added 0.16 ml of $Et_3N$ and 0.24 ml of pivalic anhydride (1.18 mmol). The reaction mixture was stirred for 18 hours at room temperature. The mixture was poured into $NaHCO_3$ solution and extracted with ethyl acetate. The organic extract was washed with brine and dried ($Na_2SO_4$). After treating with charcoal and filtering the product was obtained by trituration with hexane as white crystals, m.p. 165—167°C. The yield was 58 mg. 'H—NMR, δ ($CDCl_3$): 7.92 (1H, d, J=9); 7.91 (1H, s); 7.58 (1H, d, 5=1); 7.2 (1H, dd, J=9 and 2); 1.37 (9H, s).

Analysis Calculated for $C_{13}H_{15}NO_4S_2$: C, 49.82; H, 4.82; N, 4.47.
Found: C, 49.63; H, 4.86; N, 4.52.

Example 3

6-(2-Sulfamoylbenzo[b]thienyl) 2-Methylpropionate

To a stirred solution of 2.00 g of 6-hydroxy-2-sulfamoylbenzo[b]thiophene in 20 ml of dry acetone was added 1.3 ml of $Et_3N$ (9.3 mmol) followed by 0.96 ml of isobutyryl chloride (8.8 mmol) dropwise, with stirring at 0°. After twenty minutes the suspension was partitioned between EtOAc and $NaHCO_3$ solution. The organic phase was washed with brine and dried ($Na_2SO_4$). Evaporation of the solvent gave 2.77 g of a white solid. Recrystallization from ethyl acetate/hexane gave 1.32 g of white crystals, m.p. 164—165°C. $^1$H—NMR, δ (DMSO): 8.05 (1H, d, J=9); 7.93 (1H, s); 7.87 (3H, m); 7.25 (1H, dd, J=9 and 2); 3.84 (1H, m); 1.23 (6H, d, J=7).

Analysis Calculated for $C_{12}H_{13}NO_4S_2$: C, 48.15; H, 4.30; N, 4.68.
Found: C, 47.99; H, 4.33; N, 4.79.

Example 4

6-(2-Sulfamoylbenzo[b]thienyl) Acetate

A solution of 6-hydroxy-2-benzothiophenesulfonamide (2.5 g, 0.0109 mole) in acetone (45 ml) at −5° was treated with triethylamine (1.2133 g; 0.01199 mole). Then a solution of acetyl chloride (0.9413 g, 0.01199 mole) in acetone (5 ml) was added, dropwise, during 15 minutes at −5°C. After 15 minutes, the reaction mixture was filtered to remove the precipitated triethylamine hydrochloride. The filtrate was evaporated *in vacuo* to give an off white solid. This solid was dissolved in ethyl acetate, washed with a small quantity of water and saturated sodium chloride solution and dried over sodium sulfate. Removal of solvent *in vacuo* gave an off-white solid. The yield was 2.98 g, m.p. 120—133°C. Recrystallization from 1,2-dichloroethane gave the title compound as white prisms, m.p. 150—151°C.

Analysis Calculated for $C_{10}H_9NO_4S_2$: C, 44.27; H, 3.34; N, 5.16.
Found: C, 44.00; H, 3.48; N, 5.19.

Employing the procedure described in the above Examples 2—4, but substituting for the acid chloride starting material used therein, a substantially equivalent molar amount of the acid chlorides described in Table I, there are produced the acyloxy-2-sulfamoylbenzo[b]thiophenes also described in Table I in accordance with the following reaction scheme:

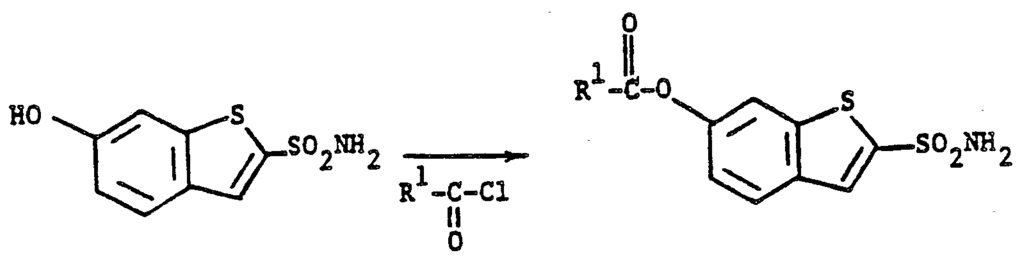

TABLE I

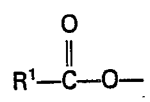

propionate (product m.p. 154—157°C)
cyclohexane carboxylate
cyclopentane carboxylate
octanoate
4,4-dimethylcyclohexane carboxylate
3-chloro-2,2-dimethyl propionate
benzoate
nicotinate (product m.p. 216—217°C)
4-methyl benzoate
4-chlorobenzoate
4-methoxybenzoate
3-phenylpropionate
4-chlorophenyl acetate
3-(4-ethylphenyl)propionate
3-hydroxy-2,2-dimethylpropionate
3-dimethylamino-2,2-dimethylpropionate
acrylate
crotonate
propiolate
cinnamate
3-methoxycarbonylpropionate (product m.p. 133—135°C)
3-ethoxycarbonylpropionate (product m.p. 111—113°C)

TABLE I (cont'd.)

| 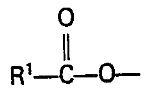 |
|---|
| N-acetylpiperidine-4-carboxylate (product m.p. 170.5—172) |
| methoxyacetate (product m.p. 140—142) |
| succinate[(1)] |
| 2-methoxy-2-methylpropionate[(2)] |
| 2-thienylcarboxylate |

[(1)] acylating reagent is succinic anhydride
[(2)] acylating reagent is 2-methoxy-2-methylpropionic N,N-diphenyl carbamic anhydride

### Example 5
### 5-Hydroxy-2-sulfamoylbenzo[b]thiophene

*Step A:* Preparation of 5-Methoxy-2-sulfamoylbenzo[b]thiophene

A solution of 5-methoxybenzo[b]thiophene (9.50 g, 0.058 m), in dry tetrahydrofuran (60 ml) was cooled to −20°C under nitrogen and 1.6 M n-butyl lithium in hexane (40 ml, 0.064 m) was added over 20 minutes, maintaining the temperature at −20 to −15°C. After stirring at −20°C for 1 hour, sulfur dioxide was passed over the surface of the reaction mixture for 45 minutes while keeping the temperature at −15 to −5°C while stirring vigorously. Ether (100 ml) was added and the precipitate was collected and dried under vacuum at ambient temperature. The solid was suspended in methylene chloride (105 ml) and the mixture was cooled to 0°C and stirred while N-chlorosuccinimide (18.55 g, 0.064 m) was added over 10 minutes. After stirring at ambient temperature for 2 hours, the mixture was filtered and the solid was washed with methylene chloride. The combined filtrate and washings were concentrated under reduced pressure below 30°C. The solid residue was dissolved in acetone (105 ml), the solution was cooled to 0°C and concentrated ammonium hydroxide (50 ml) was added over 10 minutes. After stirring at ambient temperature for 30 minutes, the solvent was evaporated under reduced pressure. The residue was dissolved in 0.5 M potassium hydroxide solution (370 ml), filtered and the filtrate was acidified with 6N HCl. The product was collected and dried at 70°C under vacuum to yield 12.11 g (86%) of material melting at 125—127°C. An analytical sample melted at 125—126°C after recrystallization from 1,2-dichloroethane and treatment with decolorizing carbon.

| | | | |
|---|---|---|---|
| Analysis calculated for $C_9H_9NO_3S_2$: | C, 44.43; | H, 3.73; | N, 5.76. |
| Found: | C, 44.67; | H, 3.77; | N, 5.97. |

*Step B:* Preparation of 5-Hydroxy-2-sulfamoylbenzo[b]thiophene

Pyridine hydrochloride (50 g) was heated to 190°C and to the melt was added 5-methoxy-2-sulfamoylbenzo[b]thiophene (12.78 g, 0.053 m). The mixture was stirred at 190°C for 2 hours and then poured into ice and extracted with ethyl acetate (3 × 200 ml). The combined extract was washed with 3N HCl, dried over $Na_2SO_4$ and filtered through a pad of filter aid and charcoal. The filtrate was concentrated under reduced pressure and the residue was crystallized from nitromethane to yield 7.83 g (64%) of product melting at 197—198.5°C. An analytical sample melted at 192.5—193.5°C after recrystallization from nitromethane and treatment with decolorizing carbon.

| | | | |
|---|---|---|---|
| Analysis calculated for $C_8H_7NO_3S_2$: | C, 41.91; | H, 3.08; | N, 6.11. |
| Found: | C, 42.08 and 42.11; | H, 3.11 and 3.10; | N, 6.38 and 6.12. |

### Example 6
### 5-Acetoxy-2-sulfamoylbenzo[b]thiophene

A solution of 5-hydroxy-2-sulfamoylbenzo[b]thiophene (4.00 g, 0.017 m) in acetone (70 ml) was cooled to −5°C, triethylamine (1.92 g, 0.019 m) was added, followed by the dropwise addition of a solution of acetyl chloride (1.49 g, 0.019 m) in acetone (10 ml) over 15 minutes, maintaining the temperature at −5 to −3°C. After stirring at −5°C for 2-1/4 hours, the mixture was filtered and the solid was washed with acetone. The combined filtrate and washings were concentrated under reduced pressure, the residue was dissolved in ethyl acetate (7.5 ml) washed with water (2 × 20 ml), dried and evaporated under reduced pressure. The residue was crystallized from 1,2-dichloroethane to yield 4.01 g (87%) of product melting at 129—132°C. An analytical sample melted at 131.5—133°C after recrystallization from 1,2-dichloroethane.

| | | | |
|---|---|---|---|
| Analysis calculated for $C_{10}H_9NO_4S_2$: | C, 44.27; | H, 3.34; | N, 5.16. |
| Found: | C, 44.53; | H, 3.28; | N, 4.99. |

Employing the procedure substantially as described in Example 6, but substituting for the acetyl chloride used therein an approximately equimolar amount of the acid chlorides or acid anhydrides described in Table II there are produced the acyloxy-2-sulfamoylbenzo[b]thiophenes also described in Table II in accordance with the following reaction scheme.

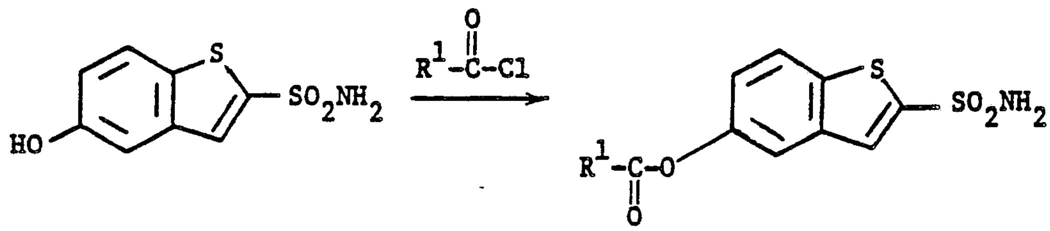

TABLE II

| 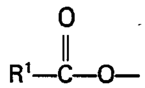 |
|---|
| 2,2-dimethylpropionate (product m.p. 144—146°C) |
| 3-methoxycarbonylpropionate (product m.p. 128—131°C) |
| 3-ethoxycarbonylpropionate |
| propionate |
| cyclohexane carboxylate |
| cyclopentane carboxylate |
| octanoate |
| 4,4-dimethylcyclohexane carboxylate |
| 3-chloro-2,2-dimethyl propionate |
| benzoate |
| nicotinate |
| 4-methyl benzoate |
| 4-chlorobenzoate |
| 4-methoxybenzoate |
| 3-phenylpropionate |
| 4-chlorophenyl acetate |
| 3-(4-ethylphenyl)propionate |
| 3-hydroxy-2,2-dimethylpropionate |
| 3-dimethylamino-2,2-dimethylpropionate |
| acrylate |
| crotonate |
| propiolate |
| cinnamate |
| N-acetylpiperidine-4-carboxylate |
| methoxyacetate |
| succinate[(1)] |
| 2-methoxy-2-methylpropionate[(2)] |
| 2-thienylcarboxylate |

[(1)] acylating reagent is succinic anhydride
[(2)] acylating reagent is 2-methoxy-2-methylpropionic N,N-diphenyl carbamic anhydride

## Example 7
## 5-Bromo-2-sulfamoylbenzo[b]thiophene

*Step A:* Preparation of 4-Bromophenyl-2,2-diethoxyethylsulfide

To a solution of p-bromothiophenol (117.2 g, 0.62 mol), $K_2CO_3$ (171.4 g, 1.24 mol) in acetone (500 ml) was added dropwise under $N_2$, bromoacetaldehyde diethylacetal (90 ml, d = 1.31, 0.6 mol). After stirring overnight at room temperature, the reaction mixture was filtered and the filtrate reconcentrated to dryness to provide a quantitative yield of product. The material was used directly in the next step without further purification.

*Step B:* Preparation of 5-Bromobenzo[b]thiophene

To a mixture of polyphosphoric acid (300 g) and chlorobenzene (300 ml) heated at 135°C under $N_2$ was added the acetal from Step A (61 g, 0.2 mol) over 10 minutes under $N_2$. After 0.5 h, the hot mixture was poured onto ice —$H_2O$ and the resulting solution extracted 4× with $CHCl_3$. The organic extracts were washed with $H_2O$, saturated $Na_2CO_3$ solution, dried, filtered and concentrated to dryness. The residue was distilled to yield 25.5 g (60%) of product; b.p. 106°C.

*Step C:* Preparation 5-Bromo-2-sulfamoylbenzo[b]thiophene

Employing the procedure substantially as described in Example 1, Step C but substituting for the 6-methoxybenzo[b]thiophene used therein an equimolar amount of 5-bromobenzo[b]thiophene, there was produced the subject compound in 17% yield, m.p. 211—213°C, after recrystallization from acetonitrile.

### Example 8

### 7-Methoxy-2-sulfamoylbenzo[b]thiophene

Employing the procedure substantially as described in Example 7, Steps A, B and C but substituting for the 4-bromothiophenol used in Step A thereof an equimolar amount of 2-methoxythiophenol there are produced in sequence:

7-methoxyphenyl-2,2-diethoxyethylsulfide in quantitative yield (not characterized);
7-methoxybenzo[b]thiophene in 42% yield, b.p. 0.5 mm 100—110°C; and
7-methoxy-2-sulfamoylbenzo[b]thiophene in 55% yield, m.p. 195—197°C.

### Example 9

### 7-Hydroxy-2-sulfamoylbenzo[b]thiophene

Employing the procedure substantially as described in Example 1, Step D but substituting for the 6-methoxy-2-sulfamoylbenzo[b]thiophene used therein, an equimolecular amount of 7-methoxy-2-sulfamoylbenzo[b]thiophene, there was produced 7-hydroxy-2-sulfamoylbenzo[b]thiophene (55% yield) m.p. 196—198°C.

Employing the procedure substantially as described in Example 6, but using as the acylating agent acetyl chloride as used therein or an approximately equimolar amount of the acid chlorides described in Table III and substituting for the 5-hydroxy-2-sulfamoylbenzo[b]thiophene used therein an equimolar amount of 7-hydroxy-2-sulfamoylbenzo[b]thiophene or 4-hydroxy-2-sulfamoylbenzo[b]thiophene there are produced the acyloxy-2-sulfamoylbenzo[b]thiophenes also described in Table III in accordance with the following reaction scheme.

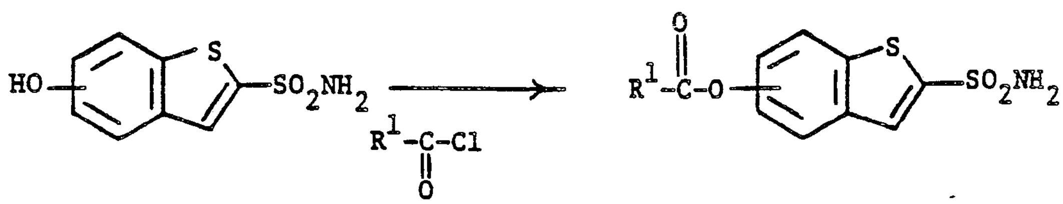

TABLE III

| Position | $R^1-\overset{O}{\overset{\|}{C}}-O-$ |
|---|---|
| 7 | 2,2-dimethylpropionate |
| 7 | 3-methoxycarbonylpropionate |
| 7 | acetate |
| 4 | 2,2-dimethylpropinoate |
| 4 | 3-methoxycarbonylpropionate |
| 4 | acetate |
| 7 | propionate |
| 4 | cyclohexane carboxylate |
| 7 | cyclopentane carboxylate |
| 4 | octanoate |
| 7 | 4,4-dimethylcyclohexane carboxylate |
| 4 | 3-chloro-2,2-dimethyl propionate |
| 7 | benzoate |
| 4 | nicotinate |
| 7 | 4-methyl benzoate |

TABLE III (cont'd.)

| Position | $R^1-\overset{\overset{O}{\|}}{C}-O-$ |
|---|---|
| 4 | 4-chlorobenzoate |
| 7 | 4-methoxybenzoate |
| 4 | 3-phenylpropionate |
| 7 | 4-chlorophenylacetate |
| 4 | 3-(4-ethylphenyl)propionate |
| 7 | 3-hydroxy-2,2-dimethylpropionate |
| 4 | 3-dimethylamino-2,2-dimethylpropionate |
| 7 | acrylate |
| 4 | crotonate |
| 7 | propiolate |
| 4 | cinnamate |
| 4 | N-acetylpiperidine-4-carboxylate |
| 7 | methoxyacetate |
| 4 | succinate(1) |
| 7 | 2-methoxy-2-methylpropionate(2) |
| 4 | 2-thienylcarboxylate |

(1) acylating reagent is succinic anhydride
(2) acylating reagent is 2-methoxy-2-methylpropionic N,N-diphenyl carbamic anhydride

Example 10

6-(2-Sulfamoylbenzo[b]thienyl 2-Methylpropyl Carbonate

A solution of 6-hydroxy-2-sulfamoylbenzo[b]thiophene (2.5 g; 0.01 mole) in acetone (45 ml) at −5° was treated with triethylamine (1.21 g; 0.01 mole). Then a solution of isobutyl chloroformate (1.64 g; 0.012 mole) in acetone (5 ml) was added, dropwise, during 15 min at −5°.

After 15 minutes, the reaction mixture was poured into water (300 ml). The resulting semi-solid was extracted into ethyl acetate, washed with saturated sodium chloride solution and dried over sodium sulfate. Removal of solvent *in vacuo* gave a solid. The yield was 3.59 g, m.p. 109—123°C. Recrystallization from butyl chloride gave the pure title compound m.p. 129—130°.

Analysis calculated for $C_{13}H_{15}NO_5S_2$: C, 47.40; H, 4.59; N, 4.25.
Found: C, 47.65; H, 4.76; N, 4.11.

Employing the procedure substantially as described in Example 10 but substituting for the 6-hydroxy-2-sulfamoylbenzo[b]thiophene used therein equimolecular amounts of the 4,5 or 7-hydroxy-2-sulfamoylbenzo[b]thiophenes and using isobutyl chloroformate as in Example 10 or substituting therefore the chloroformates described in Table IV, there are produced the 4,5,6 or 7 (2-sulfamoylbenzo[b]thienyl) carbonates also described in Table IV in accordance with the following reaction scheme:

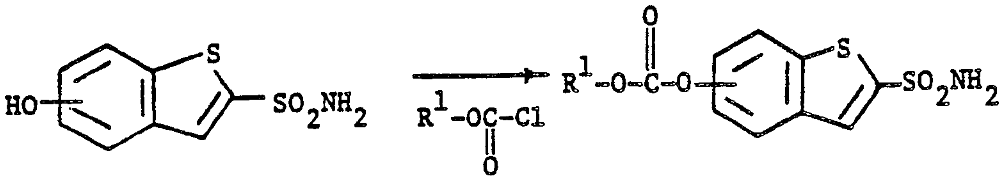

TABLE IV

| Position of Substitution | $R^1$ |
|---|---|
| 5 | 2-methylpropyl (product m.p. 100—102°C) |
| 5 | 2-methylpropyl |
| 7 | 2-methylpropyl |
| 6 | phenyl |
| 6 | ethyl |
| 6 | propyl |
| 6 | 1,1-dimethylethyl |
| 5 | n-heptyl |
| 5 | undecanyl |
| 4 | 4,4-dimethylcyclohexyl |
| 7 | 2-chloro-1,1-dimethylethyl |
| 6 | 4-methylphenyl |
| 5 | 4-chlorophenyl |
| 4 | 4-methoxyphenyl |
| 7 | 4-chlorobenzyl |

Example 11

(*S*)-6-[3-(1,1-Dimethylethyl)oxazolidin-2-on-5-yl]-methoxy-2-sulfamoylbenzo[b]thiophene

*Step A:* Preparation of N,N-Dimethyl-N'-(6-hydroxy-2-sulfamoylbenzo[b]thiophene)formamidine

To a partial suspension of 6-hydroxy-2-sulfamoylbenzo[b]thiophene (2.29 g, 10 mmol) in acetonitrile (10 ml) under a nitrogen atmosphere was added dropwise a solution of N,N-dimethylformamide dimethyl acetal (1.6 ml, 12 mmol) in acetonitrile (10 ml). After ½ hour, water was added and the product was extracted into ethyl acetate, dried ($Na_2SO_4$) and evaporated to dryness. The residue was crystallized from hot 1,2-dichloroethane, after treatment with charcoal, to give 2.1 g of product, m.p. 164—166°C.

*Step B:* Preparation of (*S*)-N,N-Dimethyl-N'-[[3-(1,1-dimethylethyl)oxazolidin-2-on-5-yl]methoxy-2-sulfamoylbenzo[b]thiophene]formamidine

A solution of (*S*)-3-(1,1-dimethylethyl)-5-(hydroxymethyl)oxazolidinone mesylate (4.26 g, 17 mmol) in DMSO (15 ml) was added dropwise to a solution of N,N-dimethyl-N'-(6-hydroxy-2-sulfamoylbenzo[b]thiophene)formamidine (4.26 g, 15 mmol) in DMSO (15 ml) containing potassium carbonate (2.8 g, 20 mmol) warmed at 70°C under a nitrogen atmosphere and stirring was continued at 70°C for 20—24 hours. The reaction mixture was poured into ice water and extracted with ethyl acetate/acetonitrile, dried ($Na_2SO_4$) and concentrated to a small volume. The crystalline product was collected (wt.; 5.5 g) and recrystallized twice from ethyl acetate, m.p. 121—25°C.

| | | | |
|---|---|---|---|
| Analysis calculated for $C_{19}H_{25}N_3O_5S_2$: | N, 9.56; | C, 51.92; | H, 5.73. |
| Found: | N, 9.58; | C, 51.55; | H, 5.76. |

*Step C:* Preparation of (*S*)-6-[3-(1,1-Dimethylethyl)oxazolidin-2-on-5-yl]methoxy-2-sulfamoylbenzo[b]thiophene

A suspension of the product from Step B (5.4 g, 14.5 mmol) was heated at 100°C in 6N HCl (75 ml) for 3—4 hours, cooled with ice and extracted with ethyl acetate, dried ($Na_2SO_4$) and evaporated to give 5.8 g of crude product. The mixture was dissolved in hot 1,2-dichloroethane, filtered hot to remove dealkylated product (1.3 g) and evaporated. The residue (4.5 g) was chromatographed on silica gel eluting with a gradient of 1.5—6% methanol/chloroform (v/v). The purified product was recrystallized from 1,2-dichloroethane to give 3.2 g of product, m.p. 147—149°C.

Analysis calculated for $C_{16}H_{20}N_2O_5S_2$: N, 7.29; C, 49.98; H, 5.24.
Found: N, 7.36; C, 50.09; H, 5.37.

Employing the procedure of Example 11, Steps A, B and C but using 4,5- or 7-hydroxy-2-sulfamoylbenzo[b]thiophene in place of the 6-hydroxy analog, there are produced the (S)-4,5- or 7-[3-(1,1-dimethylethyl)oxazolidin-2-on-5-yl]methoxy-2-sulfamoylbenzo[b]thiophenes respectively.

Example 12

(*S*)-6-[(Oxazolidin-2-on-5-yl)methoxy]-2-sulfamoylbenzo[b]thiophene

Crude by-product (1.3 gm) isolated from Step C of Example 11 was dissolved in hot ethyl acetate/acetonitrile, treated with charcoal, and the solvents were boiled off until crystallization began. Collected 0.83 gm, m.p. 198—199°C.

Analysis calculated for $C_{12}H_{12}N_2O_5S_2$: N, 8.53; C, 43.89; H, 3.68.
Found: N, 8.58; C, 44.04; H, 3.63.

Similarly prepared are the (S)-4,5 or 7-[(oxazolidin-2-on-5-yl)methoxy]-2-sulfamoylbenzo[b]thiophene.

Example 13

(*S*)-6-[3-(1,1-Dimethylethylamino)-2-hydroxy-propoxy-2-sulfamoylbenzo[b]thiophene Hydrochloride

A suspension of (*S*)-6-[3-(1,1-dimethylethyl)oxazolidin-2-on-5-yl]methoxy-2-sulfamoylbenzo[b]thiophene (2.47 g, 6.4 mmol) in a solution of water/ethanol (2:1 (v/v), 20 ml) containing 40% NaOH (w/v) (5 ml) was heated at 100°C for 3 days. The reaction mixture was cooled, acidified with hydrochloric acid, filtered to remove insoluble material and evaporated to dryness. The residue was repeatedly extracted with hot ethanol, filtered and the ethanol extracts evaporated. The resultant residue was triturated with ethanol/diethyl ether to give a solid product (wt. 1.95 g). After several recrystallizations which involved dissolution of the solid in hot methanol, followed by addition of ethanol, and boiling off solvents until crystallization began, there was obtained 1.2 g of product, m.p. 266—268°C.

Analysis calculated for $C_{15}H_{22}N_2O_4S_2.HCl.0.4NaCl.0.4H_2O$: C, 42.37; N, 6.59; H, 5.57; Cl, 11.67.
Found: C, 42.33; N, 6.62; H, 5.57; Cl, 11.42.

Similarly prepared are the (S)-4,5 or 7-[3-(1,1-dimethylethylamino)-2-hydroxypropoxy]-2-sulfamoylbenzo[b]thiophene.

Example 14

6-(2-Ketopropoxy)-2-sulfamoylbenzo[b]thiophene

*Step A:* Preparation of N,N-Dimethyl-N'-[6-(2-ketopropoxy)-2-sulfamoylbenzo[b]thiophene formamidine

Chloroacetone (1.2 ml) was added dropwise to a stirred mixture of anhydrous potassium carbonate (2.0 g) and N,N-dimethyl-N'-[6-hydroxy-2-sulfamoylbenzo[b]thiophene]formamidine (2.90 g) in dimethylsulfoxide (20 ml) at 25°C. After 24 hours, the reaction mixture was diluted with water (200 ml) and the solid that formed was collected and dried, 3.02 g, m.p. 150—153°C.

*Step B:* Preparation of 6-(2-Ketopropoxy)-2-sulfamoylbenzo[b]thiophene

The product from Step A was heated to reflux with a mixture of methanol (50 ml) and 6*N* hydrochloric acid (50 ml) for 0.5 hour. The cooled solution was diluted with water (300 ml) and chilled. The precipitated solid was collected, washed with water and dried, to give 2.25 g, m.p. 181—185°C. Recrystallization from acetonitrile gave a sample with m.p. 184—187°.

Analysis calculated for $C_{11}H_{11}NO_4S_2$: C, 46.30; H, 3.89; N, 4.91.
Found: C, 46.48; H, 3.87; N, 5.02.

Similarly prepared are the 4,5 or 7-(2-ketopropoxy)-2-sulfamoylbenzo[b]thiophenes.

Example 15

6-[(Ethoxycarbonyl)methoxy]-2-sulfamoylbenzo[b]thiophene

To a solution of ethyl bromoacetate (1.2 ml, 11 mmol) and 6-hydroxy-2-sulfamoylbenzo[b]thiophene (2.29 g, 10 mmol) in DMSO (10 ml) was added dropwise a solution of potassium bicarbonate (1.0 g, 10 mmol) and potassium carbonate (0.13 g, 1 mmol) in water (7 ml). The mixture was stirred for 1—2 days, then diluted with water. The precipitated product was collected dissolved in ethyl acetate, dried ($Na_2SO_4$)

and evaporated to dryness. The residue was recrystallized from hot ethyl acetate, boiling off solvent until crystallization began, to give 1.2 g of product, m.p. 182—184°C.

Analysis calculated for $C_{12}H_{13}NO_5S_2$: C, 45.70; H, 4.15; N, 4.44.
Found: C, 46.11; H, 4.32; N, 4.39.

Following the procedure of Example 15, there are also produced 4,5 or 7-[(ethoxycarbonyl)methoxy]-2-sulfamoylbenzo[b]thiophene. The 5-isomer has m.p. 167.5—168°C.

Example 16

6-[(Carboxy)methoxy]-2-sulfamoylbenzo[b]thiophene

Solid 6-[(ethoxycarbonyl)methoxy]-2-sulfamoylbenzo[b]thiophene (1.6 g, 5 mmol) was added to 10% NaOH (40 ml) and stirred at room temperature 4—6 hours. The solution was acidified and extracted with ethyl acetate, dried ($Na_2SO_4$) and evaporated. The residue was dissolved in hot ethyl acetate, filtered, and evaporated until crystallization began to give 0.94 g of product, m.p. 205—208°.

Analysis calculated for $C_{10}H_9NO_5S_2$: N, 4.88; C, 41.80; H, 3.16.
Found: N, 4.92; C, 42.07; H, 3.24.

By similar procedures there are prepared the 4,5 or 7-[(carboxy)methoxy]-2-sulfamoylbenzo[b]thiophene.

Example 17

6-[3-Carboxypropoxy]-2-sulfamoylbenzo[b]thiophene

*Step A:* Preparation of N,N-Dimethyl-N′-[6-(3-(ethoxycarbonyl)propoxy)-2-sulfamoylbenzo[b]thiophene formamidine

Ethyl-4-bromobutyrate (2.15 g, 11.0 mmol) was added dropwise to a stirred solution of N,N-dimethyl-N′-[6-hydroxy-2-sulfamoylbenzo[b]thiophene formamidine (2.84 g, 10.0 mmol) and potassium carbonate (2.07 g, 15.0 mmol) in dimethyl sulfoxide (20.0 ml). The reaction temperature was kept at 70°C for 18 hours. The reaction mixture was cooled, poured into water (100 ml) and extracted with ethyl acetate (3 × 150 ml). The combined ethyl acetate extracts were washed with water (3 × 15 ml), brine (2 × 25 ml) and dried ($Na_2SO_4$). The ethyl acetate was removed under vacuum to yield an oil (2.85 gm).

*Step B:* Preparation of 6-(3-carboxypropoxy)-2-sulfamoylbenzo[b]thiophene

N,N-Dimethyl-N′-[6-(3-(ethoxycarbonyl)propoxy)-2-sulfamoylbenzo[b]thiophene]formamidine (2.85 g, 7.2 mmol) was suspended in hydrochloric acid (25.0 ml, 6.0N) and warmed to 70°C for 6 hours. The reaction was cooled and the solid which formed collected via vacuum filtration. The solid was dissolved in ethyl acetate-methanol (160 ml, 3 to 1 (v/v)) and filtered through charcoal. The solvent was removed under vacuum and the remaining solid dried under vacuum to yield a white solid (2.0 g, 6.3 mmol), m.p. 186—187°C.

Analysis calculated: C, 45.70; H, 4.15; N, 4.44.
Found: C, 45.78; C, 4.14; N, 4.31.

Similarly prepared are the 4,5 or 7-(3-carboxypropoxy)-2-sulfamoylbenzo[b]thiophenes.

Example 18

6-[2,3-Epoxypropoxy]-2-sulfamoylbenzo[b]thiophene

*Step A:* Preparation of 6-allyloxy-2-sulfamoylbenzo[b]thiophene

Allyl bromide (2.07 ml, 24.0 mmol) was added dropwise to a stirred solution of 6-hydroxy-2-sulfamoylbenzo[b]thiophene (5.0 g, 22.0 mmol) in dimethylsulfoxide (15.0 ml). A solution of $K_2CO_3$ (3.34 g, 24.0 mmol) in water (7.5 ml) was added dropwise to the reaction solution at 25°C. After 24 hours, the reaction mixture was diluted with water (50 ml). The solid that formed was collected and dried, 3.75 gm (13.9 mmol), m.p. 101—102°C.

*Step B:* Preparation of 6-[3(2)-hydroxy-2(3)-bromopropoxy]-2-sulfamoylbenzo[b]thiophene

6-[Allyloxy]-2-sulfamoylbenzo[b]thiophene (5.0 gm, 18.6 mmol) was added to a solution of water (5.0 ml) in DMSO (30 ml) with stirring. The solution was cooled to 0°C and N-bromosuccinimide (3.5 g, 19.5 mmol) was added. The reaction mixture was allowed to warm to room temperature and after 2.0 hours was poured into water (150 g). The aqueous mixture was extracted with ethyl acetate (3 × 100 ml) and the combined extracts washed with water (2 × 25 ml), brine (2 × 25 ml) and dried ($Na_2SO_4$). The ethyl acetate was removed under vacuum to give a red oil (5.5 g, 15.0 mmol).

*Step C:* Preparation of 6-[2,3-epoxypropoxy]-2-sulfamoylbenzo[b]thiophene

6-[3(2)-Hydroxy-2(3)-bromopropoxy]-2-sulfamoylbenzo[b]thiophene (11.1 g, 30.2 mmol) was dissolved in methanol (25 ml). A solution of potassium hydroxide (3.9 g), 60.4 mm) in methanol (20 ml) was added dropwise. After 2.5 hours, the reaction mixture was poured into water (100 ml). The mixture was acidified with concentrated hydrochloric acid. The aqueous mixture was extracted with ethyl acetate (3 × 100 ml)

and the combined ethyl acetate extracts washed with water (2 × 100 ml), brine (2 × 100 ml) and dried ($Na_2SO_4$). The ether was removed under vacuum. The solid which remained was purified using medium pressure chromatography (55% hexane/45% ethyl acetate, v/v), m.p. 148—149°C.

Analysis calculated for $C_{11}H_{11}NO_4S_2$: C, 46.30; H, 3.89; N, 4.91.
Found: C, 46.43; C, 3.90; N, 4.93.

Using the procedures of Example 18, there are also prepared the 4,5 or 7-(2,3-epoxypropoxy)-2-sulfamoylbenzo[b]thiophenes.

Example 19

6-[3-Methoxy-2-hydroxypropoxy]-2-sulfamoylbenzo[b]thiophene

One drop of concentrated sulfuric acid was added to a solution of 6-[2,3-epoxypropoxy]-2-sulfamoylbenzo[b]thiophene (2.0 g, 7.0 mmol) in methanol (25 ml). After 18 hours, the methanol was removed under vacuum. The residue was dissolved in ethyl acetate (200 ml), washed with water (2 × 25 ml), brine (2 × 25 ml) and dried ($Na_2SO_4$). The ethyl acetate was removed under vacuum. The white solid remaining was recrystallized from boiling ethyl acetate, 1.5 g, m.p. 128—129°C.

Analysis calculated for $C_{12}H_{15}NO_5S_2$: C, 45.41; H, 4.76; N, 4.41.
Found: C, 45.38; C, 4.76; N, 4.60.

Similarly prepared are the 4,5 or 7-(3-methoxy-2-hydroxypropoxy)-2-sulfamoylbenzo[b]thiophenes.

Example 20

6-(2,3-Dihydroxypropoxy)-2-sulfamoylbenzo[b]thiophene

*Step A:* Preparation of N,N-Dimethyl-N'-[[6-(2,2-dimethyl-1,3-dioxolan-3-yl)methoxy]-2-sulfamoylbenzo[b]-thiophene]formamidine

N,N-dimethyl-N'-[6-hydroxy-2-sulfamoylbenzo[b]thiophene]formamidine (5.0 g, 17.6 mmol) was added to a mixture of $K_2CO_3$ (3.64 g, 26.4 mmol) in DMSO (25 ml). 2,2-Dimethyl-1,3-dioxolane-4-methanol (4.07 g, 19.3 mmol) was added to the reaction dropwise. The reaction temperature was kept at 70°C for 18 hours. The cooled reaction was poured into water (100 ml) and extracted with ethyl acetate containing a small amount of methanol (4 × 25 ml). The combined ethyl acetate extracts were washed with water (4 × 25 ml), brine (2 × 25 ml) and dried ($MgSO_4$). The ethyl acetate was removed under vacuum to yield a tan solid (6.0 g).

*Step B:* 6-(2,3-Dihydroxypropoxy)-2-sulfamoylbenzo[b]thiophene

N,N - dimethyl - N' - [[6 - (2,2 - dimethyl - 1,3 - dioxolan - 3 - yl) - methoxy] - 2 - sulfamoylbenzo[b]thiophene]formamidine (6.0 g, 15.0 mmol) was added to 6.0N HCl (25 ml) and the mixture warmed to 70°C for 18 hours. The cooled reaction was extracted with ethyl acetate (4 × 150 ml) and the combined extracts washed with water (1 × 50 ml), brine (2 × 50 ml) and dried ($MgSO_4$). The ethyl acetate was removed under vacuum to yield a white solid. Recrystallization from nitromethane gave 4.0 g, m.p. 134—135°C.

Analysis calculated for $C_{11}H_{13}NO_5S_2$: C, 43.56; H, 4.32; N, 4.62.
Found: C, 43.98; C, 4.31; N, 4.31.

Similarly prepared is the 4,5 or 7-(2,3-dihydroxypropoxy)-2-sulfamoylbenzo[b]thiophene.

Example 21

5-(2,3-Dihydroxypropoxy)-2-sulfamoylbenzo[b]thiophene

*Step A:* N,N-Dimethyl-N'-(5-hydroxy-2-sulfamoylbenzo[b]thiophene

A suspension of 5-hydroxy-2-sulfamoylbenzo[b]thiophene (10.65 g, 0.046 m) in acetonitrile (100 ml) was stirred while dimethylformamide dimethyl acetal (5.95 g, 0.050 m) was added quickly. After stirring at ambient temperature for 30 minutes, the mixture was added to water (200 ml) and the solid was collected and dried at 80°C under vacuum to yield 11.39 g (87%) of product melting at 208—209°C. An analytical sample melts at 208.5—210°C after recrystallization from methanol.

Analysis calculated for $C_{11}H_{12}N_2O_3S_2$: C, 46.46; H, 4.26; N, 9.85.
Found: C, 46.67; C, 4.37; N, 10.12.

*Step B:* Preparation of 5-(2,3-Dihydroxypropoxy)-2-sulfamoylbenzo[b]thiophene

A suspension of sodium hydride (0.21 g, 0.0044 mol, 50% dispersion in mineral oil) in dry dimethylformamide (10 ml) was stirred under nitrogen at 70°C while N,N-dimethyl-N'-(5-hydroxy-2-sulfamoylbenzo-[b]thiophene)formamidine (1.00 g, 0.0035 mol) was added portionwise over 15 minutes. After stirring at 70°C for 30 minutes, 2,2-dimethyl-4-hydroxymethyl-1,3-dioxolane methanesulfonate (0.74 g, 0.0035 mol) was added rapidly and the mixture was heated at 70°C for 19.5 hours. An additional 0.01 g of 50% sodium hydride and 0.19 g of 2,2-dimethyl-4-hydroxymethyl-1,3-dioxolane methanesulfonate was added and heating at 70°C was continued for 3 hours more. The reaction mixture was poured into water (100 ml) and extracted with ethyl acetate (3 × 100 ml), the combined extracts were washed with water, dried over $Na_2SO_4$ and evaporated under reduced pressure. The residue was heated and stirred in 6N HCl (20 ml) for

17.5 hours. The reaction mixture was diluted with water (75 ml) and extracted with ethyl acetate (3 × 100 ml). After washing with water and drying over $Na_2SO_4$, the solvent was evaporated under reduced pressure to yield 0.60 g (57%) of product which was crystallized from methanol-chloroform. An analytical sample melted at 162.5—163.5°C after recrystallization from methanol-chloroform and treatment with decolorizing carbon.

Analysis calculated for $C_{11}H_{13}NO_5S_2$: C, 43.55; H, 4.32; N, 4.62.
Found: C, 43.22; H, 4.29; N, 4.69.

Employing the procedures substantially as described in Examples 11, 14, 15, 17, 18 or 21, there are produced the ethers of 4,5,6 or 7-hydroxy-2-sulfamoylbenzo[b]thiophene described in Table V in accordance with the following reaction scheme:

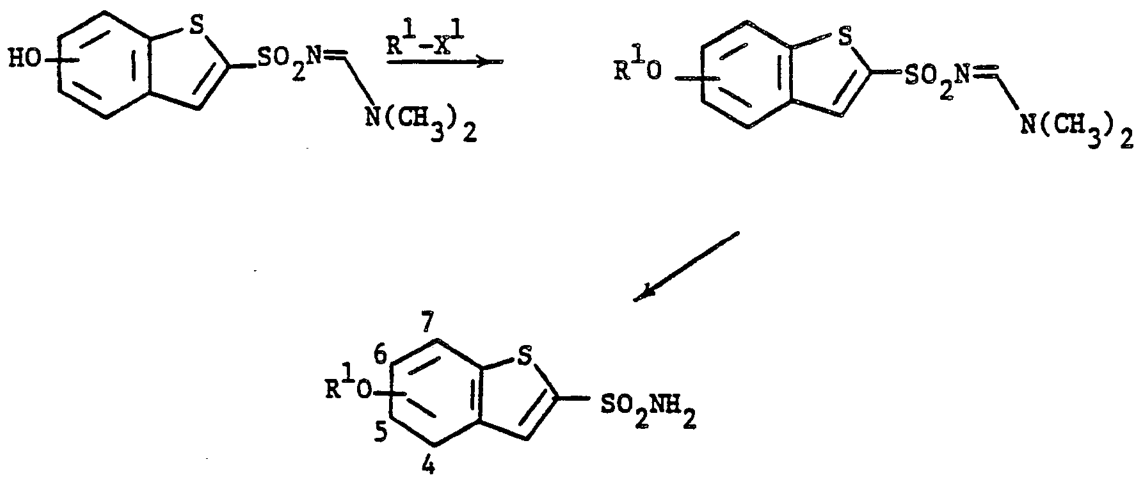


TABLE V

| Position of Substitution | $R^1$ | X' |
|---|---|---|
| 4 | $C_2H_5$— | Br |
| 5 | n-$C_3H_7$— | Br |

Example 22

5-(Dibenzyl)amino-2-sulfamoylbenzo[b]thiophene

*Step A:* Preparation of 5-Dibenzylaminobenzo[b]thiophene

A stirred solution of 5-aminobenzo[b]thiophene hydrochloride (4.65 g, 0.025 mole) in dry DMSO (40 ml) was treated with benzyl bromide (6 ml, 0.05 mole) followed by solid $NaHCO_3$ (6.3 g, 0.075 mole). After the immediate, vigorous evolution of $CO_2$, the mixture set to a solid mass. This was held at ambient temperature overnight and the solid then was collected and recrystallized from $CH_3CN$ to obtain 5.0 g (61%) of 5-(dibenzyl)aminobenzo[b]thiophene, m.p. 114—117°C. A sample recrystallized from ethyl acetate-hexane melted at 115—117°C.

Analysis calculated for $C_{22}H_{19}NS$: C, 80.20; H, 5.81; N, 4.25.
Found: C, 80.49; H, 5.86; N, 3.90.

*Step B:* Preparation of 5-(Dibenzyl)amino-2-sulfamoylbenzo[b]thiophene

The subject compound was prepared by the procedure described in Example 1, Step C, using 5-dibenzylaminobenzo[b]thiophene in place of 6-methoxybenzo[b]thiophene. The crude product was recrystallized from 1,2-dichloroethane-hexane and then from 70% ethanol to obtain 52% of 5-(dibenzyl)-amino-2-sulfamoylbenzo[b]thiophene as a light tan solid, m.p. 122—124°C.

Analysis calculated for $C_{22}H_{20}N_2O_2S_2$: C, 64.68; H, 4.93; N, 6.86.
Found: C, 64.82; H, 4.95; N, 7.11.

Example 23

5-(Dimethyl)amino-2-sulfamoylbenzo[b]thiophene

*Step A:* Preparation of 5-(Dimethyl)aminobenzo[b]thiophene

To a stirred solution of 5-aminobenzo[b]thiophene (4.55 g, 0.03 mole) in $CH_3CN$ (175 ml) was added 37% aqueous formaldehyde (28 ml, 0.035 mole) followed by sodium cyanoborohydride (6.65 g, 0.105 mole). Glacial acetic acid (3.5 ml) was added in small increments over 15 minutes. After 1 hour, another 3.5 ml of acetic acid was added and after another 1 hour, the mixture was poured into ether (700 ml). The ethereal layer was separated, washed with 1M KOH (3×) then with saturated NaCl solution, dried over anhydrous $Na_2SO_4$, and evaporated to dryness *in vacuo*. The residual dark oily solid was purified by chromatography on 800 ml of silica gel. Elution with 97 hexane:3 ethyl acetate afforded 4.6 g (88%) of 5-(dimethyl)aminobenzo[b]thiophene as a light yellow solid, m.p. 52—54°C.

| | | | |
|---|---|---|---|
| Analysis calculated for $C_{10}H_{11}NS$: | C, 67.75; | H, 6.26; | N, 7.90. |
| Found: | C, 67.12; | H, 6.24; | N, 7.85. |

*Step B:* Preparation of 5-(Dimethyl)amino-2-sulfamoylbenzo[b]thiophene

The subject compound was prepared by the procedure described in Example 1, Step C, using 5-(dimethyl)aminobenzo[b]thiophene in place of 6-methoxybenzo[b]thiophene. The crude product was obtained in 60% yield as a dark yellow solid. Two recrystallizations from 1,2-dichloroethane, decolorizing with charcoal afforded 5-(dimethyl)-amino-2-sulfamoylbenzo[b]thiophene as light yellow needles, m.p. 197—199°C.

| | | | |
|---|---|---|---|
| Analysis calculated for $C_{10}H_{12}N_2O_2S_2$: | C, 46.85; | H, 4.72; | N, 10.93. |
| Found: | C, 47.17; | H, 4.52; | N, 10.56. |

Example 24

4-Chloro-5-morpholino-2-sulfamoylbenzo[b]thiophene and
5-Morpholino-2-sulfamoylbenzo[b]thiophene

*Step A:* Preparation of 5-Morpholinobenzo[b]thiophene

5-Aminobenzo[b]thiophene (5.4 g, 0.0362 mole), bis-(2-chloroethyl)ether (5.2 g, 0.0364 mole) and 40% aqueous NaOH (0.073 mole) were stirred vigorously and heated to refluxing for 22 hours. The cooled mixture was extracted with $CHCl_3$. Evaporation of the washed and dried $CHCl_3$ extract under reduced pressure left 7.0 g (88%) of the crude product as a slightly oily, dark yellow solid. This material was combined with 1.75 g of comparable crude product from a previous run. Recrystallization from $CH_3CN$ afforded 5.50 g of 5-morpholinobenzo[b]thiophene, m.p. 127—132°C, that was characterized by nmr.

*Step B:* Preparation of 4-chloro-5-morpholinobenzo[b]thiophene-2-sulfonamide and 5-Morpholinobenzo-[b]thiophene-2-sulfonamide

Under $N_2$, a solution of 5-morpholinobenzo[b]thiophene (4.0 g, 0.0182 mole) in dry, peroxide-free THF (35 ml) was stirred and cooled to −20°C. n-Butyllithium (12.5 ml of a 1.6M solution in hexane) was added dropwise over 10—15 min. The resulting red solution was stirred at −20°C for 30 minutes and then $SO_2$ was passed over the surface for 30 minutes. After the thick suspension was stirred at ambient temperature for 1.25 hours, it was poured into ether and the solid lithium sulfinate derivative was collected and washed with ether. This solid was suspended in dry $CH_2Cl_2$ (60 ml) and cooled to 5—10°C. N-chlorosuccinimide (3.15 g, 0.0236 mole) was added in portions over 10 min. After another 30 minutes of stirring in the cold, the mixture was filtered and the solvent was stripped from the filtrate *in vacuo*. The residual dark red, oily sulfonyl chloride (6.55 g) was dissolved in acetone (50 ml) and added to a stirred mixture of conc. $NH_4OH$ (35 ml) and acetone (35 ml). After 45 minutes, the mixture was concentrated *in vacuo* and the oily product was extracted into ethylacetate. Evaporation of the washed and dried ethyl acetate extract under reduced pressure left 5.4 g of the crude mixed sulfonamides as a brown foam. This material was combined with 1.1 g of comparable crude product from a previous run and chromatographed on 500 g of silica gel, eluting with ethyl acetate-hexane (1:1).

Chromatographic fractions containing the less polar component were pooled and concentrated to yield 1.15 g (19%) of 4-chloro-5-morpholino-2-sulfamoylbenzo[b]thiophene, m.p. 195—199°C. Recrystallization from $CH_3CN$ gave 600 mg, m.p. 201—203°C.

| | | | |
|---|---|---|---|
| Analysis calculated for $C_{12}H_{13}ClN_2O_3S_2$: | C, 43.30; | H, 3.94; | N, 8.42. |
| Found: | C, 43.57; | H, 3.87; | N, 8.06. |

Chromatographic fractions containing the more polar component were pooled and concentrated to yield 4.0 g (74%) of 5-morpholino-2-sulfamoylbenzo[b]thiophene. Recrystallization from ethyl acetate gave 3.4 g, m.p. 155—156°C.

| | | | |
|---|---|---|---|
| Analysis calculated for $C_{12}H_{14}N_2O_3S_2$: | C, 48.30; | H, 4.73; | N, 9.39. |
| Found: | C, 48.59; | H, 4.81; | N, 9.57. |

Example 25

5-Methyl-2-sulfamoylbenzo[b]thiophene

5-Methyl-2-sulfamoylbenzo[b]thiophene was prepared from 5-methylbenzo[b]thiophene (8.85 g, 0.06

mol) using the procedure for the preparation of Example 1, Step C. Yield of product was 9.8 g (72%), m.p. 212—213°C.

Example 26

5-Methoxymethyl-2-sulfamoylbenzo[b]thiophene

*Step A:* Preparation of 5-Methoxymethylbenzo[b]thiophene

To a stirred mixture of powdered KOH (18.9 g, 0.336 mol) in dimethylsulfoxide (100 ml) was added 5-hydroxymethylbenzo[b]thiophene (13.8 g, 0.084 mol) in 25 ml of dimethylsulfoxide. Then methyl iodide (23.9 g, 0.168 mol) was added dropwise at ambient temperature over several minutes. Stirring was continued for 1½ hours. The mixture was filtered, diluted with water (150 ml) and extracted with methylene chloride (200 ml) in three portions. The combined extracts were washed with water, dried over anhydrous $Na_2SO_4$, filtered and concentrated *in vacuo*. This gave 14.1 g of amber liquid. Distillation gave 10.47 g of colorless liquid, b.p. 2.2 mm Hg 110—111°C. (Yield 70%).

*Step B:* Preparation of 5-Methoxymethyl-2-sulfamoylbenzo[b]thiophene

5-Methoxymethyl-2-sulfamoylbenzo[b]thiophene was prepared from 5-methoxymethylbenzo[b]thiophene (10.47 g, 0.059 mol) following the procedure for the preparation described in Example 1, Step C. Yield of product was 13.9 g (90%). M.p. (recrystallized from 1,2-dichloroethane), 124.5—125.5°C.

Example 27

5-Bromomethyl-2-sulfamoylbenzo[b]thiophene

To a suspension of 5-methoxymethyl-2-sulfamoylbenzo[b]thiophene (7.4 g, 0.029 mol) in 300 ml of dry methylene chloride cooled to −30°C was added boron tribromide (30 ml of a 1M solution in methylene chloride, 0.03 mol) over a 20 minute period. The resulting solution was stirred for 1½ hours as the temperature rose to ambient. The solution was cooled to 0°C and there was added dropwise 150 ml of water keeping the temperature below 20°C. The methylene chloride layer was separated and the aqueous suspension was extracted with three portions (200 ml) of methanol-chloroform (50/50). The combined extracts were washed with ice water, dried over anhydrous $Na_2SO_4$, filtered and concentrated *in vacuo* to give 8.8 g of tan solid (quantitative yield of crude product) which after recrystallization from 1,2-dichloroethane had m.p. 171—173°C.

Example 28

5-Dimethylaminomethyl-2-sulfamoylbenzo[b]thiophene

To an ice cold, stirred solution of 5-bromomethyl-2-sulfamoylbenzo[b]thiophene (2.0 g, 6.5 mmol) in 25 ml of methanol was bubbled in an excess of anhydrous dimethylamine. The flask was sealed and the mixture was stirred at room temperature for 1 hour. The methanol was removed *in vacuo*. The solid residue was taken up in chloroform (100 ml) and saturated $NaHCO_3$ solution (30 ml). The chloroform layer was separated and the aqueous mixture was extracted with chloroform-methanol (1/1, V/V) (50 ml). This extract was combined with the chloroform, dried over anhydrous $Na_2SO_4$, filtered and concentrated *in vacuo*. A solid (1.57 g) was obtained (yield 90%) which after recrystallization from nitromethane had m.p. 172—174.5°C.

Example 29

5-(4-Morpholinylmethyl)-2-sulfamoylbenzo[b]thiophene

To a stirred solution of morpholine (0.91 g, 10.5 mmol) and triethylamine (1.01 g, 10 mmol) in 20 ml of methanol was added 5-bromomethyl-2-sulfamoylbenzo[b]thiophene (3.06 g, 10 mmol) portionwise over 5 minutes at ambient temperature. Stirring was continued for 1¼ hours. The resulting suspension was cooled and filtered after an additional 1 hour to give 2.76 g of yellow solid. Concentration of the filtrate *in vacuo* followed by trituration of the residue with water and filtering gave an additional 0.3 g of yellow solid. (Total crude yield was 98%). Recrystallization from nitromethane gave material with m.p. 221.5—224°C.

The hydrochloride salt was also prepared using ethanolic-HCl, m.p. 244—245°C, dec.

Example 30

5-Acetoxymethyl-2-sulfamoylbenzo[b]thiophene

To a mixture of 5-bromomethyl-2-sulfamoylbenzo[b]thiophene (3.06, 0.01 mol), anhydrous sodium acetate (0.98 g, 0.01 mol) and glacial acetic acid (15 ml) was added three drops of triethylamine. The mixture was heated and the resulting solution was stirred at reflux for 6 hours and was left at room temperature overnight. The acetic acid was removed *in vacuo* and the residual gum was diluted with 25 ml of ice water. The product was extracted into 3 × 50 ml of ether. The combined extracts were washed with water, dried over anhydrous $Na_2SO_4$, filtered and concentrated *in vacuo* to give 2.5 g of yellow solid (88% yield). Recrystallization from chloroform gave material with a m.p. of 113—115°C.

Example 31

5-Hydroxymethyl-2-sulfamoylbenzo[b]thiophene

A solution of 5-acetoxymethyl-2-sulfamoylbenzo[b]thiophene (5.6 g, 19.6 mmol) in 25 ml of 1M

aqueous KOH and 25 ml of methanol was stirred at room temperature for 2 hours and at reflux for 2½ hours. The mixture was filtered and the methanol was removed *in vacuo*. The remaining aqueous suspension was acidified with excess 6N HCl and the solid was filtered, washed with ice water and dried. The crude material (3.1 g) was chromatographed on silica gel using 5% (V/V) methanol in chloroform. Product was obtained as a yellow solid (1.85 g), m.p. 174—176°C. Yield 41%.

Example 32
5-(2-methoxyethyl)-2-sulfamoylbenzo[b]thiophene

*Step A:* Preparation of 5-(2-hydroxyethyl)benzo[b]thiophene

To a flame dried flask under $N_2$ was added magnesium turnings (6 g, 0.25 mol) and THF (100 ml). The mixture was heated at reflux and a solution of methyl iodide (7.2 ml, d = 2.28, 0.116 mol), and 5-bromobenzo[b]thiophene (24.4 g, 0.114 mol) in THF (50 ml) were added dropwise. The mixture was heated at reflux for 5 hours, then cooled to 0—4°C and a solution of ethylene oxide (15 g, 0.33 mol) in THF (20 ml) was added dropwise. After the addition, the reaction mixture was allowed to stir at room temperature overnight. The suspension was then cooled and a solution of 3*N* HCl (75 ml) was added dropwise. The aqueous layer was extracted with ethyl acetate (3×) and the organic layer was backwashed with saturated $Na_2CO_3$, dried, filtered and concentrated to dryness. The residue was chromatographed on silica gel and the product was eluted with 30% ethyl acetate/hexane to yield 9.3 g (48%) of product, m.p. 56—57°C.

*Step B:* Preparation of 5-(2-methoxyethyl)benzo[b]thiophene

A suspension of KOH (6.5 g, 0.116 mol) in DMSO (55 ml) was stirred at room temperature for 5 minutes and 5-(2-hydroxyethyl)benzo[b]thiophene (5 g, 0.028 g) was added. To the above mixture, methyl iodide (3.5 ml, d = 2.28, 0.056 mol) was added. After 1 hour the mixture was poured into $H_2O$ and the aqueous layer was extracted with $CH_2Cl_2$ (3×). The organic extract was washed with $H_2O$ (3×), dried, filtered and concentrated to dryness. The residue was distilled to yield 4.7 g (87%) of product, b.p. (1.2 mm Hg) 115—120°C.

*Step C:* Preparation of 5-(2-methoxyethyl)-2-sulfamoylbenzo[b]thiophene

Employing the procedure substantially as described in Example 1, Step C but substituting for the 6-methoxybenzo[b]thiophene used therein, an equimolar amount of 5-(2-methoxyethyl)benzo[b]thiophene, there was produced the subject compound (66% yield) with m.p. 104—108°C.

Example 33
5-(2-Benzyloxyethyl)-2-sulfamoylbenzo[b]thiophene

*Step A:* Preparation of 5-(2-Benzyloxyethyl)benzo[b]thiophene

To a solution of NaH (60% oil dispersion, 2.4 g, 0.06 mol) in DMF (50 ml) heated at 60°C with stirring under $N_2$ was added dropwise a solution of 5-(2-hydroxyethyl)benzo[b]thiophene (8.9 g, 0.05 mol) in DMF (50 ml). After ½ hour, a solution of benzyl bromide (10.3 g, 7.2 ml, d = 1.438, 0.06 mol) in DMF (50 ml) was added dropwise. After 15 hours, the solution was poured into $H_2O$ and the aqueous phase extracted with ethyl acetate (3×). The organic layers were backwashed with $H_2O$, saturated NaCl, dried, filtered and concentrated to dryness. The residue was chromatographed on silica gel and the product was eluted with 10% (V/V) ethyl acetate-hexane to yield 9.7 g (72%) of product. The material was used directly in the next step without further purification.

*Step B:* Preparation of 5-(2-benzyloxyethyl)-2-sulfamoylbenzo[b]thiophene

Employing the procedure of Example 1, Step C but using the benzyl ether of Step A of this Example 33 as starting material, the product, m.p. 136—138°C was produced in 51% yield.

Example 34
5-(2-Bromoethyl)-2-sulfamoylbenzo[b]thiophene

To a solution of 5-(2-methoxyethyl)-2-sulfamoylbenzo[b]thiophene (8.3 g, 0.031 mol) in 550 ml of methylene chloride at −78°C was added dropwise under nitrogen, 100 ml of a solution of boron tribromide ($BBr_3$) (1M in hexane, 0.1 mol). The reaction was stirred at room temperature overnight (15 hours) following the addition. The mixture was cooled and diluted with 100 ml of cold water. The aqueous phase was separated and extracted 2× with methylene chloride. The original organic phase and the extracts were combined, dried, filtered and concentrated to dryness to yield 8.3 g (86%) of product with m.p. 118—120°C.

Example 35
5-(2-Acetoxyethyl)-2-sulfamoylbenzo[b]thiophene and
5-ethenyl-2-sulfamoylbenzo[b]thiophene

A solution of 4.0 g (0.012 mol) of 5-(2-bromoethyl)-2-sulfamoylbenzo[b]thiophene, 2.0 g (0.024 mol) of sodium acetate and 40 ml of DMF was heated at 100°C under nitrogen. After 15 hours the mixture was poured into water and the aqueous phase was extracted 3× with ethyl acetate. The extracts were dried, filtered and concentrated to dryness. The residue was chromatographed on silica gel by elution with 20% (V/V) ethyl acetate-hexane to yield 2.9 g (30%) of 5-ethenyl-2-sulfamoylbenzo[b]thiophene, m.p.

168—170°C (1,2-dichloroethane). Further elution with 40% (V/V) ethyl acetate-hexane yielded 3.74 g (59%) of 5-(2-acetoxyethyl)2-sulfamoylbenzo[b]thiophene, m.p. 120—122°C (1,2-dichloroethane).

Example 36

5-(2-Hydroxyethyl-2-sulfamoylbenzo[b]thiophene

A solution of 5-(2-acetoxyethyl)-2-sulfamoylbenzo[b]thiophene (1.8 g, 0.006 mol), ethanol (50 ml) and 10% NaOH (50 ml) was heated at reflux. After 4.5 h, the mixture was poured into 3*N* HCl (150 ml) and extracted with ethyl acetate (3×). The organic extracts were washed with saturated $Na_2CO_3$, dried, filtered and concentrated to dryness to yield 1.5 g (97%) of product, m.p. 162—164°C.

Example 37

6-(2-Methyldioxolan-2-yl)-2-sulfamoylbenzo[b]thiophene

*Step A:* Preparation of 6-acetylbenzo[b]thiophene

6-Acetyl-2,3-dibromobenzo[b]thiophene (6.68 g, 20 mmole) was dissolved in a mixture of ethanol and ethyl acetate (100 ml each) and MgO (1.6 g) and (20% $Pd(OH_2)/C$ (800 mg) were added under a $N_2$ atmosphere. Hydrogenolysis was carried out at 40 psi of $H_2$ at room temperature for 3 hours. The catalyst was removed by filtration and the solvent was removed *in vacuo*. The residue was partitioned between $CHCl_3$ (100 ml) and $H_2O$ (50 ml). The $CHCl_3$ layer was washed with $H_2O$ (50 ml). The aqueous layers were combined and back-extracted with $CHCl_3$ (30 ml). The $CHCl_3$ solutions were combined, dried over $Na_2SO_4$ and the solvent evaporated *in vacuo* to yield 3.8 g of product which was used in the next step without further purification.

*Step B:* Preparation of 6-(2-methyldioxolan-2-yl)-benzo[b]thiophene

A mixture of 6-acetyl benzo[b]thiophene (10.57 g, 60 mmole), p-toluenesulfonic acid (1.65 g) and ethylene glycol (33 ml) was heated to reflux in toluene (250 ml) for 2 hours with the continuous removal of $H_2O$ using a Dean-Stark trap. The reaction mixture was allowed to cool to room temperature. Then extracted with 20% saturated $NaHCO_3$ (150 ml) and $H_2O$ (2 × 150 ml). After drying over $Na_2SO_4$, the solvent was evaporated *in vacuo* and the residue was recrystallized from hexanes (50 ml) yielding 9.66 g (73%) of product.

*Step C:* Preparation of 6-(2-methyldioxolan-2-yl)-2-sulfamoylbenzo[b]thiophene

The title compound was prepared by the procedure used for the synthesis of 6-methoxy-2-sulfamoylbenzo[b]thiophene in Example 1, Step C.

After crystallization from ethyl acetate-hexanes 6.35 g (42%) of product was obtained, m.p. 127—128°C.

Example 38

6-Acetyl-2-sulfamoylbenzo[b]thiophene

The ketal product from Example 37 (4.4 g, 14.7 mmole) and p-toluenesulfonic acid (400 mg) were dissolved in acetone (90 ml). The mixture was stirred overnight. The solvent was removed *in vacuo* and the residue partitioned between ethyl acetate (200 ml) and 10% saturated $NaHCO_3$ solution (100 ml). The organic layer was washed with $H_2O$ (2 × 100 ml) and dried over $Na_2SO_4$. The solvent was removed *in vacuo* and the residue was triturated with hot $CHCl_3$ (100 ml). Upon cooling, 3.4 g (90.6%) of product was obtained, m.p. 154—155°C.

Example 39

6-(1-Hydroxyethyl)-2-sulfamoylbenzo[b]thiophene

The acetyl compound from Example 38 (1.79 g, 7 mmole) was dissolved in 40 ml of $CH_3OH$, cooled to 0°C and $NaBH_4$ (264 mg, 7 mmole) in 4 ml of 40% NaOH was added. The reaction mixture was stirred for 25 minutes then 2.5 g of $NH_4Cl$ was added followed by 1 ml glacial acetic acid. The solvent was removed *in vacuo* and the residue partitioned between ethyl acetate (75 ml) and 20% saturated $NaHCO_3$ (50 ml). The ethyl acetate layer was washed with $H_2O$ (50 ml) and the combined aqueous solution was back-extracted with ethyl acetate. The ethyl acetate extracts were combined and dried over $Na_2SO_4$. The solvent was removed *in vacuo* and the residue was crystallized from $CHCl_3$-dichloroethane yielding 1.56 g (86.7%) of product, m.p. 134—35°C.

Example 40

4-Chloro-5-hydroxy-2-sulfamoylbenzo[b]thiophene

A stirred solution of 5-hydroxy-2-sulfamoylbenzo[b]thiophene (3.0 g, 0.013 mole) in $CH_3CN$ (60 ml) was treated with N,N-dimethylformamide dimethylacetal (1.75 ml, 0.013 mole). Within 5 minutes, the protected sulfonamide started to precipitate. After 10 minutes, this was redissolved by the addition of $CH_3CN$ (65 ml). N-Chlorosuccinimide, (1.91 g, 0.0143 mole) was added in one portion and the mixture was stirred at ambient temperature. After 2 days, another 175 mg of N-chlorosuccinimide was added and stirring was continued for another 3 days. The solvent was evaporated under reduced pressure and the residual solid was triturated with $H_2O$ (100 ml) and collected. This solid then was suspended in 6N HCl (70 ml) and heated on the steam bath for 6½ hours. The resulting solid was extracted into ethyl acetate. Evaporation of the

washed and dried extract under reduced pressure left 3.1 g (90%) of 4-chloro-5-hydroxy-2-sulfamoylbenzo-[b]thiophene, m.p. 198—201°C. Recrystallization from $H_2O$, decolorizing with charcoal, gave 2.6 g, m.p. 203—205°C.

Example 41

6-Hydroxy-2-sulfamoylbenzo[b]thiophene-6-sodium sulfate

A mixture of 3.00 g of 6-hydroxy-2-sulfamoylbenzo[b]thiophene and 3.00 g of sulfamic acid in 20 ml of dry pyridine were refluxed gently for 36 hours. At the end of the reaction, the pyridine was distilled from the mixture under vacuum at 50°C. The residue was dissolved in water and made basic by addition of concentrated ammonia. The solvent was evaporated. The product was separated from residual ammonium sulfamate by extraction into ethanol. The ethanol extract was filtered and evaporated to give 3.2 g of crude sulfate as the ammonium salt. The salt was dissolved in distilled water and titrated with 1 equivalent of sodium hydroxide. The solvent was evaporated leaving the crude sodium sulfate salt. A 2.64 g portion of the product was boiled with 40 ml of saturated sodium chloride solution and sufficient water was added to obtain a clear solution. Upon cooling, 2.00 g of a white solid separated. Elemental analysis showed the material to be a mixture of the desired product and 11.7% (by weight) sodium chloride.

| | | | | |
|---|---|---|---|---|
| Analysis calculated for $C_8H_6NNaO_6S_3.0.117NaCl$: | C, 25.60; | N, 3.73; | H, 1.61; | Cl, 7.12. |
| Found: | C, 25.87; | N, 3.97; | H, 1.54; | Cl. 7.12. |

Treating the ammonium salt produced in Example 41 with potassium chloride, tetramethylammonium chloride, pyridine, imidazole, pralidoxime chloride or thiamine in place of the sodium chloride used in Example 41 there are prepared the corresponding salts.

Example 42

6-Hydroxy-2-sulfamoylbenzo[b]thiophene-6-disodium phosphate

A solution of 2.5 g of 6-hydroxy-2-sulfamoylbenzo[b]thiophene in 10 ml of pyridine was added over a 1 minute period to a well-stirred solution of 1.02 ml of phosphorus oxychloride in pyridine (10 ml) at 0°C. After 15 to 30 minutes the reaction mixture was poured into ice-water and the resulting solution was stirred for 15 minutes. The solvents were evaporated under high vacuum on a rotary evaporator. The product was resuspended in water and the pH of the solution was adjusted to 7.8 ± 0.6. The solvents were removed and the solid dried under high vacuum. The solid was redissolved in 100 ml of distilled water. Gradual addition of 400 ml of acetone lead to precipitation of the title compound (1.50 g) as a monohydrate.

| | | | | |
|---|---|---|---|---|
| Elemental analysis calculated for $C_8N_6NNa_2O_6PS_2.H_2O$: | N, 3.77; | C, 25.88; | H, 2.17; | S, 17.27. |
| Found: | N, 3.85; | C, 25.64; | H, 2.09; | S, 17.32. |

Other salts of the phosphate group are obtained by using the appropriate hydroxide in place of sodium hydroxide in the procedure above, such as potassium hydroxide, tetramethylammonium hydroxide, pyridine, imidazole, pralidoxime hydroxide and thiamine.

Mixed esters of the type:

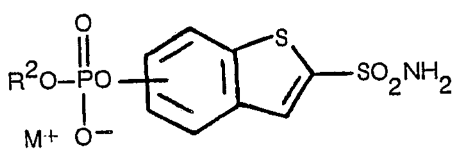

wherein $R^2$ is $C_{1-3}$alkyl or phenyl-$C_{1-3}$alkyl are prepared by reacting a hydroxy-2-sulfamoylbenzo[b]thiophene with an appropriate alkyldichlorophosphate; e.g. ethyldichlorophosphate, or benzyldichlorophosphate.

Example 43

6-Hydroxy-5-methoxy-2-sulfamoylbenzo[b]thiophene

*Step A:* Preparation of 5-(4-Hydroxy-3-methoxybenzylidene)rhodanine

A mixture of vanillin (30.4 g, 0.2 mol), rhodanine (26.6 g, 0.2 mol), anhydrous sodium acetate (41 g, 0.5 mol) and glacial acetic acid was stirred and heated at reflux for one and one-half hours. The cooled reaction mixture was added to water (1200 mL) and the yellow-orange solid that separated was collected, washed with water and air dried; yield 46 g.

*Step B:* Preparation of 3-(4-Hydroxy-3-methoxyphenyl)-2-mercaptoacrylic acid

The product from Step A was added to 20% sodium hydroxide solution (250 mL) and heated to 70—75°C with stirring for one hour. The resulting solution was chilled in an ice bath and added rapidly, with stirring, to cold 10% hydrochloric acid (350 mL). The yellow solid was collected, washed with water and dried at 50°C; yield 36 g.

*Step C:* Preparation of 6-Hydroxy-5-methoxybenzo[b]thiophene-2-carboxylic acid

Iodine (90 g) was added to a stirred mixture of the product from Step B (73 g) and 90% ethanol (2000 mL). The resultant dark mixture was heated at reflux for 24 hours. Saturated sodium bisulfite solution (200 mL) was added to the cooled reaction mixture and the ethanol was removed *in vacuo*. The aqueous residue was diluted to 1500 ml with water and extracted with ethyl acetate (4 × 400 mL). The combined extracts were evaporated *in vacuo* and the residue was heated on the steam bath with 1*N* sodium hydroxide solution (1500 mL) for 3 hours. The solution was treated with charcoal, filtered, diluted to 2500 mL with water and acidified with concentrated hydrochloric acid. The precipitated solid was collected, washed with water and dried; yield 36.8 g.

*Step D:* Preparation of 6-Hydroxy-5-methoxybenzo[b]thiophene

A mixture of the product from Step C (8 g), copper dust (2 g) and quinoline (40 mL) was heated under reflux for two hours. The hot reaction mixture was poured onto crushed ice (200 g), acidified with 6*N* hydrochloric acid and filtered. The filtrate was washed with saturated sodium chloride solution, dried ($Na_2SO_4$) and filtered and the solvent was evaporated *in vacuo*, leaving 5 g of waxy solid.

*Step E:* 6-Hydroxy-5-methoxy-2-sulfamoylbenzo[b]thiophene

To a cooled (−20°C) solution of 6-hydroxy-5-methoxybenzo[b]thiophene (15 g) in dry tetrahydrofuran (300 mL) was added butyl lithium (117 mL, 1.6*N* in hexane). After addition was complete, the reaction mixture was allowed to warm to 20°C and $SO_2$ gas was introduced over the surface of the stirred mixture. Addition was continued until an aliquot dissolved in water no longer tested alkaline (pH paper). After stirring for one hour, ether (500 mL) was added and the greyish-green solid was collected on a filter. This solid was added to a solution of sodium acetate (13.8 g) in water (500 mL) followed by the addition of hydroxylamine-O-sulfonic acid (41.7 g). After 24 hours, the solid was collected, washed with chloroform and recrystallized from water, 4.9 g, m.p. 201—204°C.

| | | | |
|---|---|---|---|
| Analysis calculated for $C_9H_9NO_4S_2$: | C, 41.69; | H, 3.50; | N, 5.40. |
| Found: | C, 42.50; | H, 3.55; | N, 5.38. |

Employing the procedures described in Example 43, Steps A through E, but starting with 3-hydroxy-4-methoxybenzaldehyde, there is produced 5-hydroxy-6-methoxy-2-sulfamoylbenzo[b]thiophene.

Example 44

5,6-Dihydroxy-2-sulfamoylbenzo[b]thiophene

A stirred mixture of 6-hydroxy-5-methoxysulfamoylbenzo[b]thiophene (4.9 g) and pyridine hydrochloride (15 g) was heated to 190—200°C for 1.5 hours. The warm reaction mixture was diluted with water (200 mL) and extracted with ethyl acetate (4 × 150 ml). The extracts were washed with water, saturated sodium chloride solution, dried ($Na_2SO_4$), filtered and evaporated under reduced pressure to give a tan solid residue of 1.8 g. Recrystallization from nitromethane gave material of m.p. 233—235°C.

| | | | |
|---|---|---|---|
| Analysis Calculated for $C_8H_7NO_4S_2$: | C, 39.16; | H, 2.88; | N, 5.71 |
| Found: | C, 38.85; | N, 2.74; | N, 5.77. |

Example 45

5-N,N-Dimethylcarbamoyloxy-2-sulfamoylbenzo[b]thiophene

A solution of 2.3 g (10 mmoles) of 5-hydroxy-2-sulfamoylbenzo[b]thiophene in 50 ml of pyridine was treated with 200 mg of 4-dimethylaminopyridine and finally 2.5 ml (2.9 g, 27 mmoles) of dimethylcarbamyl chloride. The resultant clear solution was stirred at room temperature for 48 hours, when it was poured into a mixture of 200 g of chopped ice and 100 ml of concentrated HCl. The precipitated solid was collected and washed with cold $H_2O$ until the washings were neutral. Drying gave 2.25 g (75%) of tan powder which was crystallized from isopropyl alcohol to give 1.6 g of product, m.p. 192—194°C.

Example 46

5-(Carboxymethoxy)-2-sulfamoylbenzo[b]thiophene

A suspension of 50% sodium hydride (0.32 g, 0.0066 m) in dry dimethylformamide (15 ml) was stirred at 25°C under nitrogen while N,N-dimethyl-N′-(5-hydroxybenzo[b]thiophene-2-sulfonyl)amidine (1.50 g, 0.0053 m) was added portionwise over 15 minutes. The mixture was stirred at 70°C for 15 minutes, then ethyl bromoacetate (1.10 g, 0.0066 m) was added rapidly and the mixture was heated at 70°C for 16.5 hours. The reaction was added to water (150 ml) and extracted with ethyl acetate (3 × 100 ml). The extracts were washed with $H_2O$, dried over $Na_2SO_4$ and concentrated under reduced pressure. The residue was stirred at 40°C for 4 hours in a mixture of 2N NaOH (14 ml) and methanol (7 ml). After evaporating the solvent under reduced pressure, the residue was dissolved in water (35 ml), acidified with 3N HCl and the solid was collected to yield 0.95 g (63%) of product melting at 200—203°C. An analytical sample melted at 202.5—204°C after recrystallization from nitromethane.

Example 47

5-(Carbamoylmethoxy)-2-sulfamoylbenzo[b]thiophene

A mixture of 5-(carboxymethoxy)-2-sulfamoylbenzo[b]thiophene (5.00 g, 0.017 m) in thionyl chloride (50 ml) was refluxed for one hour and then concentrated under reduced pressure. The residue was treated with cold concentrated ammonium hydroxide solution (75 ml) and stirred at 25°C for one hour. The solution was concentrated under reduced pressure for a few minutes until a solid began to precipitate. The solid was collected to afford 3.34 g (69%) of product melting at 197—198°C. An analytical sample melted at 200—201°C on recrystallization from 95% ethanol.

Example 48

5-(2-Aminoethoxy)-2-sulfamoylbenzo[b]thiophene Hydrochloride

A suspension of 5-(carbamoylmethoxy)-2-sulfamoylbenzo[b]thiophene (0.50 g, 0.0017 mole) in dry tetrahydrofuran was stirred and heated to reflux in an oil bath at 80°C under nitrogen while 10.0 M borane-dimethylsulfide complex (0.4 ml, 0.0040 m) was added over 20 minutes, allowing dimethylsulfide to distill from the reaction mixture using a Claisen distillation head. An additional 5 ml of tetrahydrofuran and 0.2 ml of 10.0 M borane-dimethylsulfide complex was added and the mixture was refluxed for one hour. After cooling to room temperature, methanol (3 ml) was added dropwise to decompose excess borane-dimethylsulfide complex and then 5 ml of ether saturated with hydrogen chloride was added and the mixture was stirred at 25°C for approximately 16 hours. The product was collected to give 0.37 g (71%) of material melting at 222—224°C. An analytical sample melted at 234—236°C after recrystallization from methanol-ether.

Example 49

6-Amino-2-sulfamoylbenzo[b]thiophene

*Step A:* Preparation of 6-Acetamido-2-sulfamoylbenzo[b]thiophene

A solution of 6-acetylbenzo[b]thiophene-2-sulfonamide (5.1 g, 0.02 mole) in glacial acetic acid (50 ml) and concentrated $H_2SO_4$ (20 ml) was stirred and heated to 65°C. Sodium azide (5.0 g, 0.077 mole) was added in portions over a 1 hour period. After heating the mixture at 80°C for another 3 hours, it was poured into a stirred, saturated NaOAc solution (500 ml) cooled in an ice bath. The resulting mixture was refrigerated ovenight. The solid was collected, resuspended in water (300 ml), and recollected to obtain 5.25 g (97%) of crude product, m.p. 214—217°C. Recrystallizations from $CH_3OH$ with charcoal decolorization and from $CH_3CN$ afforded 1.5 g of 6-acetamido-2-sulfamoylbenzo[b]thiophene, m.p. 231—232.5°C.

*Step B:* Preparation of 6-Amino-2-sulfamoylbenzo[b]thiophene

6-Acetamido-2-sulfamoylbenzo[b]thiophene (0.8 g, 3 mmole) was suspended in 1N HCl (24 ml). The stirred mixture was heated to refluxing for 1 hour when a clear solution was obtained. After dilution with water (25 ml), the cooled solution was neutralized with saturated $NaHCO_3$ solution. The product precipitated and was collected to obtain 0.57 g (83%), m.p. 240—241°C dec. This material was combined with 1.3 g of comparable crude product from two other runs and recrystallized from $CH_3CN$, decolorizing with charcoal, to yield 1.46 g of 6-amino-2-sulfamoylbenzo[b]thiophene, m.p. 239—240°C (dec).

Example 50

5-(2-Aminoethyl)-2-sulfamoylbenzo[b]thiophene

*Step A:* Preparation of 5-(2-Azidoethyl)-2-sulfamoylbenzo[b]thiophene

Under nitrogen, a solution of 5-(2-bromoethyl)-2-sulfamoylbenzo[b]thiophene (1.2 g, 0.0037 mol), $NaN_3$ (0.6 g, 0.0092 mol) and DMF (25 ml) was heated at 100°C. After 18 hours, the solution was cooled, poured into $H_2O$, and filtered to yield 0.8 g of product. The filtrate was extracted with ethyl acetate (2 ×). The organic layers were dried, filtered and concentrated to dryness to yield an additional 0.4 g of product (75%).

*Step B:* Preparation of 5-(2-aminoethyl)-2-sulfamoylbenzo[b]thiophene

A solution of product from Step A (1.2 g, 0.004 mol), ethanol (125 ml), $CHCl_3$ (2.2 ml), and 10% Pd on C (0.45 g) was hydrogenated on a Parr shaker at 55 psi. After overnight shaking, the contents were filtered under a blanket of $N_2$ through a filter aid pad. The solution was concentrated to dryness and the residue crystallized from $CH_3OH$-$CH_3CN$ to yield 0.62 g of product; m.p. 295—297°C.

Example 51

5-(Trifluoromethylimidazol-2-yl)-2-sulfamoylbenzo[b]thiophene

*Step A:* Preparation of 5-Formylbenzo[b]thiophene

Under $N_2$, NBS (6 g, 0.034 mol) was added to a solution of 5-methylbenzo[b]thiophene (30.8 g, 0.21 mol), benzoylperoxide (1.6 g) in $CCl_4$ (300 ml) and was heated at reflux. After 15 minutes, the remainder of the NBS (35 g, 0.2 mol) was added over a period of 5 minutes and heated at reflux for 1.5 hours. The reaction mixture was then cooled, filtered and the filtrate washed with $H_2O$ (2 ×), dried, filtered and concentrated to dryness. The residue was dissolved in $CHCl_3$ (130 ml) and added to a solution of hexamethylenetetramine (33.6 g, 0.24 mol) in $CHCl_3$ (60 ml). After 0.5 hour, the reaction mixture was cooled, filtered and the solid washed with hexane. After drying, the solid was dissolved in acetic acid-$H_2O$ (275 ml) and heated at reflux for 2 hours. Then concentrated HCl (55 ml) was added and the mixture refluxed an additional 5 minutes. After cooling, the solution was extracted with ether (3 ×), and the organic extracts were backwashed with saturated $Na_2CO_3$ (2 ×), dried, filtered, and concentrated to dryness to yield 16.4 g (48%) of product.

*Step B:* Preparation of 5-(1,3-Dioxolan-2-yl)benzo[b]thiophene

A solution of product from Step A (16.4 g, 0.1 mol), toluene (300 ml), p-toluenesulfonic acid (1.0 g) and ethylene glycol (20 ml) was heated at reflux with the aid of a Dean-Stark trap. After 6 hours, the organic layer was cooled, washed with saturated $Na_2CO_3$. The $Na_2CO_3$ layer was extracted with ethyl acetate (2 ×). The combined organic extracts were dried, filtered and concentrated to dryness. The residue was distilled at 149—153°C at 0.3 mm Hg to yield 16.4 g (79%) of product.

*Step C:* Preparation of 5-Formyl-2-sulfamoylbenzo[b]thiophene

Under $N_2$, a solution of product from Step B (6.2 g, 0.03 mol) in dry THF (75 ml) was cooled to less than −40°C and then n-butyl lithium (20 ml, 1.6*M* in hexane, 0.032 mol) was added dropwise maintaining the temperature below −40°C. After stirring for an additional 0.5 hour at about −40°C, $SO_2$ gas was introduced over the surface of the reaction for 20 minutes. Then, ether was added to the suspension and allowed to stir at ambient temperature over 1 hour. The solid was removed by filtration and dried. The solid was suspended in $CH_2Cl_2$ (150 ml) and the mixture cooled in an ice bath while adding N-chlorosuccinimide (4.0 g, 0.03 mol). After complete addition, the suspension was stirred at room temperature for 18 hours, when the suspension was filtered and concentrated to dryness. The residue was treated with acetone (50 ml) and aqueous $NH_3$ (50 ml), and the solution concentrated to remove the acetone. The resulting suspension was filtered and the resulting solid (5 g) was dissolved in acetone (90 ml) and 1*N* HCl (90 ml) and heated on a steam bath for 15 minutes. The mixture was poured into $H_2O$ and extracted with ethyl acetate (3 ×). The organic layers were dried, filtered and concentrated to dryness to yield 3.3 g (48%) of product.

*Step D:* Preparation of 5-(4-trifluoromethylimidazol-2-yl)-2-sulfamoylbenzo[b]thiophene

A solution of

O
||
$CF_3CCHBr_2$

(1.32 g, 0.005 mol), sodium acetate trihydrate (0.01 mol) in $H_2O$ (10 ml) was heated on a steam bath for 15 minutes and then added in one portion to a solution of the product from Step C (1.1 g, 0.005 mol) in $CH_3OH$ (40 ml) and concentrated aqueous $NH_3$ (10 ml). After stirring overnight at room temperature, an additional amount of

O
||
$CF_3CCHBr_2$

(1 g) was added. After 5 hours, the solvents were removed under reduced pressure and the residue chromatographed on silica gel eluting gradiently with 2% $CH_3OH$—$CHCl_3$ to 100% $CH_3OH$. The crude product was crystallized from $CH_3CN$ to yield 0.85 g (50%) of product; m.p. 285—286°C.

Example 52

5-[2-(Dimethylamino)ethylthiomethyl]-2-sulfamoylbenzo[b]thiophene

To a stirred solution of 2-dimethylaminoethanethiol hydrochloride (14.17 g, 0.10 mol) in 125 ml of dry DMF was added portionwise 60% sodium hydride in mineral oil (8.0 g, 0.2 mol) under a nitrogen atmosphere over ½ hour with warming on the steam bath. The mixture was stirred an additional ½ hour and then cooled in ice. To the cold suspension was added dropwise a solution of 5-bromomethyl-2-sulfamoylbenzo-[b]thiophene (7.66 g, 0.025 mol) in 25 ml of DMF. The mixture was stirred for an additional 1 hour at ice bath temperature. The mixture was filtered and the filtrate was concentrated *in vacuo* at 0.5 mm at room temperature. The solid residue was combined with the filtered solid and was dissolved in 3N HCl (100 ml). The acid solution was extracted with $CHCl_3$ (2 × 50 ml), neutralized with $NaHCl_3$ and concentrated to dryness *in vacuo*. The residual solid was extracted with $CHCl_3$ (3 × 100 ml). The $CHCl_3$ extracts were dried over anhydrous $Na_2SO_4$, filtered and concentrated *in vacuo* at room temperature. The solid obtained contained some polar impurity which was removed by trituration with hot water. There was recovered 2.8 g of product (Yield 34%); m.p. 132—133%.

Example 53

5-[2-(Dimethylamino)ethylsulfinylmethyl]-2-sulfamoylbenzo[b]thiophene

To a partial solution of 5-[2-(dimethylamino)ethylthiomethyl]-2-sulfamoylbenzo[b]thiophene (1.32 g, 0.004 mol) in 20 ml of ethanol and 40 ml of water was added sodium metaperiodate (0.94 g, 0.0044 mol) and the mixture was stirred at room temperature for 16 hours overnight. The reaction mixture was filtered and the filtrate was evaporated to dryness *in vacuo*. The white solid residue was recrystallized twice from 4/1 (V/V) water/ethanol and chromatographed on silica gel eluting with 7.5% (V/V) methanol/$CHCl_3$. There was recovered 0.88 g of white solid, m.p. 180—183°C, dec. (Yield 64%).

Example 54

5-[2-(Dimethylamino)ethylsulfonylmethyl]-2-sulfamoylbenzo[b]thiophene

To a solution of 5-[2-(dimethylamino)ethylthiomethyl]-2-sulfamoylbenzo[b]thiophene (2.34 g, 0.0064 mol) in 20 ml of water at 40—45°C was added potassium peroxymonosulfate, "oxone", (5.93 g, 0.0096 mol). The mixture was stirred at room temperature over night and heated on the steam bath for $2\frac{1}{2}$ hours. The mixture was cooled and basified with $NaHCO_3$. Filtration gave 2.5 g of crude product. Purification by silica gel, chromatography eluting with 10% (V/V) methanol/$CHCl_3$ gave 0.78 g of pale pink solid, m.p. 185.5—187°C, dec. (Yield 34%).

Example 55

5-(Carboethoxymethoxy)-2-sulfamoylbenzo[b]thiophene

A mixture of 5-(carboxymethoxy)benzo[b]thiophene-2-sulfonamide (1.50 g, 0.0052 m) in ethanol (15 ml) and concentrated sulfuric acid (3 ml) was refluxed with stirring for 5 hours. Ethanol was evaporated under reduced pressure, the residue was treated with $H_2O$ (25 ml) and extracted with ethyl acetate (50 ml and 2 × 25 ml). The combined extracts were washed with $H_2O$, saturated sodium bicarbonate solution (2 × 25 ml), and three times with $H_2O$. After drying over $Na_2SO_4$, the solvent was evaporated under reduced pressure to afford 1.43 g (87%) of product. Recrystallization from nitromethane afforded 1.12 g of analytically pure product melting at 167.5—168°C.

Example 56

5-(N-Hydroxycarbamoylmethoxy)-2-sulfamoylbenzo[b]thiophene

A warm solution of potassium hydroxide (2.58 g, 0.046 m) in methanol (7 ml) was added to a warm solution of hydroxylamine hydrochloride (1.60 g, 0.023 m) in methanol (10 ml) under nitrogen. After cooling in an ice bath for 10 minutes, the solid was filtered and washed with methanol. The combined filtrate and washings were added to a warm solution of 5-(carboethoxymethyl-2-sulfamoylbenzo[b]thiophene (3.57 g, 0.011 m) in methanol (170 ml) and the mixture was stirred at ambient temperature for 49 hours. The solid was collected, washed with ether, and then heated to reflux in 1.5N acetic acid (17 ml) until a clear solution resulted. After cooling to ambient temperature, the product was collected to yield 2.16 g (65%) of crude product melting at 138°C. Preparative TLC afforded 0.67 g of pure product melting at 153°C. An analytical sample melted at 156°C after recrystallization from nitromethane.

For use in treatment of conditions relieved by the inhibition of carbonic anhydrase, the active compound can be administered either systemically, or, in the treatment of the eye, topically. The dose administered can be from as little as 0.1 to 25 mg or more per day, singly, or preferably on a 2 to 4 dose per day regimen although a single dose per day is satisfactory.

When administered for the treatment of elevated intraocular pressure or glaucoma, the active compound is most desirably administered topically to the eye, although systemic treatment is, as indicated, also possible.

When given systemically, the drug can be given by any route, although the oral route is preferred. In oral administration the drug can be employed in any of the usual dosage forms such as tablets or capsules, either in a contemporaneous delivery or sustained release form. Any number of the usual excipients or tableting aids can likewise be included.

When given by the topical route, the active drug or an ophthalmologically acceptable salt thereof such as the sodium or potassium salt is formulated into an ophthalmic preparation. In such formulations, from 0.1% to 15% by weight can be employed. The objective is to administer a dose of from 0.1 to 10 mg per eye per day to the patient, with treatment continuing so long as the condition persists.

Thus, in an ophthalmic solution, insert, ointment or suspension for topical delivery, or a tablet, intramuscular, or intravenous composition for systemic delivery, the active medicament or an equivalent amount of a salt thereof is employed, the remainder being carrier, excipients, preservatives and the like as are customarily used in such compositions.

The active drugs of this invention are most suitably administered in the form of ophthalmic pharmaceutical compositions adapted for topical administration to the eye such as a suspension, ointment, or as a solid insert. Formulations of these compounds may contain from 0.01 to 15% and especially 0.5% to 2% of medicament. Higher dosages as, for example, about 10%, or lower dosages can be employed provided the dose is effective in reducing or controlling elevated intraocular pressure. As a unit dosage from between 0.001 to 10.0 mg, preferably .005 to 2.0 mg, and especially 0.1 to 1.0 mg of the compound is generally applied to the human eye, generally on a daily basis in single or divided doses so long as the condition being treated exists.

These hereinbefore described dosage values are believed accurate for human patients and are based on the known and presently understood pharmacology of the compounds, and the action of other similar entities in the human eye. They reflect the best mode known. As will all medications, dosage requirements are variable and must be individualized on the basis of the disease and the response of the patient.

The pharmaceutical preparation which contains the active compound may be conveniently admixed with a non-toxic pharmaceutical organic carrier, or with a non-toxic pharmaceutical inorganic carrier. Typical of pharmaceutically acceptable carriers are, for example, water, mixtures of water and water-miscible solvents such as lower alkanols or aralkanols, vegetable oils, polyalkylene glycols, petroleum based jelly, ethyl cellulose, ethyl oleate, carboxymethylcellulose, polyvinylpyrrolidone, isopropyl myristate and other conventionally employed acceptable carriers. The pharmaceutical preparation may also contain non-toxic auxiliary substances such as emulsifying, preserving, wetting agents, bodying agents and the like, as for example, polyethylene glycols 200, 300, 400 and 600, carbowaxes 1,000, 1,500, 4,000, 6,000 and 10,000, antibacterial components such as quaternary ammonium compounds, phenylmercuric salts known to have cold sterilizing properties and which are non-injurious in use, thimerosal, methyl and propyl paraben, benzyl alcohol, phenyl ethanol, buffering ingredients such as sodium chloride, sodium borate, sodium acetates, gluconate buffers, and other conventional ingredients such as sorbitan monolaurate, triethanolamine, oleate, polyoxyethylene sorbitan monopalmitylate, dioctyl sodium sulfosuccinate, monothioglycerol, thiosorbitol, ethylenediamine tetraacetic acid, and the like. Additionally, suitable ophthalmic vehicles can be used as carrier media for the present purpose including conventional phosphate buffer vehicle systems, isotonic boric acid vehicles, isotonic sodium chloride vehicles, isotonic sodium borate vehicles and the like.

The pharmaceutical preparation may also be in the form of a solid insert such as one which after dispensing the drug remains essentially intact, or a bio-erodible insert that either is soluble in lacrimal fluids, or otherwise disintegrates.

The following examples of ophthalmic formulations are given by way of illustration.

Example 57

| | | |
|---|---|---|
| 6-(2-Sulfamoylbenzo[b]thienyl)-2,2-dimethyl propionate (I) | 1 mg. | 15 mg. |
| Monobasic sodium phosphate $.2H_2O$ | 9.38 mg. | 6.10 mg. |
| Dibasic sodium phosphate $.12H_2O$ | 28.48 mg. | 16.80 mg. |
| Benzalkonium chloride | 0.10 mg. | 0.10 mg. |
| Water for injection q.s. ad. | 1.0 ml. | 1.0 ml. |

Compound I, phosphate buffer salts, and benzalkonium chloride are added to and dissolved in water. The pH of the composition is adjusted to 6.8 and diluted to volume. The composition is rendered sterile by ionizing radiation.

Example 58

| | |
|---|---|
| 6-(2-Sulfamoylbenzo[b]thienyl) 2-methylpropionate (II) | 5 mg. |
| petrolatum q.s. ad. | 1 gram |

Compound II and the petrolatum are aseptically combined.

Example 59

| | |
|---|---|
| 6-(2-Sulfamoylbenzo[b]thienyl) 2-methylpropionate | 1 mg. |
| Hydroxypropylcellulose q.s. | 12 mg. |

Ophthalmic inserts are manufactured from compression molded films which are prepared on a Carver Press by subjecting the powdered mixture of the above ingredients to a compressional force of 12,000 lbs. (gauge) at 300°F for one to four minutes. The film is cooled under pressure by having cold water circulate in the platen. Ophthalmic inserts are then individually cut from the film with a rod-shaped punch. Each insert is placed into a vial, which is then placed in a humidity cabinet (88% R.H. at 30°C) for two to four days. After removal from the humidity cabinet, the vials are stoppered and then capped. The vials containing the hydrate insert are then autoclaved at 250°F for ½ hour.

Example 60

| | |
|---|---|
| 6-(2-Sulfamoylbenzo[b]thienyl) acetate | 1 mg. |
| Hydroxypropyl cellulose q.s. ad. | 12 mg. |

Ophthalmic inserts are manufactured from a solvent cast film prepared by making a viscous solution of the powdered ingredients listed above using methanol as the solvent. The solution is placed on a Teflon plate and allowed to dry at ambient conditions. After drying, the film is placed in a 88% R.H. cabinet until it is pliable. Appropriately sized inserts are cut from the film.

Example 61

| | |
|---|---|
| 6-(2-Sulfamoylbenzo[b]thienyl) acetate | 1 mg. |
| Hydroxypropyl methyl cellulose q.s. ad. | 12 mg. |

Ophthalmic inserts are manufactured from a solvent cast film which is prepared by making a viscous solution of the powdered blend of the above ingredients using a methanol/water solvent system (10 ml. methanol is added to 2.5 g. of the powdered blend, to which 11 ml. of water (in three divided portions) is added. The solution is placed on a Teflon plate and allowed to dry at ambient conditions. After drying, the film is placed in an 88% R.H. cabinet until it is pliable. Appropriately sized inserts are then cut from the film.

Example 62

| | |
|---|---|
| 6-(2-Sulfamoylbenzo[b]thienyl) acetate | 1 mg. |
| Hydroxypropylmethyl cellulose q.s. ad. | 12 mg. |

Ophthalmic inserts are manufactured from compression molded films which are prepared on a Carver Press by subjecting the powdered mixture of the above ingredients to a compressional force of 12,000 lbs. (gauge) at 350°F for one minute. The film is cooled under pressure by having cold water circulate in the platen. Ophthalmic inserts are then individually cut from the film with a punch. Each insert is placed into a vial, which is then placed in a humidity cabinet (88% R.H. at 30°C) for two to four days. After removal from the humidity cabinet, the vials are stoppered and then capped. The vials containing the hydrated insert are then autoclaved at 250°F for one-half hour.

It is highly preferred that the solid inserts of this invention are available for use by the patient in a pathogen free condition. Thus, it is preferred to sterilize the inserts and to insure against recontamination, the sterilization is preferably conducted after packaging. The best mode of sterilizing is to employ ionizing radiation including radiation emanating from Cobalt 60 or high energy electron beams.

The following examples illustrate preparation of the improved ophthalmic suspension compositions of the present invention.

Example 63

The following materials are admixed in a 1250 ml bottle: 24 g of 6-(2-sulfamoylbenzo[b]thienyl) 2,2-dimethylpropionate which is a sufficient amount of medicament to result in a concentration of 10 mg per ml in the final samples, allowing for previously established 3.0% average; 0.4 g sodium bisulfite, 12 g NaCl, and 28 ml water (at 180°F). This mixture, (I), is autoclaved for 30 minutes at 121°C under 15 psig. Separately, 3 g of hydroxyethylcellulose in 720 ml of water (II) and 0.4 g of lecithin in 80 ml of water (III) were autoclaved for 30 minutes at 121°C. Then, (III) is admixed with (I) for 2 hours, and the resultant mixture poured into (II). Another mixture (IV) is prepared from 20 g of sorbitol, 2.36 ml of benzalkonium chloride, 10 g of disodium edetate, and water to give a final solution volume of 900 ml. Then, (IV) is added to the mixture of (I), (II), and (III) in sufficient quantity to give 1.8 l. overall. The 1.8 l. mixture of I, II, III, and IV is then taken and homogenized using a homogenizer at 2000 psig. Stock solutions are then prepared for polyoxyethylene (20) sorbitan monooleate by dissolving 3 g of the material in 100 ml of water, and of benzyl alcohol/β-phenyl-ethyl alcohol by admixing 50 ml of each alcohol. Varying quantities of the two stock solutions are then added to four 90 ml aliquots of the homogenized mixture of (I), (II), (III), and (IV) prepared as described above, together with sufficient water to give a total of 100 ml for each of four different samples.

Other formulations, in an oil vehicle and an ointment are exemplified in the following examples.

Example 64

*Solution Composition*

| | |
|---|---|
| 6-(2-Sulfamoylbenzo[b]thienyl) 2,2-dimethylpropionate | 0.1 mg. |
| Peanut oil q.s. ad. | 0.10 mg. |

The solution is rendered sterile by filtration through a sterilizing filter.

Example 65

| | |
|---|---|
| 6-(2-Sulfamoylbenzo[b]thienyl) cyclopentaneacetate | 0.5 mg. |
| Petroplatum q.s. ad. | 1 gram |

The compound and the petrolatum are aseptically combined.

One of the novel compounds of this invention of particular importance has structural formula:

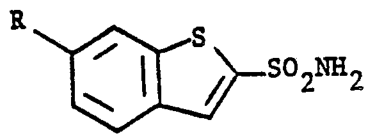

or a pharmaceutically acceptable salt thereof, wherein R is hydroxy or acetoxy, and especially hydroxy. Example 1 describes the preparation of the compound wherein R is hydroxy, and Example 4, describes the preparation of the compound wherein R is acetoxy by acylation of the hydroxy compound.

The following description and Examples provide alternate processes for the preparation of the preferred 6-hydroxy-2-sulfamoylbenzo[b]thiophene which as indicated above may be acetylated.

1. Reduction of the thiophene-1,1-dioxide

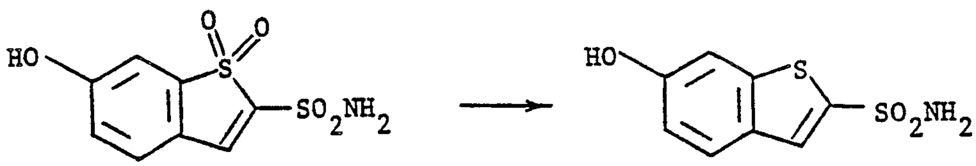

This reduction may be accomplished with zinc in an acidic medium such as acetic acid, hydrochloric acid, or mixtures thereof.

Example 66

*Step A:* 6-hydroxybenzo[b]thiophene-1,1-dioxide

A solution of 10.0 gm of 6-hydroxybenzo[b]thiophene (0.067 mol) in acetic acid is treated with 2.05 equivalent of peracetic acid. When the reaction is complete, as ascertained by TLC analysis, the mixture is diluted with water and the product extracted into ethyl acetate. The extract is washed with $NaHCO_3$ solution, brine, dried and evaporated to dryness. The residue is used directly in the next step.

*Step B:* 2,3-dihydro-6-hydroxybenzo[b]thiophene-1,1-dioxide

The crude product from Step A is reduced with zinc dust and sodium hydroxide. The mixture is extracted with ether, washed with HCl, brine and dried. Evaporation of the solvent provides the product which is used without purification in the next step.

*Step C:* Lithium 2,3-dihydro-6-hydroxybenzo[b]thienylsulfinate-1,1-dioxide

The sulfone produced in Step B is converted to the dianion with 2 equivalents of n-BuLi in THF at −20°C. Sulfur dioxide (one equiv.) gas is introduced maintaining a temperature of less than −15°C. The mixture is allowed to warm to room temperature and the sulfinic acid salt is isolated by filtration.

*Step D:* 2-chlorosulfonyl-2,3-dihydro-6-hydroxybenzo[b]thiophene-1,1-dioxide

The product of Step C is suspended in methylene chloride, and one equivalent of acetic acid is added. N-chlorosuccinimide (1.05 equivalent) is added at 0—5°C. The mixture is stirred while warming to room temperature. The suspension is filtered and the filtrate evaporated to give the sulfonyl chloride.

*Step E:* 2,3-dihydro-6-hydroxy-2-sulfamoylbenzo[b]thiophene-1,1-dioxide

The crude sulfonyl chloride from Step D is dissolved in acetone and added to a well stirred solution of excess $NH_4OH$ in acetone at 0°C. The acetone is evaporated and the residue partitioned between ethyl acetate and 1N HCl. The extract is washed and dried. Evaporation of the solvent provides the product which is used directly in the next step.

*Step F:* 6-hydroxy-2-sulfamoylbenzo[b]thiophene-1,1-dioxide

A solution of the product from Step E in dioxane is treated with a slight excess of 2,3-dichloro-5,6-di-

cyanobenzoquinone at room temperature to reflux. Upon completion of the reaction, the mixture is cooled and partitioned between water and ethyl acetate. The extract is washed and dried and the product obtained by evaporation of the solvent is purified by chromatography.

*Step G:* 6-hydroxy-2-sulfamoylbenzo[b]thiophene

The purified product from Step F is treated with excess zinc in acetic acid containing aqueous HCl. The mixture is heated on a steam bath until reaction is complete. The mixture is cooled and poured into ice-water. The product is extracted into ethyl acetate. The extract is washed with dilute HCl, $NaHCO_3$ solution and brine. After drying, the solvent is removed to provide the title compound.

2. Oxidation of the 2-sulfenamide to the 2-sulfonamide

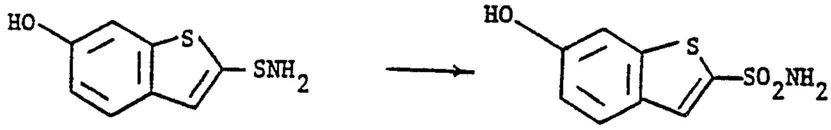

The sulfenamine is oxidized with potassium permanganate in an inert solvent such as water/acetone mixtures of about pH 8 at about 5 to about 50°C for about 0.5 to about 8 hours. Other suitable oxidizing agents are hydrogen peroxide, or peracids such as m-chloroperbenzoic, or trifluoroperacetic acid.

Example 67

*Step A:* 6-hydroxy-2-mercaptobenzo[b]thiophene

Butyllithium (2 mmoles, 1.6*M* solution in hexane) is added under nitrogen to a stirred solution of 6-hydroxybenzo[b]thiophene (1 mmole) in THF (5 ml) at −20°C. The reaction mixture is allowed to warm to 0°C and propylene sulfide (1.1 mmole) in THF (1 mL) is added dropwise. After an additional hour at 0°C, the mixture is stirred one hour at 25°C. The solvent is removed *in vacuo*, the residue is taken up in water and acidified with 1N hydrochloric acid. The aqueous mixture is extracted with ethyl acetate and the combined extracts are washed with water and saturated brine. After drying over sodium sulfate, the solvent is removed and the residue is used directly in the next step.

*Step B:* 6-hydroxybenzo[b]thiophene-2-sulfenamide

Sodium hypochlorite (5.25% solution, 2.4 mL) is added dropwise to a stirred solution of the product from Step A (2 mmols) and sodium hydroxide (2.1 mmols) in concentrated aqueous ammonia (50 mL) at 0°C. The solid that separates is collected, washed well with water and used directly in the next step.

*Step C:* 6-hydroxy-2-sulfamoylbenzo[b]thiophene

To a stirred mixture of the product from Step B in water (40 mL) and actone (40 mL) is added dropwise a solution of potassium permanganate (2.3 mmols) in water (75 mL). The pH of the mixture is maintained at 8.0 to 8.1 by periodic addition of 1N sulfuric acid. Stirring is continued for an additional hour and the mixture is treated with 10N sodium hydroxide solution (5 mL) decolorizing charcoal and filtered. The filtrate is acidified to pH 2 with concentrated hydrochloric acid and the solid that precipitates is filtered, washed with water and dried to give the title compound.

3. Dehalogenation

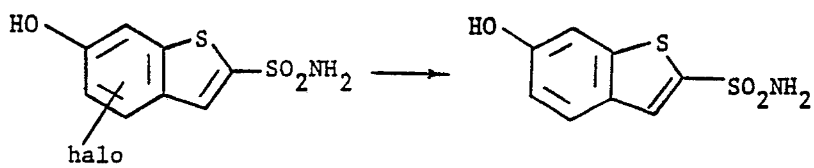

Aromatic halogens such as chloro, bromo or iodo may be removed by hydrogenolysis under the influence of a noble metal catalyst such as palladium on charcoal, platinum oxide, rhodium on carbon, or ruthenium on carbon in an inert solvent such as ethyl acetate, acetic acid, a $C_{1-3}$alkanol; water; esters such as THF, dioxane; or aromatic solvents such as benzene, or toluene; or DMF or hexanethyl phosphoric acid triamide; at or near atmospheric pressure and about 5 to 35°C, until the requisite amount of hydrogen is consumed.

Removal of halogen from aromatic rings can also be accomplished by various reducing agents, among them $Ph_3SnH$, HI, Sn and HBr, or HCl, $Ph_3P$, Cu and $H_2O$, hydrazine and Pd—C, $LiAlH_4$, $NaAlH_4$, t-BuOK in 1:1 t-BuOH—$Me_2SO$, $NaBH_4$ in the presence of Pd—C, NaH, and Raney nickel in alkaline solution, the latter method being effective for fluorine as well as for the other halogens. Photochemical reduction is also known. Halogen can also be removed from aromatic rings indirectly, by conversion to Grignard reagents followed by hydrolysis.

Example 68

*Step A:* 5-chloro-6-hydroxybenzo[b]thiophene-2-sulfonic acid sodium salt

Concentrated sulfuric acid (1.1 mL, 1.96 g, 0.02 mol), diluted with 5 mL of ethyl acetate is added to a cold (0—5°C) stirred solution of 5-chloro-6-hydroxybenzo[b]thiophene and acetic anhydride (4 mL) in ethyl acetate (10 mL). After one hour, the solvents are removed and the residue is treated with ice-water and saturated sodium chloride solution. The solid that precipitates is collected and dried.

*Step B:* 5-chloro-6-hydroxybenzo[b]thiophene-2-sulfonyl chloride

The product from Step A is treated with a mixture of phosphorus pentachloride and phosphorous oxychloride on the steam bath for one hour. The volatiles are removed *in vacuo* and the residue used directly in the next step.

*Step C:* 5-chloro-6-hydroxy-2-sulfamoylbenzo[b]thiophene

The product from Step B is dissolved in acetone and added to cold (0.5°C) concentrated aqueous ammonia. After one hour the solid is collected, washed with water and dried.

*Step D:* 6-Hydroxy-2-sulfamoylbenzo[b]thiophene

To a solution of 5-chloro-6-hydroxy-2-sulfamoylbenzo[b]thiophene in ethanol is added 10% palladium on charcoal and the mixture is shaken under three atmospheres of hydrogen until one molar equivalent of hydrogen is absorbed. The catalyst is filtered from the reaction mixture and the solvent is removed *in vacuo* to give the desired product.

Example 69

*Step A:* (4-Methoxyphenyl)acetylene sulfinic acid lithium salt

A solution of 11.9 g (0.09 mole) of (4-methoxyphenyl)acetylene in 100 ml of THF is cooled to −70°C. Fifty-seven mL of 1.6N butyllithium in hexane is added dropwise while holding the temperature at −50°C. After aging for 30 minutes sulfur dioxide gas is bubbled into the solution at −35 to −40°C. The precipitated salt is filtered off, washed with THF and dried to give (4-methoxyphenyl)acetylene sulfinic acid lithium salt.

*Step B:* (4-Methoxyphenyl)acetylene sulfonyl chloride

The above lithium salt, 16.7 g (0.09 mole) is suspended in 150 ml of methylene chloride and cooled to 0—5°C. N-chlorosuccinimide (13.2 g, 0.095 mole) is added over 10 minutes. The heterogeneous mixture is warmed to 25°C and aged at 25°C for 2 hours. The insolubles are filtered off and washed with 25 ml of methylene chloride. The filtrate is concentrated *in vacuo* to give (4-methoxyphenyl) acetylenesulfonyl chloride.

*Step C:* (4-Methoxyphenyl)acetylene sulfonamide

A solution of 15 g (0.07 mole) of (4-methoxyphenyl)acetylene sulfonyl chloride in 200 ml of acetone is cooled to 0—5°C and 20 ml of conc. ammonium hydroxide is added over 15 minutes. The mixture is concentrated *in vacuo* to dryness and triturated with 100 ml of water. The slurry is filtered and dried to give (4-methoxyphenyl)acetylene sulfonamide.

*Step D:* 3-Bromo-6-methoxy-2-sulfamoylbenzo[b]thiophene

To a solution of 20 ml of liquid sulfur dioxide in 100 ml of ether is added 12 g (0.06 mole) of (4-methoxyphenyl)acetylenesulfonamide. Anhydrous hydrogen bromide (6 g) is bubbled into the above solution over one hour at 0—5°C. The mixture is warmed to 25°C over 30 minutes and aged at 25°C for 2 hours. The excess HBr and ether is evaporated to give 3-bromo-6-methoxy-2-sulfamoylbenzo[b]thiophene.

*Step E:* 3-Bromo-6-hydroxy-2-sulfamoylbenzo[b]thiophene

A mixture of 12.9 g (0.05 mole) of 3-bromo-6-methoxy-2-sulfamoylbenzo[b]thiophene and 40 g of pyridine hydrochloride is stirred at 190°C for 2 hours. The melt, on cooling, is mixed with 100 ml of saturated sodium chloride solution and extracted with 2 × 200 ml of ethyl acetate. The organic layer is dried over anhydrous magnesium sulfate and after filtration concentrated *in vacuo* to give 3-bromo-6-hydroxy-2-sulfamoylbenzo[b]thiophene.

*Step F:* 6-Hydroxy-2-sulfamoylbenzo[b]thiophene

3-Bromo-6-hydroxy-2-sulfamoylbenzo[b]thiophene 12.2 g (0.05 mole), 25 g of granulated tin and 125 ml of concentrated hydrochloric acid in 400 ml of ethanol are refluxed for 3 hours. On cooling the insolubles are filtered off and the filtrate is concentrated to remove ethanol. The slurry is filtered, washed with water and dried to give 6-hydroxy-2-sulfamoylbenzo[b]thiophene.

4. Amination of a sulfonyl chloride

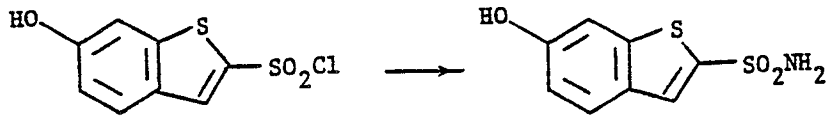

This amination is accomplished by treatment with ammonia; either by anhydrous ammonia being admitted to a solution of the sulfonyl chloride in an appropriate solvent such as THF, or acetone at about −10°C to +10°C until the reaction is complete; or by adding a solution of the sulfonyl chloride in one of those solvents to an excess of aqueous ammonia at about 10°C to 30°C. or to liquid ammonia.

Example 70

*Step A:* 6-hydroxybenzo[b]thienyl-2-sulfinic acid, dilithium salt

A THF solution of 6-hydroxybenzo[b]thiophene (1.50 gm, 0.010 mol) is treated with 2 equiv. of n-BuLi at 0°C. After 1 hour, one equivalent of $SO_2$ is introduced into the flask, slowly with cooling to maintain a temperature of −10°C or less. The mixture is stirred for one hour while warming to room temperature. The product sulfinic acid salt is isolated by filtration.

*Step B:* 6-hydroxybenzo[b]thienyl-2-sulfonyl chloride

The sulfinic acid salt is suspended in 15 mL $CH_2Cl_2$ and treated with 1 equiv. of acetic acid. The mixture is cooled to 0—5°C and N-chlorosuccinimide (1.40 gm, 1.05 equiv.) is added in a single portion. After 1 hour the mixture is filtered and the solvent is evaporated to provide the desired sulfonyl chloride.

*Step C:* 6-hydroxy-2-sulfamoylbenzo[b]thiophene

The crude sulfonyl chloride from Step B may be converted to the title compound by either of two methods:

1) The sulfonyl chloride is dissolved in THF and cooled to 0°C. Anhydrous ammonia gas is bubbled slowly into the solution until the sulfonyl chloride is consumed.

2) The sulfonyl chloride is added to excess aqueous ammonia in THF or acetone.

In either case, the crude reaction mixture is concentrated to dryness and the residue partitioned between dilute acid and ethyl acetate. The organic phase is separated, washed and dried. Evaporation provides the title compound.

5. Conversion of the sulfinic acid salt to the sulfonamide

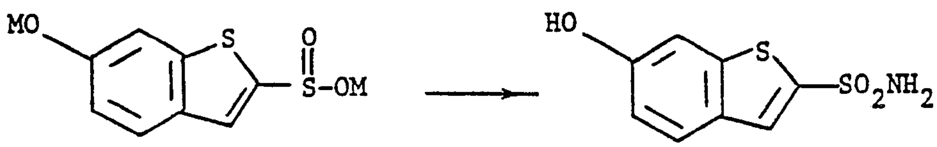

wherein M is an alkali metal cation such as sodium or potassium.

The novel process comprises treatment of the salt with hydroxylamine-O-sulfonic acid and is conducted in aqueous medium at temperature between about 0°C and 35°C. Although a higher temperature may be employed it is unnecessary and may result in lower yields. The reaction proceeds quickly and exothermically so that only a few minutes are required but times of up to several hours are not detrimental.

Equimolar amounts of the sulfinic acid salt and the hydroxylamine-O-sulfonic acid or a slight excess of the latter are recommended. It is possible to use excesses of hydroxylamine-O-sulfonic acid but it is wasteful and unnecessary.

The reaction is conducted in the presence of a buffering agent in sufficient amount to maintain pH 3—9. The preferred buffer is sodium acetate and about 1—3 moles of it per mole of sulfinic acid salt is found appropriate. Other reagents for carrying out this transformation are chloramine, and related sources of electrophilic nitrogen species including arylsulfonyl esters of hydroxylamine.

Example 71

The crude sulfinic acid salt is dissolved in water containing acetic acid (10 mmol) and sodium acetate (10 mmol). With external cooling to 18 to 20°C hydroxylamine-O-sulfonic acid (1.15 gm, 0.010 mol) is added in a single portion. After the reaction subsides, the mixture is cooled to 5—10°C and the product sulfonamide is isolated by filtration.

6. Removal of Protecting Groups from the Sulfonamide Nitrogen

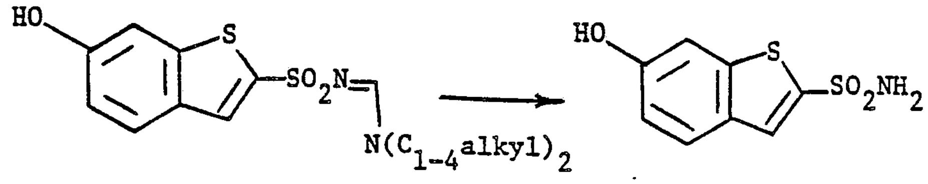

Protective groups such as the (N,N-dimethyl) in ethylidene are removed by treatment with a dilute alkali such as sodium hydroxide, or potassium hydroxide at about 25°C to 100°C for about 0.5 to about 12 hours, preferably about 60°C for about 2—4 hours.

Alternatively, the group is also removed by refluxing in 1—2N HCl for about 1 to 12 hours, preferably about 3 to 6 hours.

Example 72

N,N-dimethyl-N'-[6-hydroxybenzo[b]thiophene-2-sulfenyl]formamidine (10 mmol) (Example 11, Step A) in methanol (50 mL) and 2*N* NaOH (50 mL) is warmed at 60°C for 2—4 hours. The reaction mixture is acidified with conc. HCl and extracted with ethyl acetate. Evaporation of the solvent and crystallization of the residue from 1,2-dichlorethane provides the desired product.

7. Ring Closure of the thiophene ring

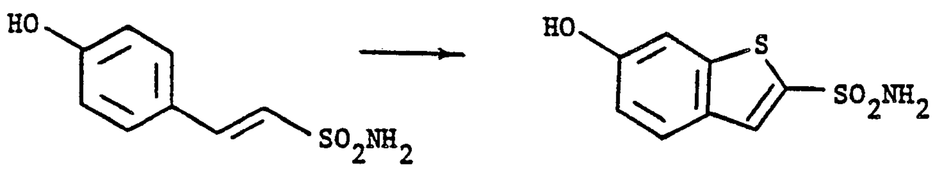

The ring closure is accomplished by treating the ethenesulfonamide with anhydrous hydrogen halide, such as HBr, HCl, or HI in liquid sulfur dioxide followed by heating in a sealed container at about 25°C to 100°C, preferably at about 50°C, for about 1 to 8 hours, preferably about 2 hours. The reaction can be run neat or in solvents such as ether, 1,2-dimethoxyethane, THF or the like; esters such as isopropyl acetate, ethyl acetate or the like; or in ketones such as acetone, or methyl ethyl ketone.

Example 73

*Step A:* 1-(4-Methoxyphenyl)ethene-2-sulfonic acid sodium salt

(4-Methoxyphenyl)acetylene (13.2 g, 0.1 mole) is added to a solution of 10.4 g (0.1 mole) of sodium bisulfite in 100 ml of water at 25°C. With vigorous stirring, oxygen is bubbled through the mixture for 2 hours. The reaction mixture is then concentrated *in vacuo* to give 1-(4-methoxyphenyl) ethene-2-sulfonic acid sodium salt.

*Step B:* 1-(4-Methoxyphenyl)ethene-2-sulfonamide

To a mixture of 11.8 g (0.05 mole) of 1-(4-methoxyphenyl)ethene-2-sulfonic acid sodium salt on 50 ml of DMF at 0—5°C is added 6.35 g (0.05 mole) of oxalyl chloride dropwise over 30 minutes. The reaction is aged at 0—5° for 30 minutes and then added to 30 ml of concentrated ammonium hydroxide. The reaction is diluted with 150 ml of water. The precipitated solid is washed with water and dried to give 1-(4-methoxyphenyl)ethene-2-sulfonamide.

*Step C:* 1-(4-Hydroxyphenyl)ethene-2-sulfonamide

A mixture 21.3 g (0.10 mole) of 1-(4-methoxyphenyl)ethene-2-sulfonamide and 60 g of pyridine hydrochloride is heated to 180°C and stirred at that temperature for 2 hours. The reaction mixture is cooled to 25°C and mixed with 500 ml of saturated sodium chloride solution. The product is filtered, washed with 35 ml of water and dried to give 1-(4-hydroxyphenyl)ethene-2-sulfonamide.

*Step D:* 6-Hydroxy-2-sulfamoylbenzo[b]thiophene

1-(4-hydroxyphenyl)ethene-2-sulfonamide (10.0 g, 0.05 mole) is added to a solution of 20 ml of liquid sulfur dioxide and 6 g of anhydrous hydrogen bromide at 0°C. This mixture, in a sealed tube is then warmed to 50°C. After 2 hours at 50°C, the excess hydrogen bromide and sulfur dioxide are evaporated. The organic layer is washed with water, dried over anhydrous magnesium sulfate and concentrated *in vacuo* to give the desired product.

8. Introduction of 6-hydroxy group

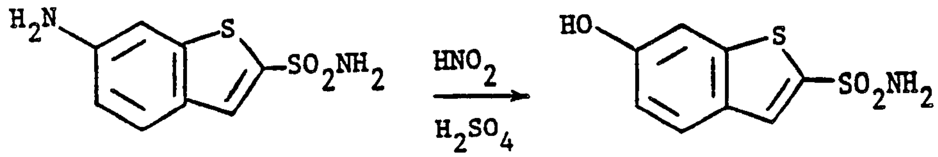


The 6-hydroxy group may be introduced by diazotization of a 6-amino followed by hydrolysis of the diazonium salt.

The diazotization may be conducted by any of the well known procedures, such as with sulfuric acid/sodium nitrite at about −25°C to +10°C; or hydrochloric acid/sodium nitrite at −25°C to +10°C.

Hydrolysis of the diazonium salt to the hydroxyl group may be accomplished with about 10N sulfuric acid or concentrated hydrochloric at about 75°C to the reflux temperature; by treating the diazonium salt with $NaBF_4$ to form the diazonium fluoborate which is then decomposed by refluxing in excess trifluoroacetic acid; by treating the diazonium fluoborate salt with about 0.1*N* sulfuric acid to about 35° to 55°C for about 12 to 24 hours, optionally in the presence of copper powder or cuprous oxide which reduces the reaction time to about 5—10 minutes, or optionally in the presence of dioxane and cupric nitrate, cupric sulfate, or cupric acetate at about 20—35°C for about 1 to 8 hours.

Example 74

6-Amino-2-sulfamoylbenzo[b]thiophene (11.2 g, 0.05 mole) is dissolved in a solution of 36 ml of conc. sulfuric acid in 64 ml of water, cooled to 0°C and diazotized with 8 g of sodium nitrite in 40 ml of water. The diazonium solution is then added to a refluxing solution of 80 ml of conc. sulfuric acid in 60 ml of water. After 15 minutes, the precipitated solid is filtered off, washed with water and dried to give 6-hydroxy-2-sulfamoylbenzo[b]thiophene.

9. Conversion of a sulfonic acid group and derivatives to the sulfonamide

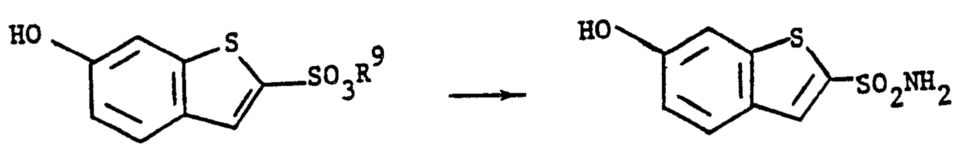

wherein $R^9$ is H, $C_{1-4}$alkyl, phenyl, $C_{1-4}$alkyl or phenyl.

The process comprises treating the sulfonic acid or ester with ammonia at elevated temperature. In the case of the sulfonic acid, it is added to liquid ammonia, sealed in a heavy walled tube and heated slowly to about 180°C to 225°C and held at that temperature for about 1 to 5 hours.

In the case of esters, treatment with a concentrated source of ammonium ions such as ammonium hydroxide or an ammonium salt of a weak acid such as ammonium acetate, ammonium carbonate, ammonium bicarbonate, or ammonium carbonate, at about 50°C to 250°C for about 1 to 5 hours is adequate to prepare the sulfonamide.

Example 75

*Step A:* 6-Hydroxybenzo[b]thiophene-2-sulfonic acid

A mixture of 28.2 g (0.10 mole) of 6-methoxybenzo[b]thiophene-2-sulfonic acid and 75 g of pyridine hydrochloride is heated at 170°C for 3 hours. The reaction is cooled and quenched into a mixture of 30 ml of concentrated hydrochloric acid and 100 ml of saturated sodium chloride solution. The precipitate is filtered and air-dried to give 6-hydroxybenzo[b]thiophene-2-sulfonic acid.

*Step B:* 6-Hydroxy-2-sulfamoylbenzo[b]thiophene

6-Hydroxybenzo[B]thiophene-2-sulfonic acid (11.5 g, 0.05 mole) is added to 50 g of liquid ammonia in a heavy walled tube cooled with dry-ice acetone. The tube is sealed and slowly warmed to 200°C over 3 hours. After 2 hours at 200°C, the tube is cooled to 5°C and vented. The residue is diluted with 100 ml of water, filtered, washed with water and dried to give 6-hydroxy-2-sulfamoylbenzo[b]thiophene.

Example 76

*Step A:* Benzyl (6-hydroxybenzo[b]thiophene)-2-sulfonate

To a solution of 20.6 g (0.10 mole) of dicyclohexylcarbodiimide in 100 ml of DMF at 25°C is added, 10.8 g (0.10 mole) of benzyl alcohol followed by the slow addition (30 minutes) of 11.5 g (0.10 mole) of 6-hydrobenzo[b]thiophene-2-sulfonic acid in 100 ml of DMF. The mixture is aged for 30 minutes and concentrated *in vacuo*. The residue is diluted with ethyl acetate and dicyclohexylurea is removed by filtration. The filtrate is concentrated *in vacuo* to give benzyl (6-hydroxybenzo[b]thiophene)-2-sulfonate.

*Step B:* 6-hydroxy-2-sulfamoylbenzo[b]thiophene

A mixture of 16 g (0.05 mole) of benzyl (6-hydroxybenzo[b]thiophene)-2-sulfonate and 100 ml of concentrated ammonium hydroxide is heated in a sealed tube at 100°C for 2 hours. After cooling, the mixture is filtered, washed with water and dried to give 6-hydroxy-2-sulfamoylbenzo[b]thiophene.

10. De-etherification

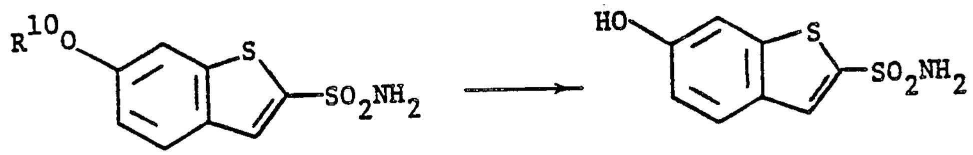

wherein $R^{10}$ is $C_{1-4}$alkyl or phenyl-$C_{1-4}$alkyl.

Employing the procedures substantially as described in Example 1, there are prepared the various $R^{10}$ ethers such as the methoxy, ethoxy, benzyloxy, or 3-phenylpropoxy compound.

These ether groups may be removed by heating in pyridine hydrochloride as described in Example 1, or quinoline hydrochloride in quinoline at about 120°C to 230°C for about 10 minutes to 48 hours.

Similarly, heating the ethers with an alkali metal $C_{1-4}$alkylmercaptide such as sodium n-propylmercaptide, or sodium sulfide, alkali metal thiophenoxides in a solvent such as DMF, or hexamethylphosphoramide at about 100°C to 200°C, especially at reflux temperature also serves to remove the ether group.

Treating the ether with a Lewis acid, such as aluminum chloride, in an aromatic solvent such as toluene, xylene or the like at about 40 to 100°C for about 1 to 5 hours is an alternative procedure to obtain the 6-hydroxy compound. Also boron tribromide or trichloride in a halogenated solvent such as methylene chloride at about 0° to reflux temperature, preferably at about 0—20°C for 0.5 to 10 hours will serve to cleave ether groups.

Another procedure comprises heating the ether in 48% HBr in the presence of lithium bromide at about 75 to 125°C for about 15 minutes to 2 hours.

Example 77
De-etherification with Mercaptides

A solution of the methoxy compound (2.6 mmol) and sodium n-propylmercaptide (9.0 mmol) in 6 ml dimethyl formamide is heated at reflux for 40 minutes. The mixture is cooled, diluted with $H_2O$, acidified with HCl, and the product is extracted into ethyl acetate. The organic solution is dried ($MgSO_4$) and concentrated to give the final product.

Example 78
De-etherification with Aluminium Chloride

To a mixture of aluminum chloride (16 mmole) in toluene (10 ml) is added the methoxy compound (2.0 mmole). After stirring at 60°C for 2 hours, the reaction is quenched by addition of dilute ag. HCl. The product is extracted into ethyl acetate. The organic layer is dried and concentrated to give the final product.

Example 79
De-etherification with HBr

A mixture of the methoxy compound (10 mmole) and lithium bromide (10 mmole) in 10 ml of 48% aq. HBr is heated at reflux for 30 minutes. The mixture is cooled and the product extracted into ethyl acetate. The extracts are dried and concentrated to give the final product.

11. Hydrogenolysis of benzyl ethers

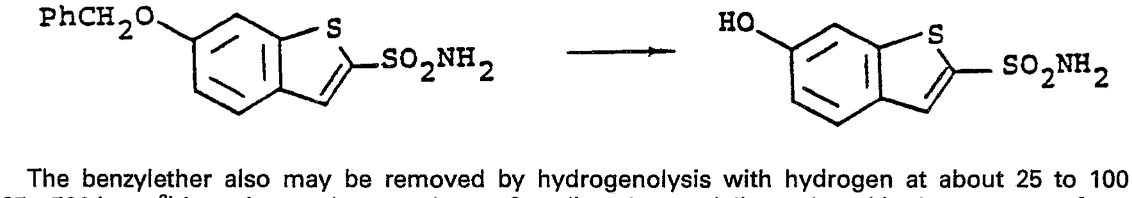

The benzylether also may be removed by hydrogenolysis with hydrogen at about 25 to 100 psi (125—500 $kgm^{-2}$) in an inert solvent such as a $C_{1-3}$alkanol, especially methanol in the presence of a noble metal catalyst such as palladium on carbon, platinum on carbon or copper chromite, at about 15 to 30°C for about 2 to 6 hours or until the requisite amount of hydrogen is consumed.

Alternatively the benzylether may be cleaved via hydrosilylation. For example, the benzylether (1 mmole) in an alkylsilane, preferably triethylsilane, and triethylamine (1 drop) is treated with 10 mg of palladium chloride and refluxed for 3 hours to produce the triethylsilyl ether. The mixture is cooled, filtered,

and the filtrate is diluted with isopropanol to soluolyze the triethylsilyl ether to produce the desired product which precipitates.

Example 80

6-Hydroxy-2-sulfamoylbenzo[b]thiophene

A mixture of the benzyl compound (1.0 mmole) and 10% Pd/C (100 mg) in methanol is shaken under 50 psi (250 kg $m^{-2}$) hydrogen at room temperature for 4 hours. The reaction is vented, filtered, and the filtrate is concentrated *in vacuo* to give the final product.

12. Deacylation of esters

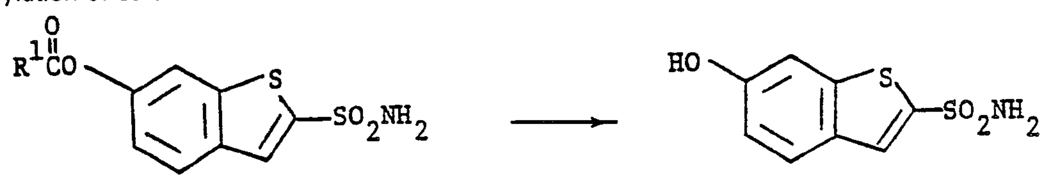

Esters may be removed by basic hydrolysis with bases such as ammonium hydroxide; an alkali metal hydroxide such as sodium or potassium hydroxide; an alkaline earth metal hydroxide such as barium or calcium hydroxide; in a hydroxylic solvent such as water or a lower alkanol or mixtures thereof, at about 0 to 100°C for about 10 minutes to 20 hours.

Acidic hydrolysis will also serve to cleave the ester groups and there may be used any of the usual mineral acids such as hydrochloric, sulfuric, phosphoric or the like in aqueous medium with or without a co-solvent such as a $C_{1-3}$alkanol, THF, ether, toluene or the like at about 0° to 110°C for about 30 minutes to 20 hours.

Example 81

A mixture of 6-acetoxy-2-sulfamoylbenzo[b]thiophene (0.050 mole) in 50 ml DMF is added to 300 ml conc. aq. $NH_4OH$ and heated at 70° for 1 hour. The mixture is cooled, diluted with satd. aqueous NaCl (250 ml) and extracted with several portions of ethyl acetate. The combined extracts are dried ($MgSO_4$) and concentrated *in vacuo* to give the final product.

13. Reduction and dehydration of a 2,3-dihydro-3-oxobenzothio[b]phene

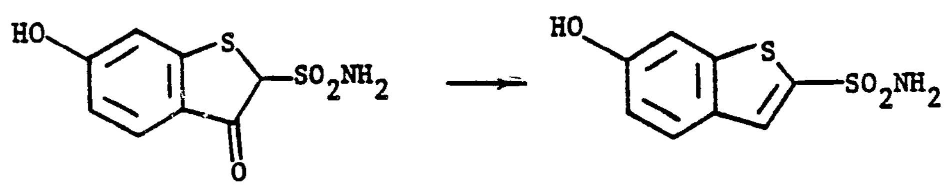

The 3-oxo group is conveniently reduced with a complex metal hydride such as: sodium borohydride in aqueous alkali such as sodium or potassium hydroxide with or without a co-solvent such as a $C_{1-3}$alkanol, or isopropanol with or without alkali at about 0 to 85°C for about 1 to 48 hours; or lithium aluminum hydride in an ethereal solvent such as ether, 1,2-dimethoxyethane, THF or the like at about 20—30°C for about 2 to 24 hours.

Other reducing agents include zinc in acetic acid or zinc amalgam in acetic acid.

Acidifying the reduced product with a common mineral acid causes almost spontaneous dehydration to the desired benzo[b]thiophene.

Example 82

*Step A:* 2-Dimethythiocarbamyloxy-4-methoxybenzoic acid

Dimethylthiocarbamyl chloride (1.1 mole) is added to a solution of 4-methoxysalicylic acid (1.0 mole) and 1,4-diazobicyclo[2.2.2]octane (2.4 mole) in 600 ml dimethyl formamide. The mixture is stirred at room temperature for 18 hours and then poured into 2.5 l $H_2O$ and acidified with aqueous HCl. The product is extracted into toluene. The organic layer is washed with $H_2O$, dried over $MgSO_4$, and concentrated to give the o-aryl dimethylthiocarbamate.

*Step B:* 2-Mercapto-4-methoxybenzoic acid

The o-aryl dimethylthiocarbamate (0.80 mole) is heated at 220°C for 40 minutes. A solution of KOH (2 moles) in aqueous ethylene glycol is added and the mixture is heated at reflux for 1 hour. The cooled reaction is diluted with $H_2O$ and washed with two portions of $CH_2Cl_2$. The aqueous layer is acidified with conc. aqueous HCl and the product is extracted into $CH_2Cl_2$. The organic layer is dried ($MgSO_4$), filtered, and concentrated to give the mercapto acid.

*Step C:* 4-Methoxy-2-(sulfamoylmethylthio)benzoic acid

Potassium t-butoxide (1.0 mole) is added to a mixture of the mercapto acid (0.40 mole) and bromomethanesulfonamide (0.50 mole) in 800 ml tetrahydrofuran and the mixture is refluxed for 20 hours. The reaction is cooled, diluted with water, and washed with ethyl acetate. The aqueous layer is acidified with conc. aq. HCl and the product is extracted into methylene chloride. The organic layer is dried and concentrated to give the sulfide product.

*Step D:* 2,3-Dihydro-6-methoxy-3-oxo-2-sulfamoylbenzo[b]thiophene

To a cold (0°C) solution of the sulfide acid (0.10 mole) in tetrahydrofuran is added a solution of diazomethane (0.10 mole) in ether over 15 minutes. After aging at 0°C for another 10 minutes, a solution of sodium methoxide (0.12 moles) in methanol is added and the mixture is aged at room temperature for 16 hours. The reaction mixture is concentrated nearly to dryness and the residue is partitioned between aq. HCl and methylene chloride. The organic layer is concentrated to give the cyclic product.

*Step E:* 2,3-Dihydro-6-hydroxy-3-oxo-2-sulfamoylbenzo[b]thiophene

A mixture of the methoxy compound (0.077 mole) in 60 g of pyridine hydrochloride is heated at 160°C for 12 hours. The solution is cooled, diluted with $H_2O$, and the product is extracted with ethyl acetate. After washing with aqueous HCl, the organic layer is dried over $MgSO_4$, filtered, and concentrated to give the phenol.

*Step F:* 6-Hydroxy-2-sulfamoylbenzo[b]thiophene

To a solution of the phenol (0.045 mole) in 200 ml of methanol containing 70 ml of 10% aq. NaOH solution is added sodium borohydride (0.05 mole). The mixture is warmed at 50°C for 7 hours and then concentrated *in vacuo* nearly to dryness. Water is added and the mixture is carefully acidified with aqueous HCl. The product is extracted with several portions of ethyl acetate. The combined extracts are dried ($MgSO_4$) and concentrated to give the benzothiophene.

**Claims**

1. A compound of structural formula:

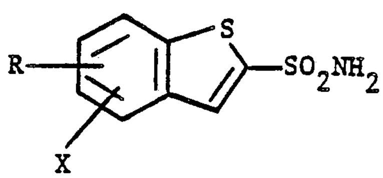

or an ophthalmologically or pharmaceutically acceptable salt thereof, wherein:

X is hydrogen, halo, such as chloro, bromo or fluoro, $C_{1-3}$alkyl, hydroxy or $C_{1-3}$alkoxy; and

R is:

1) hydroxy,

2) $R^1_a$ wherein $R^1_a$ is

a) $C_{1-18}$ alkyl either straight or branched chain substituted with one or more of

i) $C_{3-6}$ cycloalkyl,

ii) halo,

iii) aryl, wherein the aryl group is carbocyclic or heterocyclic and wherein the aryl group can be substituted with one or more of $C_{1-10}$ alkyl, halo, $C_{1-4}$ alkoxy, $C_{2-5}$ alkanoyl, or trifluoromethyl,

iv) hydroxy,

v) $C_{1-3}$ alkoxy,

vi) aryl-$C_{1-3}$ alkoxy,

vii) $C_{1-4}$ alkoxy-$C_{1-3}$ alkoxy,

viii) amino,

ix) ($C_{1-3}$ alkyl) amino,

x) di($C_{1-3}$ alkyl) amino,

xi) 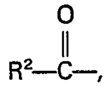

wherein $R^2$ is

1) HO—,

2) $M^+O^-$, wherein $M^+$ is a pharmaceutically acceptable cation,

3) $C_{1-10}$ alkoxy,

4) $R^3R^4N$— wherein $R^3$ and $R^4$ are independently hydrogen, hydroxy, $C_{1-15}$ alkyl, or taken together form a 3—7 membered heterocycle with the nitrogen to which they are attached, or

xii) $C_{2-5}$ alkanoyl,

b) $C_{3-6}$ cycloalkyl,
c) $C_{1-18}$ alkyl-$C_{3-6}$ cycloalkyl,
d) aryl as previously defined,
e) $R^3R^4N$—
f) $C_{2-6}$ alkenyl,
g) aryl-$C_{2-6}$ alkenyl,
h) $C_{2-6}$ alkynyl, or
i) heterocycle of 5 or 6 members with one or two heteroatoms selected from O, N and S;

3) $R^1_a$—O—

4) 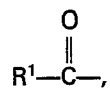

wherein $R^1$ is $R^1_a$ or $C_{1-18}$alkyl,

5) 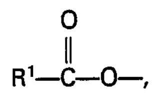

6) 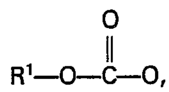

7) 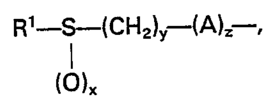

wherein x is 0—2; y is 0—3; z is 0 or 1; and A is a heteroatom selected from S, O and N,

8) 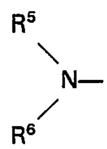

wherein $R^5$ and $R^6$ are independently:
a) hydrogen,
b) $C_{1-18}$ alkyl, either straight or branched chain,
c) $C_{3-6}$ cycloalkyl,
d) $C_{3-6}$ cycloalkyl-$C_{1-3}$ alkyl,
e) aryl-$C_{1-3}$ alkyl wherein the aryl group is either unsubstituted or substituted with one or more of chloro, bromo, fluoro, $C_{1-3}$ alkyl or $C_{1-3}$ alkoxy,

f) 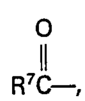 or 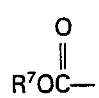

wherein $R^7$ is
i) $C_{1-18}$ alkyl, either straight or branched chain,
ii) aryl, either unsubstituted or substituted with one or more of chloro, bromo, fluoro, $C_{1-3}$ alkyl, or $C_{1-3}$ alkoxy,
iii) aryl-$C_{1-3}$ alkyl wherein the aryl group is either unsubstituted or substituted with one or more of chloro, bromo, fluoro, $C_{1-3}$ alkyl or $C_{1-3}$ alkoxy,
iv) amino-$C_{1-18}$ alkyl either straight or branched chain; or
(g) $R^5$ and $R^6$ if lower alkyl, are joined together directly or through a heteroatom selected from O or N to form a 5 or 6 membered heterocycle with the nitrogen to which they are attached;

9) 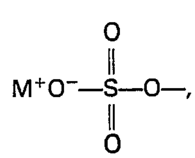

wherein $M^+$ is an ophthalmologically acceptable cation selected from sodium, potassium ammonium, tetra($C_{1-4}$alkyl)ammonium, pyridinium, imidazolium, prolidoxime, and thiamine

10) $(M^+O^-)_2-\overset{\overset{\displaystyle O}{\|}}{P}-O-$,

wherein $M^+$ is as previously defined;

11) $(M^+O^-)-\overset{\overset{\displaystyle O}{\|}}{\underset{\underset{\displaystyle OR^8}{|}}{P}}-O-$,

wherein $R^8$ is $C_{1-3}$ alkyl or phenyl-$C_{1-3}$ alkyl; or

12) $(R^8O)_2-\overset{\overset{\displaystyle O}{\|}}{P}-O-$,

wherein $R^8$ is as previously defined, and the two may be the same or different; and

R and X, joined together, represent methylenedioxy.

2. The compound of Claim 1, wherein R is in the 5 or 6 position.

3. The compound of Claim 2, wherein X is hydrogen, R is HO—,

$$R^1-\overset{\overset{\displaystyle O}{\|}}{C}-O-,\quad R_1-O-\overset{\overset{\displaystyle O}{\|}}{C}-O-\quad \text{or}\quad R^5R^6N-.$$

4. The compound of Claim 3, wherein $R^1$ is $C_{1-4}$ alkyl.

5. The compound of Claim 3, which is:

5(or 6)-hydroxy-2-sulfamoylbenzo[b]thiophene;

5(or 6)-(2-sulfamoylbenzo[b]thienyl) acetate;

5(or 6)-(2-sulfamoylbenzo[b]thienyl) 2,2-dimethylpropionate;

5(or 6)-(2-sulfamoylbenzo[b]thienyl) 2-methylpropionate; or

5(or 6)-(2-sulfamoylbenzo[b]thienyl) 3-methoxycarbonylpropionate.

6. 6-Hydroxy-2-sulfamoylbenzo[b]thiophene.

7. An ophthalmic composition for the topical treatment of glaucoma and elevated intraocular pressure comprising an ophthalmologically acceptable carrier and an effective intraocular pressure lowering amount of a compound with structural formula:

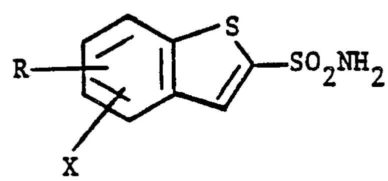

or an ophthalmologically acceptable salt thereof, wherein R and X are as defined in Claim 1.

8. The composition of Claim 7, wherein R is in the 5 or 6 position.

9. The composition of Claim 8, wherein X is hydrogen, R is HO—,

$$R^1-\overset{\overset{\displaystyle O}{\|}}{C}-O-,\quad R_1-O-\overset{\overset{\displaystyle O}{\|}}{C}-O-\quad \text{or}\quad R^5R^6N-.$$

10. The composition of Claim 9, wherein $R^1$ is $C_{1-4}$ alkyl.

11. The composition of Claim 9, wherein the compound is:

5(or 6)-hydroxy-2-sulfamoylbenzo[b]thiophene;

5(or 6)-(2-sulfamoylbenzo[b]thienyl) acetate;

5(or 6)-(2-sulfamoylbenzo[b]thienyl) 2,2-dimethylpropionate;

5(or 6)-(2-sulfamoylbenzo[b]thienyl) 2-methylpropionate; or

5(or 6)-(2-sulfamoylbenzo[b]thienyl) 3-methoxycarbonylpropionate.

12. The composition of Claim 11 wherein the compound is 6-hydroxy-2-sulfamoylbenzo[b]thiophene.

13. A process for the preparation of a compound of structural formula:

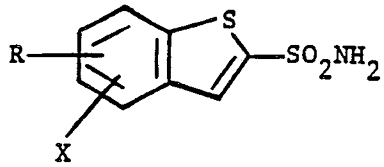

or an ophthalmologically or pharmaceutically acceptable salt thereof, wherein:

X is hydrogen, halo, such as chloro, bromo or fluoro, $C_{1-3}$alkyl, hydroxy or $C_{1-3}$alkoxy; and

R is:

1) hydroxy,

2) $R^1_a$ wherein $R^1_a$ is

a) $C_{1-18}$ alkyl either straight or branched chain substituted with one or more of

i) $C_{3-6}$ cycloalkyl,

ii) halo,

iii) aryl, wherein the aryl group is carbocyclic or heterocyclic and wherein the aryl group can be substituted with one or more of $C_{1-10}$ alkyl, halo, $C_{1-4}$ alkoxy, $C_{2-5}$ alkanoyl, or trifluoromethyl,

iv) hydroxy,

v) $C_{1-3}$ alkoxy,

vi) aryl-$C_{1-3}$ alkoxy,

vii) $C_{1-4}$ alkoxy-$C_{1-3}$ alkoxy,

viii) amino,

ix) ($C_{1-3}$ alkyl) amino,

x) di($C_{1-3}$ alkyl) amino,

xi) 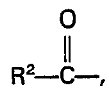

wherein $R^2$ is

1) HO—,

2) $M^+O^-$, wherein $M^+$ is a pharmaceutically acceptable cation,

3) $C_{1-10}$ alkoxy,

4) $R^3R^4N$— wherein $R^3$ and $R^4$ are independently hydrogen, hydroxy, $C_{1-15}$ alkyl, or taken together form a 3—7 membered heterocycle with the nitrogen to which they are attached, or

xii) $C_{2-5}$ alkanoyl,

b) $C_{3-6}$ cycloalkyl,

c) $C_{1-18}$ alkyl-$C_{3-6}$ cycloalkyl,

d) aryl as previously defined,

e) $R^3R^4N$—

f) $C_{2-6}$ alkenyl,

g) aryl-$C_{2-6}$ alkenyl,

h) $C_{2-6}$ alkynyl, or

i) heterocycle of 5 or 6 members with one or two heteroatoms selected from O, N and S;

3) $R^1_a$—O—

4) 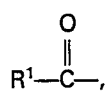

wherein $R^1$ is $R^1_a$ or $C_{1-18}$alkyl,

5) 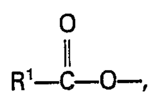

6) 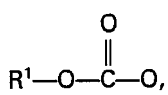

7) 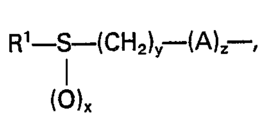

wherein x is 0—2; y is 0—3; z is 0 or 1; and A is a heteroatom selected from S, O and N,

8) 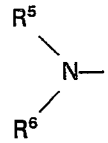

wherein $R^5$ and $R^6$ are independently:

a) hydrogen,

b) $C_{1-18}$ alkyl, either straight or branched chain,

c) $C_{3-6}$ cycloalkyl,

d) $C_{3-6}$ cycloalkyl-$C_{1-3}$ alkyl,

e) aryl-$C_{1-3}$ alkyl wherein the aryl group is either unsubstituted or substituted with one or more of chloro, bromo, fluoro, $C_{1-3}$ alkyl or $C_{1-3}$ alkoxy,

f) 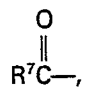 or 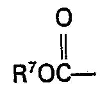

wherein $R^7$ is

i) $C_{1-18}$ alkyl, either straight or branched chain,

ii) aryl, either unsubstituted or substituted with one or more of chloro, bromo, fluoro, $C_{1-3}$ alkyl, or $C_{1-3}$ alkoxy,

iii) aryl-$C_{1-3}$ alkyl wherein the aryl group is either unsubstituted or substituted with one or more of chloro, bromo, fluoro, $C_{1-3}$ alkyl or $C_{1-3}$ alkoxy,

iv) amino-$C_{1-18}$ alkyl either straight or branched chain; or

(g) $R^5$ and $R^6$ if lower alkyl, are joined together directly or through a heteroatom selected from O or N to form a 5 or 6 membered heterocycle with the nitrogen to which they are attached;

9) 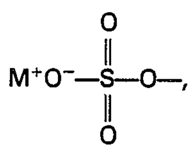

wherein $M^+$ is an ophthalmologically acceptable cation selected from sodium, potassium ammonium, tetra($C_{1-4}$alkyl)ammonium, pyridinium, imidazolium, prolidoxime, and thiamine

10) 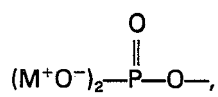

wherein $M^+$ is as previously defined;

11) 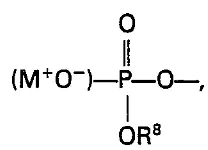

wherein $R^8$ is $C_{1-3}$ alkyl or phenyl-$C_{1-3}$ alkyl; or

12) 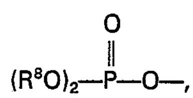

wherein $R^8$ is as previously defined, and the two may be the same or different; and

R and X, joined together, represent methylenedioxy, which comprises:

a) if R is —OH, hydrolysis of the corresponding methyl ether;

b) if R is 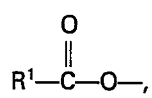

treatment of the compound

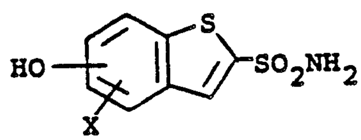

with $R^1$—CO—$X^1$ wherein $R^1$ is as previously defined and $X^1$ is chloro, bromo, iodo, —$OCOR^1$, —$OCOCH_2CH(CH_3)_2$ or —$OCON(C_6H_5)_2$;

c) if R is $R^1$O—CO—O, treatment of the compound

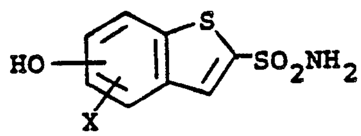

with a chloroformate of formula $R^1$—OCOCl, or a *bis*-carbonate of formula $R^1OCOOR^1$;

d) if R is $R^1_\alpha$—O—, treatment of the compound

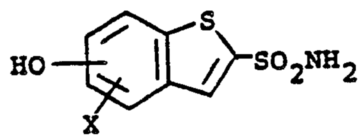

with a compound $R^1_\alpha X^2$ wherein $X^2$ is halo, mesylate, tosylate or benzene sulfonate;

e) if R is $R^1_\alpha$, treatment of the compound

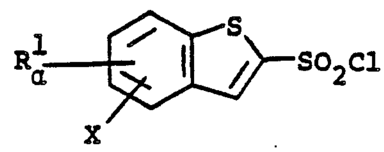

with ammonia.

14. A process for the preparation of a compound of structural formula:

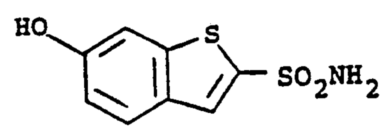

characterized in that:

a) a compound of structural formula:

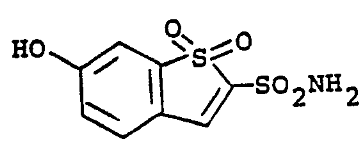

is reduced;

b) a compound of structural formula:

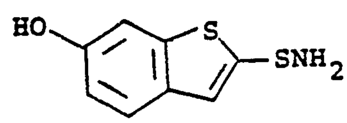

is oxidized;

c) a compound of structural formula:

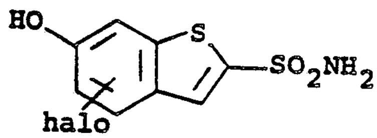

wherein halo is Cl, Br or I is dehalogenated;

d) a compound of structural formula:

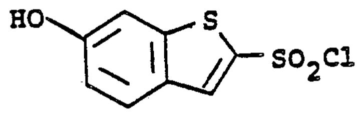

is aminated;

e) a compound of structural formula:

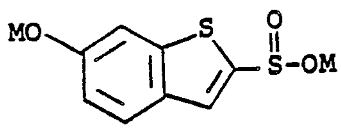

wherein M is an alkali metal cation is treated with an electrophilic nitrogen species;

f) a compound of structural formula:

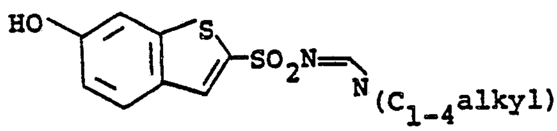

is treated with acid or base;

g) a compound of structural formula:

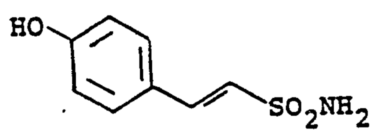

is treated with anhydrous hydrogen halide in liquid sulfur dioxide;

h) a compound of structural formula:

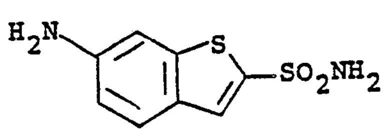

is diazotized;

i) a compound of structural formula:

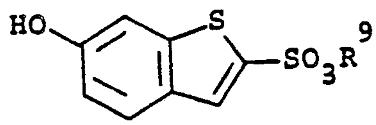

wherein $R^9$ is hydrogen, $C_{1-4}$alkyl, phenyl-$C_{1-4}$alkyl, or phenyl is treated with ammonia or ammonium ions;

j) a compound of structural formula:

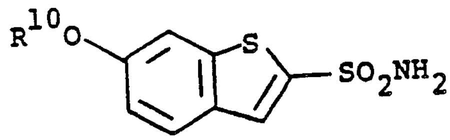

wherein $R^{10}$ is $C_{1-4}$alkyl, or phenyl-$C_{1-4}$alkyl is deetherified;

k) a compound of structural formula:

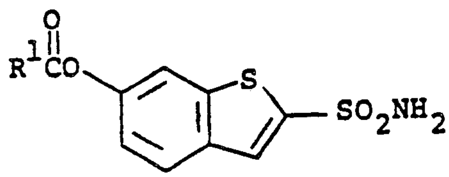

is deacylated;

l) a compound of structural formula:

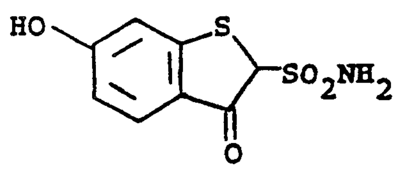

is reduced.

**Patentansprüche**

1. Verbindung der Strukturformel

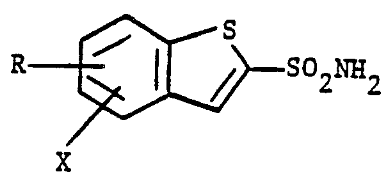

oder ein ophthalmologisch oder pharmazeutisch annehmbares Salz davon, worin

X Wasserstoff, Halogen, wie Chlor, Brom oder Fluor, $C_{1-3}$-Alkyl, Hydroxy oder $C_{1-3}$-Alkoxy ist; und

R 1) Hydroxy,

2) $R^1\alpha$, worin $R^1\alpha$

a) entweder geradkettiges oder verzweigtes $C_{1-18}$- Alkyl, substituiert mit einem oder mehreren aus

i) $C_{3-6}$- Cycloalkyl,

ii) Halogen,

iii) Aryl, worin die Arylgruppe mit einem oder mehreren aus $C_{1-10}$-Alkyl, Halogen, $C_{1-4}$-Alkoxy, $C_{2-5}$-Alkanoyl oder Trifluormethyl substituiert sein kann,

iv) Hydroxy,

v) $C_{1-3}$-Alkoxy,

vi) Aryl-$C_{1-3}$-alkoxy,

vii) $C_{1-4}$-Alkoxy-$C_{1-3}$-alkoxy,

viii) Amino,

ix) ($C_{1-3}$-Alkyl)amino,

x) Di($C_{1-3}$-alkyl)amino,

xi) 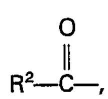

worin $R^2$

1) HO—,

2) $M^+O^-$, worin $M^+$ ein pharmazeutisch annehmbares Kation ist,

3) $C_{1-10}$-Alkoxy,

4) $R^3R^4N$— ist, worin $R^3$ und $R^4$ unabhängig Wasserstoff, Hydroxy, $C_{1-15}$-Alkyl sind oder zusammengenommen mit dem Stickstoff, en den sie gebunden sind, einen 3- bis 7-gliedrigen Heterocyclus bilden, oder

xii) $C_{2-5}$-Alkanoyl ist,

b) $C_{3-6}$-Cycloalkyl,

c) $C_{1-18}$-Alkyl-$C_{3-6}$-cycloalkyl,

d) Aryl, wie vorstehend definiert,

e) $R^3R^4N$—,

f) $C_{2-6}$-Alkenyl,

g) Aryl-$C_{2-6}$-alkenyl,

h) $C_{2-6}$-Alkinyl oder

i) ein 5- oder 6-gliedriger Heterocyclus mit einem oder zwei aus O, N oder S ausgewählten Heteroatomen ist;

3) $R^1a$—O—,

4) 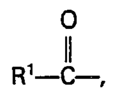

worin $R^1$ $R^1a$ oder $C_{1-18}$-Alkyl ist,

5) 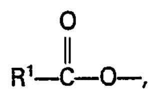

6) 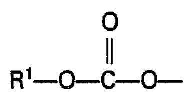

7) 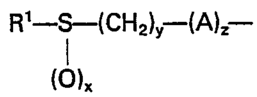

worin x 0—2 ist; y 0—3 ist; z 0 oder 1 ist; und A ein aus S, O und N ausgewähltes Heteroatom ist,

8) 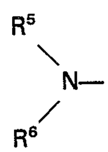

worin $R^5$ und $R^6$ unabhängig

a) Wasserstoff,

b) $C_{1-18}$-Alkyl, entweder geradkettig oder verzweigt,

c) $C_{3-6}$-Cycloalkyl,

d) $C_{3-6}$-Cycloalkyl-$C_{1-3}$-alkyl,

e) Aryl-$C_{1-3}$-alkyl, worin die Arylgruppe entweder unsubstituiert oder mit einem oder mehreren aus Chlor, Brom, Fluor, $C_{1-3}$-Alkyl oder $C_{1-3}$-Alkoxy substituiert ist,

f) 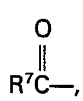 oder 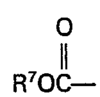

worin $R^7$

i) $C_{1-18}$-Alkyl, entweder geradkettig oder verzweigt,

ii) Aryl, entweder unsubstituiert oder mit einem oder mehreren aus Chlor, Brom, Fluor, $C_{1-3}$-Alkyl oder $C_{1-3}$-Alkoxy substituiert,

iii) Aryl-$C_{1-3}$-alkyl, worin die Arylgruppe entweder unsubstituert oder mit einem oder mehreren aus Chlor, Brom, Fluor, $C_{1-3}$-Alkyl oder $C_{1-3}$-Alkoxy substituiert ist,

iv) Amino-$C_{1-18}$-alkyl, entweder geradkettig oder verzweigt, ist; sind, oder

g) $R^5$ und $R^6$, falls Niederalkyl, direkt oder durch ein aus O oder N ausgewähltes Heteroatom mit dem Stickstoff, an den sie gebunden sind, unter Bildung eines 5- oder 6-gliedrigen Heterocyclus zusammengenommen werden;

9) 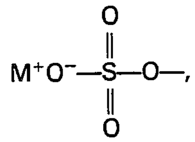

worin $M^+$ ein ophthalmologisch annehmbares Kation ist, ausgewählt aus Natrium, Kalium, Ammonium, Tetra($C_{1-4}$-alkyl)ammonium, Pyridinium, Imidazolium, Prolidoxim und Thiamin,

10) 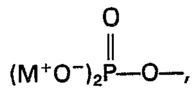

worin $M^+$ wie vorstehend definiert ist,

11) 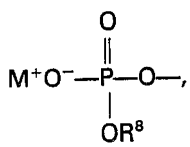

worin $R^8$ $C_{1-3}$-Alkyl oder Phenyl-$C_{1-3}$-alkyl ist; oder

12) 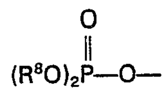 ist,

worin $R^8$ wie vorstehend definiert ist und die beiden gleich oder verschieden sein können, ist; und R und X, zusammengenommen, Methylendioxy darstellen.

2. Verbindung nach Anspruch 1, worin R in der 5- oder 6-Stellung ist.

3. Verbindung nach Anspruch 2, worin X Wasserstoff ist und R HO—,

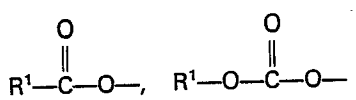 oder $R^5R^6N$— ist.

4. Verbindung nach Anspruch 3, worin $R^1$ $C_{1-4}$-Alkyl ist.

5. Verbindung nach Anspruch 3, welche
5-(oder 6-)Hydroxy-2-sulfamoylbenzo[b]thiophen;
5-(oder 6-)(2-Sulfamoylbenzo[b]thienyl)acetat;
5-(oder 6-)(2-Sulfamoylbenzo[b]thienyl)-2,2-dimethylpropionat;
5-(oder 6-)(2-Sulfamoylbenzo[b]thienyl)-2-methylpropionat; oder
5-(oder 6-)(2-Sulfamoylbenzo[b]thienyl)-3-methoxycarbonylpropionat ist.

6. 6-Hydroxy-2-sulfamoylbenzo[b]thiophen.

7. Ophthalmische Zusammenzetzung zur topischen Behandlung von Glaucom und erhöhtem Augeninnendruck, welche einen ophthalmologisch annehmbaren Träger und eine den Augeninnendruck wirksam vermindernde Menge einer Verbindung der Strukturformel

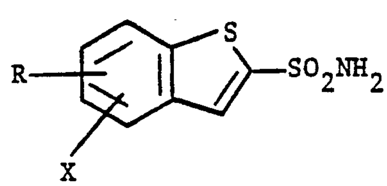

oder ein ophthalmologisch annehmbares Salz davon, worin R und X wie in Anspruch 1 definiert sind, umfaßt.

8. zusammensetzung nach Anspruch 7, worin R in der 5- oder 6-Stellung ist.

9. Zusammensetzung nach Anspruch 8, worin X Wasserstoff ist und R HO—,

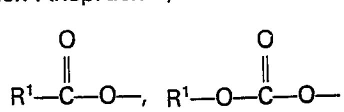 oder $R^5R^6N$—, ist.

10. Zusammensetzung nach Anspruch 9, worin $R^1$ $C_{1-4}$-Alkyl ist.

11. Zusammensetzung nach Anspruch 9, worin die Verbindung
5-(oder 6-)Hydroxy-2-sulfamoylbenzo[b]thiophen;
5-(oder 6-)(2-Sulfamoylbenzo[b]thienyl)acetat;
5-(oder 6-)(2-Sulfamoylbenzo[b]thienyl)-2,2-dimethylpropionat;
5-(oder 6-)(2-Sulfamoylbenzo[b]thienyl)-2-methylpropionat; oder
5-(oder 6-)(2-Sulfamoylbenzo[b]thienyl)-3-methoxycarbonylpropionat ist.

12. Zusammensetzung nach Anspruch 11, worin die Verbindung 6-Hydroxy-2-sulfamoylbenzo[b]-thiophen ist.

13. Verfahren zur Herstellung einer Verbindung der Strukturformel

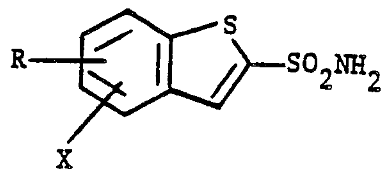

oder eines ophthalmologisch oder pharmazeutisch annehmbaren Salzes davon, worin
X Wasserstoff, Halogen, wie Chlor, Brom oder Fluor, $C_{1-3}$-Alkyl, Hydroxy oder $C_{1-3}$-Alkoxy ist; und
R 1) Hydroxy,
2) $R^1\alpha$, worin $R^1\alpha$
a) entweder geradkettiges oder verzweigtes $C_{1-18}$- Alkyl, substituiert mit einem oder mehreren aus
i) $C_{3-6}$- Cycloalkyl,
ii) Halogen,
iii) Aryl, worin die Arylgruppe mit einem oder mehreren aus $C_{1-10}$-Alkyl, Halogen, $C_{1-4}$-Alkoxy, $C_{2-5}$-Alkanoyl oder Trifluormethyl substituiert sein kann,
iv) Hydroxy,
v) $C_{1-3}$-Alkoxy,
vi) Aryl-$C_{1-3}$-alkoxy,
vii) $C_{1-4}$-Alkoxy-$C_{1-3}$-alkoxy,
viii) Amino,
ix) ($C_{1-3}$-Alkyl)amino,
x) Di($C_{1-3}$-alkyl)amino,
xi) 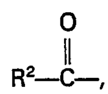
worin $R^2$
1) HO—,
2) $M^+O^-$, worin $M^+$ ein pharmazeutisch annehmbares Kation ist,
3) $C_{1-10}$-Alkoxy,
4) $R^3R^4N$— ist, worin $R^3$ und $R^4$ unabhängig Wasserstoff, Hydroxy, $C_{1-15}$-Alkyl sind oder zusammengenommen mit dem Stickstoff, en den sie gebunden sind, einen 3- bis 7-gliedrigen Heterocyclus bilden, oder
xii) $C_{2-5}$-Alkanoyl ist,
b) $C_{3-6}$-Cycloalkyl,
c) $C_{1-18}$-Alkyl-$C_{3-6}$-cycloalkyl,
d) Aryl, wie vorstehend definiert,
e) $R^3R^4N$—,
f) $C_{2-6}$-Alkenyl,
g) Aryl-$C_{2-6}$-Alkenyl,
h) $C_{2-6}$-Alkinyl oder
i) ein 5- oder 6-gliedriger Heterocyclus mit einem oder zwei aus O, N oder S ausgewählten Heteroatomen ist;
3) $R^1\alpha$—O—,
4) 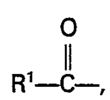
worin $R^1$ $R^1\alpha$ oder $C_{1-18}$-Alkyl ist,
5) 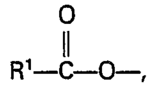

6) 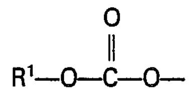

7) 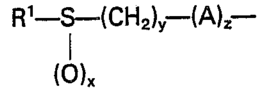

worin x 0—2 ist; y 0—3 ist; z 0 oder 1 ist; und A ein aus S, O und N ausgewähltes Heteroatom ist,

8) 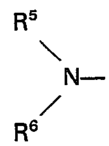

worin $R^5$ und $R^6$ unabhängig

a) Wasserstoff,

b) $C_{1-18}$-Alkyl, entwder geradkettig oder verzweigt,

c) $C_{3-6}$-Cycloalkyl,

d) $C_{3-6}$-Cylcloalkyl-$C_{1-3}$-alkyl,

e) Aryl-$C_{1-3}$-alkyl, worin die Arylgruppe entweder unsubstituiert oder mit einem oder mehreren aus Chlor, Brom, Fluor, $C_{1-3}$-Alkyl oder $C_{1-3}$-Alkoxy substituiert ist,

f) 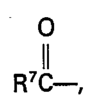, oder 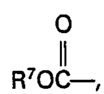,

worin $R^7$

i) $C_{1-18}$-Alkyl, entweder geradkettig oder verzweigt,

ii) Aryl, entweder unsubstituiert oder mit einem oder mehrreren aus Chlor, Brom, Fluor, $C_{1-3}$-Alkyl oder $C_{1-3}$-Alkoxy substituiert,

iii) Aryl-$C_{1-3}$-alkyl, worin die Arylgruppe entweder unsubstituert oder mit einem oder mehreren aus Chlor, Brom, Fluor, $C_{1-3}$-Alkyl oder $C_{1-3}$-Alkoxy substituiert ist,

iv) Amino-$C_{1-18}$-alkyl, entweder geradkettig oder verzweigt, ist; sind, oder

g) $R^5$ und $R^6$, falls Niederalkyl, direkt oder durch ein aus O oder N ausgewähltes Heteroatom mit dem Stickstoff, an den sie gebunden sind, unter Bildung eines 5- oder 6-gliedrigen Heterocyclus zusammengenommen werden;

9) 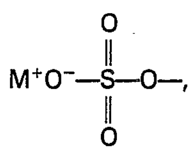

worin $M^+$ ein ophthalmologisch annehmbares Kation ist, ausgewählt aus Natrium, Kalium, Ammonium, Tetra($C_{1-4}$-alkyl)ammonium, Pyridinium, Imidazolium, Prolidoxim und Thiamin,

10) 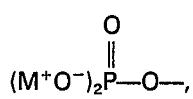

worin $M^+$ wie vorstehend definiert ist,

11) 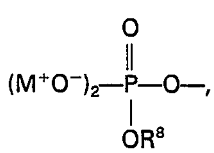

worin $R^8$ $C_{1-3}$-Alkyl oder Phenyl-$C_{1-3}$-alkyl ist; oder

12) 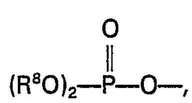

worin $R^8$ wie vorstehend definiert ist und die beiden gleich oder verschieden sein können, ist; und R und X, zusammengenommen, Methylendioxy darstellen, durch

a) falls R —OH ist, Hydrolyse des entsprechenden Methylethers;

b) falls R $R^1-\overset{\overset{O}{\|}}{C}-O-$

ist, Behandeln der Verbindung

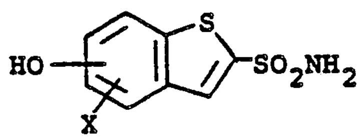

mit $R^1$—CO—$X^1$, worin $R^1$ wie vorstehend definiert ist und $X^1$ Chlor, Brom, Iod, —$OCOR^1$, —$OCOCH_2CH(CH_3)_2$ oder —$OCON(C_6H_5)_2$ ist;

c) falls R $R^1$O—CO—O— ist, Behandeln der Verbindung

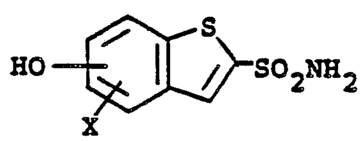

mit einem Chlorformiat der Formel $R^1$—OCOCl oder einem *Bis*-carbonat der Formel $R^1OCOOR^1$;

d) falls R $R^1\alpha$—O— ist, Behandeln der Verbindung

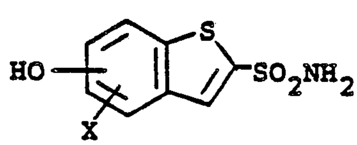

mit einer Verbindung $R^1\alpha X^2$, worin $X^2$ Halogen, Mesylat, Tosylat oder Benzolsulfonat ist;

e) falls R $R^1\alpha$ ist, Behandeln der Verbindung

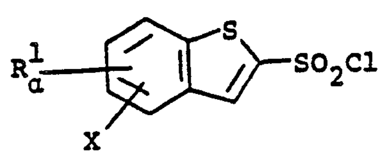

mit Ammoniak.

14. Verfahren zur Herstellung einer Verbindung der Strukturformel

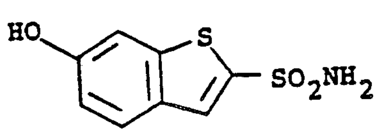

dadurch gekennzeichnet, daß

a) eine Verbindung der Strukturformel

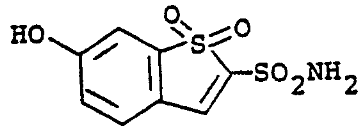

reduziert wird;

b) eine Verbindung der Strukturformel

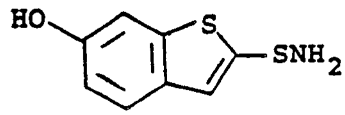

oxidiert wird;

c) eine Verbindung der Strukturformel

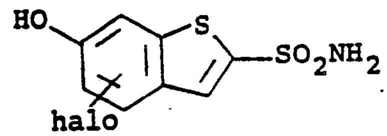

worin halo Cl, Br oder I ist, dehalogeniert wird;

d) eine Verbindung der Strukturformel

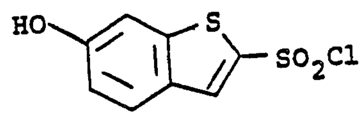

aminiert wird;

e) eine Verbindung der Strukturformel

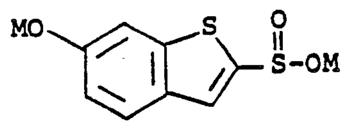

worin M ein Alkalimetallkation ist, mit einer elektrophilen Stickstoffspezies behandelt wird;

f) eine Verbindung der Strukturformel

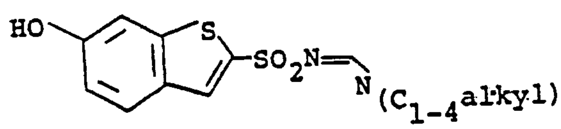

mit Säure oder Base behandelt wird;

g) eine Verbindung der Strukturformel

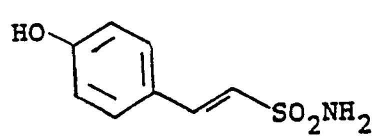

mit wasserfreiem Wasserstoffhalogenid in flüssigem Schwefeldioxid behandelt wird;

h) eine Verbindung der Strukturformel

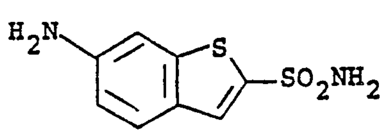

diazotiert wird;

i) eine Verbindung der Strukturformel

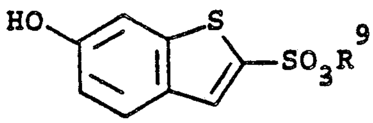

worin $R^9$ Wasserstoff, $C_{1-4}$-Alkyl, Phenyl-$C_{1-4}$-alkyl oder Phenyl ist, mit Ammoniak oder Ammoniumionen behandelt wird;

j) eine Verbindung der Strukturformel

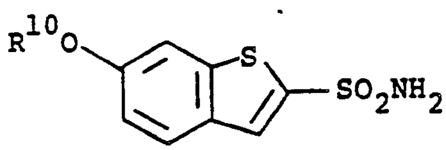

worin $R^{10}$ $C_{1-4}$-Alkyl oder Phenyl-$C_{1-4}$-alkyl ist, deetheifiziert wird;

k) eine Verbindung der Strukturformel

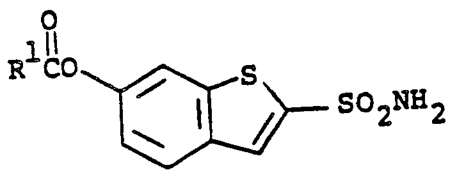

deacyliert wird;

l) eine Verbindung der Strukturformel

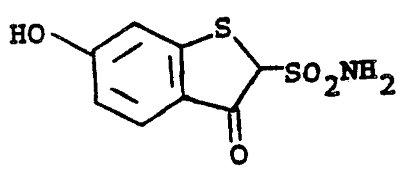

reduziert wird.

**Revendications**

1. Composé de formule structurelle

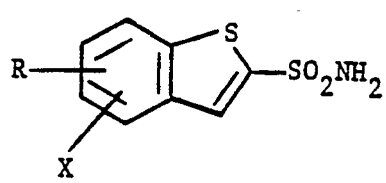

ou un sel de celui-ci acceptable en ophtalmologie ou en pharmacie, dans laquelle:

X est un hydrogène, un halo, tel que chloro, bromo ou fluoro, un alkyle $C_{1-3}$, un hydroxy ou un alcoxy $C_{1-3}$ et

R est:

1) un hydroxy,

2) $R_a^1$ où $R_a^1$ est

a) un alkyle $C_{1-18}$ à chaîne droite un ramifiée substitué avec au moins un

i) cycloalkyl $C_{3-6}$,

ii) halo,

iii) aryl, où le groupe aryle est carboxylique ou hétérocyclique et où le groupe aryle peut être substitué avec au moins un des alkyle $C_{1-10}$, halo, alcoxy $C_{1-4}$, alcanoyle $C_{2-5}$, ou trifluorométhyle,

iv) hydroxy,

v) alcoxy $C_{1-3}$
vi) aryl-alcoxy $C_{1-3}$,
vii) alcoxy $C_{1-4}$ -alcoxy $C_{1-3}$,
viii) amino,
ix) (alkyl $C_{1-3}$)amino,
x) di(alkyl $C_{1-3}$)amino

xi) 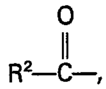

où $R^2$ est
1) $HO^-$,
2) $M^+O^-$, ou $M^+$ est un cation acceptable en pharmacie,
3) alcoxy $C_{1-10}$,
4) $R^3R^4N$— où $R^3$ et $R^4$ sont, de façon indépendante, un hydrogène, un hydroxy, un alkyle $C_{1-15}$, ou pris ensemble, forment un hétérocycle ayant de 3 à 7 chaînons avec l'azote auquel ils sont attachés, ou
xii) alcanoyle $C_{2-5}$,
b) un cycloalkyle $C_{3-6}$,
c) un alkyl $C_{1-18}$-cycloalkyle $C_{3-6}$,
d) un aryle tel que défini précédemment,
e) $R^3R^4N$—
f) un alkényle $C_{2-6}$,
g) un aryl-alkényle $C_{2-6}$,
h) un alkynyle $C_{2-6}$, ou
i) un hétérocycle à 5 ou 6 chaînons avec un ou deux hétéroatomes choisis parmi O, N, et S
3) $R^1_a$—O—

4) 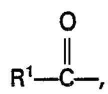

où $R^1$ est $R^1_a$ ou un alkyle $C_{1-18}$

5) 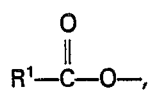

6) 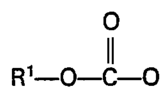

7) 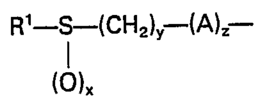

où x est 0—2; y est 0—3; z est 0 ou 1; et A est un hétéroatome choisi parmi S, O et N,

8) 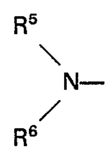

où
$R^5$ et $R^6$ sont, de façon indépendante:
a) un hydrogène,
b) un alkyle $C_{1-18}$, à chaîne droite ou ramifiée,
c) un cycloalkyl $C_{3-6}$,
d) un cycloalkyl $C_{3-6}$ -alkyle $C_{1-3}$,
e) un aryl-alkyle $C_{1-3}$ dans lequel le groupe aryle est soit non substitué, soit substitué avec un ou plusieurs des chloro, bromo, fluoro, alkyle $C_{1-3}$ ou alcoxy $C_{1-3}$,

f) 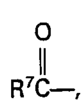, ou 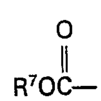

où $R^7$ est,
i) un alkyle $C_{1-18}$, à chaîne droite ou ramifiée,

ii) un aryle, non substitué ou substitué avec un ou plusieurs des chloro, bromo, fluoro, alkyle $C_{1-3}$, ou alcoxy $C_{1-3}$

iii) un aryl-alkyle $C_{1-3}$ dans lequel le groupe aryle est non substitué ou substitué avec un ou plusieurs des chloro, bromo, fluoro, alkyle $C_{1-3}$, ou alcoxy $C_{1-3}$,

iv) un amino-alkyle $C_{1-18}$ à chaîne droite ou ramifiée: ou

g) $R^5$ et $R^6$, s'ils sont des alkyles inférieurs, sont réunis ensemble directement ou par un hétéroatome choisi parmi O ou N pour former un hétérocycle à 5 ou 6 chaînons avec l'azote auquel ils sont reliés;

9) 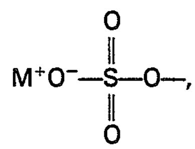

où

$M^+$ est un cation acceptable en ophtalomologie choisi parmi le sodium, le potassium, l'ammonium, le tétra (alkyl $C_{1-4}$) ammonium, le pyridinium, l'imidazolium, le prolidoxime, et la thiamine

10) 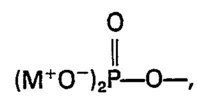

où $M^+$ est tel que défini précédemment;

11) 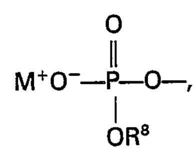

où $R^8$ est un alkyle $C_{1-3}$ ou un phényl-alkyle $C_{1-3}$; ou

12) 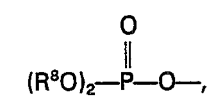

où $R^8$ est tel que défini précédemment, et les deux peuvent être identiques ou différents; et

R et X, réunis ensemble, représentent un méthylènedioxy.

2. Composé selon la revendication 1, où R est dans la position 5 ou 6.

3. Composé selon la revendication 2, où X est un hydrogène, R est $HO^-$,

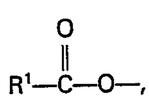, 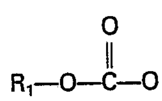 ou $R^5R^6N$—.

4. Composé selon la revendication 3, où $R^1$ est un alkyle $C_{1-4}$.

5. Composé selon la revendication 3, qui est:

le 5 (ou 6)-hydroxy-2-sulfamoylbenzo[b]thiophène;

le 5 (ou 6)-(2-sulfamoylbenzo[b]thiényl) acétate;

le 5 (ou 6)-(2-sulfamoylbenzo[b]thiényl) 2,2-diméthylpropionate;

le 5 (ou 6)-(2-sulfamoylbenzo[b]thiényl) 2-méthylpropionate; ou

le 5 (ou 6)-(2-sulfamoylbenzo[b]thiényl) 3-méthoxycarbonylpropionate.

6. 6-hydroxy-2-sulfamoylbenzo[b]thiophène.

7. Composition ophtalmologique pour le traitement topique du glaucome et d'une pression intraoculaire élevée, comprenant un support acceptable en ophtalmologie et une quantité efficace, pour diminuer la pression intraoculaire de l'oeil, d'un composé ayant la formule structurelle:

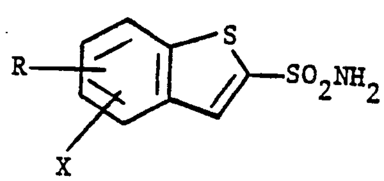

ou un sel de celui-ci acceptable en ophtalmologie, dans lequel R et X sont tels que définis à la revendication 1.

8. Composition selon la revendication 7, où R est dans la position 5 ou 6.

9. Composition selon la revendication 8, où X est un hydrogène, R est $HO^-$,

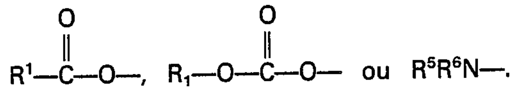

10. Composition selon la revendication 9, où $R^1$ est un alkyle $C_{1-4}$.

11. Composition selon la revendication 9, où le composé est:
le 5 (ou 6)-hydroxy-2-sulfamoylbenzo[b]thiophène;
le 5 (ou 6)-(2-sulfamoylbenzo[b]thiényl) acétate;
le 5 (ou 6)-(2-sulfamoylbenzo[b]thiényl) 2,2-diméthylpropionate;
le 5 (ou 6)-(2-sulfamoylbenzo[b]thiényl) 2-méthylpropionate; ou
le 5 (ou 6)-(2-sulfamoylbenzo[b]thiényl) 3-méthoxycarbonylpropionate.

12. Composition selon la revendiction 11 où le composé est le 6-hydroxy-2-sulfamoylbenzo [b] thiophène.

13. Procédé pour la préparation d'un composé de formule structurelle:

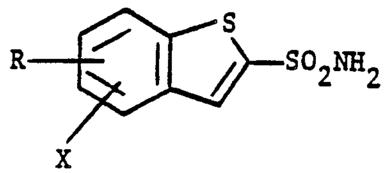

ou d'un sel de celui-ci acceptable en ophtalmologie ou en pharmacie, dans lequel:

X est un hydrogène, un halo, tel que chloro, bromo ou fluoro, un alkyle $C_{1-3}$, un hydroxy ou un alcoxy $C_{1-3}$ et

R est
1) un hydroxy,
2) $R_a^1$ où $R_a^1$ est
a) un alkyle $C_{1-18}$ à chaîne droite un ramifiée substitué avec au moins un
i) cycloalkyle $C_{3-6}$,
ii) halo,
iii) aryle, où le groupe aryle est carbocyclique ou hétérocyclique et où le groupe aryle peut être substitué avec un ou plusieurs des alkyle $C_{1-10}$, halo, alcoxy $C_{1-4}$, alcanoyle $C_{2-5}$, ou trifluorométhyle,
iv) hydroxy,
v) alcoxy $C_{1-3}$,
vi) aryl-alcoxy $C_{1-3}$,
vii) alcoxy $C_{1-4}$-alcoxy $C_{1-3}$,
viii) amino,
ix) (alkyl $C_{1-3}$)amino,
x) di(alkyl $C_{1-3}$)amino

xi) 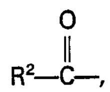

où $R^2$ est
1) $HO^-$,
2) $M^+O^-$, où $M^+$ est un cation acceptable en pharmacie,
3) un alcoxy $C_{1-10}$,
4) $R^3R^4N$— où $R^3$ et $R^4$ sont, de façon indépendante, un hydrogène, un hydroxy, un alkyle $C_{1-15}$, ou, pris ensemble, forment un hétérocycle à 3—7 chaînons avec l'azote auquel ils sont rattachés, ou
xii) alcanoyle $C_{2-5}$,
b) un cycloalkyle $C_{3-6}$,
c) un alkyl $C_{1-18}$-cycloalkyle $C_{3-6}$,
d) un aryle tel que défini précédemment,
e) $R^3R^4N$—
f) un alkényle $C_{2-6}$,
g) un aryl-$C_{2-6}$ alkényle,
h) un alkynyle $C_{2-6}$, ou
i) un hétérocycle à 5 ou 6 chaînons avec un ou deux hétéroatomes choisis parmi O, N et S;
3) $R_a^1$—O—

4) 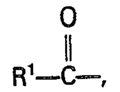

où $R^1$ est $R^1_\alpha$ ou un alkyle $C_{1-18}$

5) 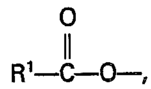

6) 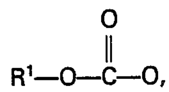

7) 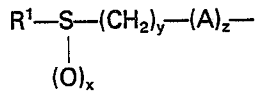

où x est 0—2; y est 0—3; z est 0 ou 1; et A est un hétéroatome choisi parmi S, O et N,

8) 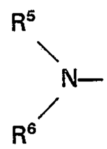

où

$R^5$ et $R^6$ sont, de façon indépendante:

a) un hydrogène,

b) un alkyle $C_{1-18}$, à chaîne droite ou ramifiée,

c) un cycloalkyle $C_{3-6}$,

d) un cycloalkyle $C_{3-6}$ -alkyl $C_{1-3}$,

e) un aryl-alkyle $C_{1-3}$ où le groupe aryle est non substitué ou substitué avec au moins un des chloro, bromo, fluoro, alkyle $C_{1-3}$ ou alcoxy $C_{1-3}$

f) 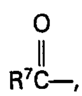, ou 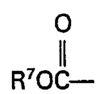

où $R^7$ est

i) un alkyle $C_{1-18}$, à chaîne droite ou ramifiée,

ii) un aryle, non substitué ou substitué avec au moins un des chloro, bromo, fluoro, alkyle $C_{1-3}$, ou alcoxy $C_{1-3}$,

iii) un aryl-alkyle $C_{1-3}$ où le groupe aryle est non substitué ou substitué avec au moins un des chloro, bromo, fluoro, alkyle $C_{1-3}$, ou alcoxy $C_{1-3}$

iv) un amino-alkyle $C_{1-18}$ à chaîne droite ou ramifiée: ou

g) $R^5$ et $R^6$, si ce sont des alkyles inférieurs, sont réunis ensemble directement ou par un hétéroatome choisi parmi O ou N pour former un hétérocycle à 5 ou 6 chaînons avec l'azote auquel ils sont attachés;

9) 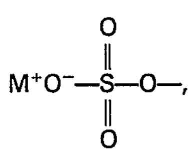

où

$M^+$ est un cation acceptable en ophtalomologie choisi parmi le sodium, le potassium, l'ammonium, le tétra (alkyl $C_{1-4}$) ammonium, le pyridinium, l'imidazolium, la prolidoxime, et la thiamine

10) 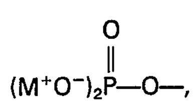

où $M^+$ est tel que défini précédemment,

11) 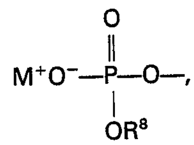

où $R^8$ est un alkyle $C_{1-3}$ ou un phényl-alkyle $C_{1-3}$; ou

12) 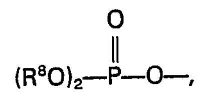

où $R^8$ est tel que défini précédemment, et les deux peuvent être identiques ou différents; et R et X, réunis ensemble, représentent un méthylènedioxy, qui comporte:

a) si R est —OH, l'hydrolyse du méthyl éther correspondant;

b) si R est 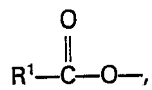

le traitement du composé

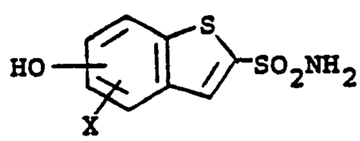

avec $R^1$—CO—$X^1$ où $R^1$ est tel que défini précédemment et $X^1$ est un chloro, un bromo, un iodo, —$OCOR^1$, —$OCOCH_2CH(CH_3)_2$ ou —$OCON(C_6H_5)_2$;

c) si R est $R^1O$—CO—O, le traitement du composé

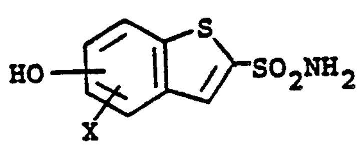

avec un chloroformate de formule $R^1$—$OCOC^1$, ou un *bis*-carbonate de formule $R^1OCOOR^1$;

d) si R est $R^1_\alpha$—O—, le traitement du composé

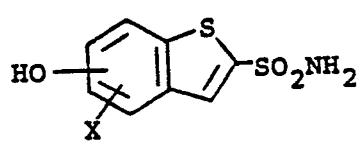

avec un composé $R^1_\alpha$ $X^2$ où $X^2$ est un halo, un mésylate un tosylate ou un benzène sulfonate;

e) si R est $R^1_\alpha$, le traitement du composé

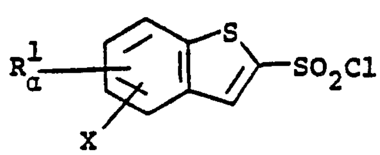

avec de l'ammoniac.

14. Procédé pour la préparation d'un composé de formule structurelle:

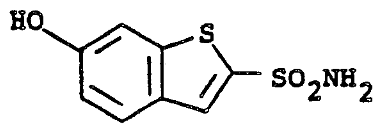

caractérisé en ce que:

a) un composé de formule structurelle:

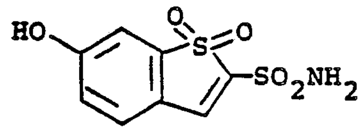

est réduit;

b) un composé de formule structurelle:

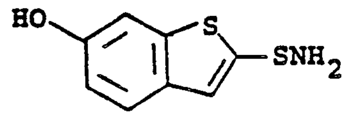

est oxydé;

c) un composé de formule structurelle:

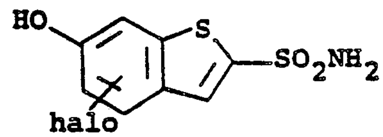

où halo est Cl, Br ou I, est deshalogéné;

d) un composé de formule structurelle:

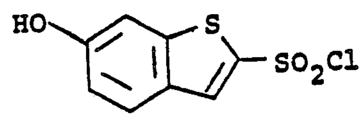

est aminé;

e) un composé de formule structurelle:

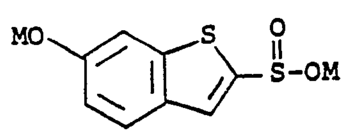

dans laquelle M est un cation de métal alcalin, est traité avec un composé de l'azote électrophile

f) un composé de formule structurelle:

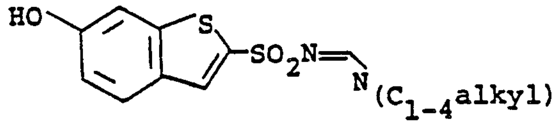

est traité avec un acide ou une base;

g) un composé de formule structurelle:

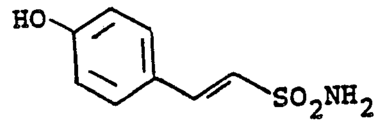

est traité avec un halogénure d'hydrogène anhydre dans du dioxyde de soufre liquide;

h) un composé de formule structurelle:

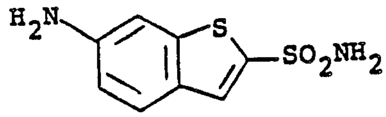

est diazoté;

i) un composé de formule structurelle:

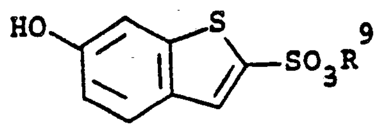

où $R^9$ est un hydrogène, un alkyle $C_{1-4}$, un phénylalkyle $C_{1-4}$, ou un phényle, est traité avec de l'ammoniac ou des ions ammonium;

j) un composé de formule structurelle:

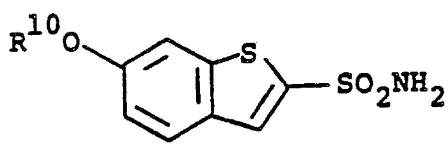

où $R^{10}$ est un alkyle $C_{1-4}$, ou un phényl-alkyle $C_{1-4}$ est déethérifié;

k) un composé de formule structurelle:

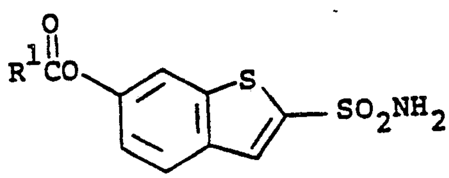

est déacylé;

l) un composé de formule structurelle:

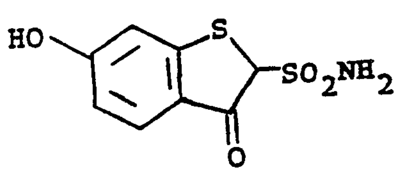

est réduit.